# EUROPÄISCHE PATENTSCHRIFT

(11) **EP 2 234 958 B1**
(45) Veröffentlichungstag und Bekanntmachung des Hinweises auf die Patenterteilung: **18.09.2013**
(21) Anmeldenummer: 08864515.5
(22) Anmeldetag: 10.12.2008
(51) Int. Cl.: C07C 209/26, C07C 209/52, C07C 209/62, C07C 211/07, C07C 211/27

(54) **VERFAHREN ZUR DIASTEREOSELEKTIVEN UMSETZUNG VON CHIRALEN IMINEN**
METHOD FOR DIASTEREO-SELECTIVE CONVERSION OF CHIRAL IMINES
PROCÉDÉ DE TRANSFORMATION DIASTÉRÉOSÉLECTIVE D'IMINES CHIRALES

(30) Priorität: 21.12.2007 EP 07150290
(43) Veröffentlichungstag der Anmeldung: 06.10.2010
(73) Patentinhaber: BASF SE, 67056 Ludwigshafen (DE)
(72) Erfinder: SIEGEL, Wolfgang, 67117 Limburgerhof (DE); HAHN, Thilo, 67292 Kirchheimbolanden (DE); STÄB, Tobias, 67227 Frankenthal (DE)
(86) Internationale Anmeldenummer: PCT/EP2008/067191
(87) Internationale Veröffentlichungsnummer: WO 2009/080511

(56) Entgegenhaltungen:
- EP-A- 0 443 606
- WO-A-01/09080
- WO-A-2006/008171
- WO-A-2006/030017

## Beschreibung

Die vorliegende Erfindung betrifft ein Verfahren zur diastereoselektiven Umsetzung von von chiralen Iminen der Formel I zu Aminen der Formel II, 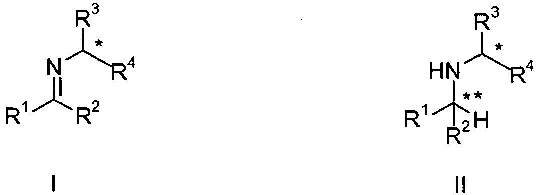 wobei
- R¹, R²: C₁-C₆-Alkyl, C₃-C₆-Cycloalkyl, C₂-C₆-Alkenyl, C₂-C₆-Alkinyl, C₂-C₆-Halogen-alkenyl, C₂-C₆-Halogenalkinyl, C₁-C₆-Alkoxycarbonyl, C₃-C₆-Alkenyloxycarbonyl, C₃-C₆-Alkinyloxycarbonyl, Aminocarbonyl, C₁-C₆-Alkylaminocarbonyl, C₃-C₆-Alkenylaminocarbonyl, C₃-C₆-Alkinylaminocarbonyl, C₁-C₆-Alkylsulfonylamino-carbonyl, Di(C₁-C₆-alkyl)aminocarbonyl, N-(C₃-C₆-Alkenyl)-N-(C₁-C₆-alkyl)amino-carbonyl, N-(C₃-C₆-Alkinyl)-N-(C₁-C₆-alkyl)-aminocarbonyl, N-(C₁-C₆-Alkoxy)-N-(C₁-C₆-alkyl)-amino-carbonyl, N-(C₃-C₆-Alkenyl)-N-(C₁-C₆-alkoxy)aminocarbonyl, N-(C₃-C₆-Alkinyl)-N-(C₁-C₆-alkoxy)-aminocarbonyl, (C₁-C₆-Alkyl)aminothio-carbonyl, Di(C₁-C₆-alkyl)aminothiocarbonyl oder C₁-C₆-Alkylcarbonyl-C₁-C₆-alkyl, wobei die genannten Alkyl-, Cycloalkyl- und Alkoxyreste partiell oder vollständig halogeniert sein können und/oder eine bis drei der folgenden Gruppen tragen können: Cyano, Hydroxy, C₁-C₄-Alkyl, C₃-C₆-Cycloalkyl, C₁-C₆-Alkoxy-C₁-C₄-alkyl, C₁-C₄-Alkoxy-C₁-C₄-alkoxy-C₁-C₄-alkyl, C₁-C₄-Alkoxy, C₁-C₄-Alkylthio, Ami-no, C₁-C₄-alkyl-amino, Di(C₁-C₄-alkyl)amino, C₁-C₄-Alkyl-carbonylamino, Hydroxycarbonyl, C₁-C₄-Alkoxycarbonyl, Aminocarbonyl, C₁-C₄-Alkylaminocarbonyl, Di(C₁-C₄-alkyl)aminocarbonyl oder C₁-C₄-Alkylcarbonyloxy;
Aryl, Aryl-C₁-C₄-alkyl, Aryl-C₂-C₄-alkenyl, Aryl-C₂-C₄-alkinyl, Aryl-C₁-C₄-halogen-alkyl, Aryl-C₂-C₄-halogenalkenyl, Aryl-C₃-C₄-halogenalkinyl, Aryl-C₁-C₄-hydroxy-alkyl, Arylcarbonyl-C₁-C₄-alkyl, Aryl-C₁-C₄-alkyl carbonyl-C₁-C₄-alkyl, Arylcarbonyloxy-C₁-C₄-alkyl, Aryloxycarbonyl-C₁-C₄-alkyl, Aryloxy-C₁-C₄-alkyl, Arylamino-C₁-C₄-alkyl, Arylthio-C₁-C₄-alkyl, Arylsulfinyl-C₁-C₄-alkyl, Arylsulfonyl-C₁-C₄-alkyl,
Heterocyclyl, Heterocyclyl-C₁-C₄-alkyl, Heterocyclyl-C₂-C₄-alkenyl, Heterocyclyl-C₂-C₄-alkinyl, Heterocyclyl-C₁-C₄-halogenalkyl, Heterocyclyl-C₂-C₄-halogen-alkenyl, Heterocyclyl-C₃-C₄-halogenalkinyl, Heterocyclyl-C₁-C₄-hydroxyalkyl, He-terocyclyl-carbonyl-C₁-C₄-alkyl, Heterocyclyl-C₁-C₄-alkyl-carbonyl-C₁-C₄-alkyl, Heterocyclylcarbonyloxy-C₁-C₄-alkyl, Heterocyclyloxycarbonyl-C₁-C₄-alkyl, Hete-rocyclyloxy-C₁-C₄-alkyl, Heterocyclylamino-C₁-C₄-alkyl Heterocyclylthio-C₁-C₄-alkyl, Heterocyclylsulfinyl-C₁-C₄-alkyl, Heterocyclylsulfonyl-C₁-C₄-alkyl, wobei die vorstehend genannten Reste partiell oder vollständig halogeniert sein können und/oder ein bis drei Reste aus der Gruppe Cyano, Nitro, C₁-C₆-Alkyl, C₁-C₆-Halogenalkyl, Hydroxy, C₁-C₆-Hydroxyalkyl, Hydroxycarbonyl-C₁-C₆-alkyl, C₁-C₆-Alkoxycarbonyl-C₁-C₆-alkyl, C₁-C₆-Alkoxy, C₁-C₆-Halogenalkoxy, Hydro-xycarbonyl, C₁-C₆-Alkoxycarbonyl, Aminocarbonyl, (C₁-C₆-Alkyl)aminocarbonyl, Di(C₁-C₆-alkyl)aminocarbonyl, Hydroxycarbonyl-C₁-C₆-alkoxy, C₁-C₆-Alkoxy-carbonyl-C₁-C₆-alkoxy, Amino, C₁-C₆-Alkylamino, Di(C₁-C₆-alkyl)amino, C₁-C₆-Alkylsulfonylamino, C₁-C₆-Halogenalkylsulfonylamino, (C₁-C₆-Alkyl)amino-carbonylamino, Di(C₁-C₆-alkyl)aminocarbonylamino, Aryl und Aryl(C₁-C₆-alkyl) tragen können;
wobei die Reste R¹ und R² voneinander verschieden sind;
- R³: C₁-C₆-Alkyl;
- R⁴: Aryl, welches partiell oder vollständig halogeniert sein kann und/oder ein bis drei Reste aus der Gruppe Cyano, Nitro, C₁-C₆-Alkyl, C₁-C₆-Halogenalkyl, Hydroxy, C₁-C₆-Hydroxyalkyl, C₁-C₆-Alkoxy, C₁-C₆-Halognalkoxy, Hydroxycarbonyl, C₁-C₆-Alkoxycarbonyl, C₁-C₆-Alkylamino, Di(C₁-C₆-alkyl)amino, Aryl und Aryl(C₁-C₆-alkyl) tragen kann;
bedeuten;
und
* für die S oder R-Konfiguration, und
** für die S und/oder R-Konfiguration;
stehen;
indem man das Imin der Formel I in Gegenwart von Wasserstoff und einem heterogenen Kupfer-haltigen Katalysator umsetzt,
dadurch gekennzeichnet, dass der heterogene Kupfer-haltige Katalysator, bezogen auf das Gesamtgewicht des Katalysators

| | |
|---|---|
| 0,1 - 95 Gew.% | Kupfer; |
| 0,1 - 85 Gew.% | mindestens ein Metall ausgewählt aus der Gruppe Nickel, Kobalt und Zink; |
| 0 - 15% Gew.% | mindestens eines Promotors ausgewählt aus der Gruppe Eisen, Rhodium, Ruthenium, Palladium, Platin, Iridium, Osmium, Silber, |
| | Gold, Molybdän, Wolfram, Rhenium, Cadmium, Blei, Mangan, Zinn, Chrom, Lithium, Natrium, Kalium, Cäsium, Magnesium, Barium, Phosphor, Arsen, Antimon, Wismut, Selen und Tellur; |

wobei die Summe der Gew.% 100% nicht übersteigt,
enthält.

Häufig wird in der Literatur die diastereoselektive Umsetzung von chiralen Iminen, wobei der Substituent am Imino-Stickstoff die Chiralität trägt, in Gegenwart von Wasserstoff zu entsprechenden Aminen in Gegenwart von Platinoxid, Palladium auf Kohle etc. beschrieben. EP 443 606 beschreibt die Umsetzung von optisch aktivem 1-Phenyl-ethylamin mit 4-(4-Methoxyphenyl)-2-butanon und anschließende Hydrierung mit Was-serstoff in Gegenwart von Palladium auf Kohle (Beispiel 4).

Weiterhin ist bekannt, dass diastereoselektive Hydrierungen von Iminen, wobei der Substituent am Imino-Stickstoff die Chiralität trägt, zu entsprechenden Aminen in Gegenwart von Nickel-Skelett-Katalysatoren (Raney™-Typ) durchgeführt werden können (WO 2006/008171). EP 443 606 beschreibt ebenfalls die Umsetzung von optisch aktivem 1-Phenylethylamin mit 4-(4-Methoxyphenyl)-2-butanon und anschließende Hydrie-rung mit Wasserstoff in Gegenwart von Raney-Nickel (Beispiel 1 B). WO 2006/030017 offenbart unter anderem Kupfer-haltige Katalysatoren, vorzugsweise vom Raney-Typ, für die diastereoselektive Umsetzung von chiralen Iminen zu den entsprechenden Aminen.

Nachteilig bei diesen Verfahren ist die zum Teil schlechte Diastereoselektivität der Hydrierung und/oder die schwierige Abtrennung des Nickel-Skelett-Katalysators (Raney™-Typ).

Weiterhin wird in WO 01/09080 ein Verfahren zur cis-selektiven Darstellung von cycli-schen Aminen des Sertralin-Typs beschrieben, indem ein cyclisches Keton mit einen achiralen Amin zu dem entsprechenden Imin umgesetzt wird und dieses dann einer katalytischen Hydrierung im Gegenwart eines Kupfer-haltigen Kataysators, insbesondere Kupferchromit, unterworfen wird.

Aufgabe der vorliegenden Erfindung war es ein allgemein anwendbares Verfahren zur diastereoselektiven Umsetzung von chiralen Iminen, wobei also der Substituent am Imino-Stickstoff die Chiralität trägt, zu entsprechenden Aminen zu finden, welches die oben genannten Nachteile nicht aufweist.

Gemäß der voranstehenden Aufgabe wurde gefunden, dass die Diastereoselektivität und/oder der Umsatz bei der Reaktion von chiralen Iminen der Formel I zu Aminen der Formel II, verbessert werden kann, wenn heterogenen Katalysatoren, welche Nickel, Kobalt und/oder Zink enthalten, verwendet werden, welche zusätzlich Kupfer enthalten.

Das erfindungsgemäße Verfahren geht von chiralen Iminen der Formel I aus, wobei der Substituent an Iminostickstoff (-C*HR³R⁴) wahlweise in R oder S-configuration vorliegt.

Die Umsetzung erfolgt in der Regel in einem Lösungsmittel. Es ist aber auch möglich die Umsetzung in Substanz durchzuführen, insbesondere wenn das Imin der Formel I bei der Reaktionstemperatur flüssig ist. Als Lösungsmittel werden unter den Reaktionsbedingungen inerte Lösungsmittel, wie Alkohole, beispielseweise Methanol, Ethanol, n-Propanol, Isopropanol,Cyclopentanol, Cyclohexanol, Ethylenglycol, Propylenglycol etc., aromatische Kohlenwasserstoffe, beispielsweise Benzol, Toluol, Ethylbenzol, Xy-lol etc., chlorierte Kohlenwasserstoffe, beispielsweise Methylenchlorid, Chloroform, Tetrachlorkohlenstoff, 1,2-Dichlormethan, Chlorbenzol etc., Ether, beispielsweise Diethylether, Methyl-tert.-butylether, Ethylengylcoldimethylether, Tetrahydrofuran, Dioxan, dipolar aprotische Lösungsmittel, beispielsweise N-Methylpyrrolidon, Dimethylsulfoxid, Sulfolan, Dimethylformamid, Dimethylacetamid etc., oder Gemische hiervon eingesetzt. Vorzugsweise wird die Umsetzung in einem Alkohol, wie Methanol, Ethanol, n-Propanol, Isopropanol,Cyclopentanol, Cyclohexanol, Ethylenglycol, Propylenglycol etc., vorzugsweise Methanol, Ethanol oder Isopropanol, oder einen aromatischen Koh-lenwasserstoff, wie Benzol, Toluol, Ethylbenzol, Xylol etc., vorzugsweise Toluol oder Ethylbenzol, oder Gemischen hiervon durchgeführt.

Das Gewichtverhältnis von Imin zu Lösungsmittel kann in weiten Bereichen variiert werden. Üblicherweise liegt es im Bereich von 0,01% bis 99%, vorzugsweise 0, 1 % bis 95%, insbesondere 1% bis 90%, besonders bevorzugt 10% bis 70%, außerordentlich bevorzugt 15-60%.

Üblicherweise wird die Umsetzung bei einer Temperatur von Raumtemperatur bis Rückflußtemperatur des Reaktionsgemisches, in der Regel von Raumtemperatur bis 200°C, durchgeführt.

Im allgemeinen wird die Reaktion bei einem Druck von Normaldruckbis 200 bar, vorzugsweise von 40 bis 150 bar, insbesondere von 50 bis 100 bar, durchgeführt. Es ist möglich, den Druck stufenweise bis zum gewünschten Druck zu erhöhen oder auch kontinuierlich.

Der Wasserstoff bzw. der Wasserstoff des Wasserstoff-enthaltende Gasstroms kann vollständig oder teilweise zur Reaktion gebracht werden. In letzteren Fall kann es von Fall zu Fall von Vorteil sein diesen Gasstrom teilweise oder ganz rückzuführen bzw. im Kreis zu führen. Für den Fall, dass der eingesetzte Kupfer-haltige Kataysator vor der Umsetzung aktiviert wird, dann kann dieser Gasstrom auch hierfür verwendet werden.

Üblicherweise wird der Wasserstoff von technischer Qualität eingesetzt. Der Wasserstoff kann aber auch in der Form eines Wasserstoff enthaltenden Gases, d.h. als Beimengung eines Inertgases, wie Stickstoff, Helium, Neon, Argon oder Kohlendioxid, vorzugsweise Stickstoff oder Argon, eingesetzt werden.

Die erfindungsgemäße Umsetzung wird in der Gegenwart eines heterogenen Kupfer-haltigen Katalysators durchgeführt, der bezogen auf das Gesamtgewicht des Katalysa-tors

| | |
|---|---|
| 0,1 - 95 Gew.% | Kupfer ; |
| 0,1 - 85 Gew.% | mindestens ein Metall ausgewählt aus der Gruppe Nickel, Kobalt und Zink; |
| 0 - 15% Gew.% | mindestens eines Promotors ausgewählt aus der Gruppe Eisen, Rhodium, Ruthenium, Palladium, Platin, Iridium, Osmium, Silber, Gold, Molybdän, Wolfram, Rhenium, Cadmium, Blei, Mangan, Zinn, |
| | Chrom, Lithium, Natrium, Kalium, Cäsium, Magnesium, Barium, Phosphor, Arsen, Antimon, Wismut, Selen und Tellur; |

wobei die Summe der Gew.% 100% nicht übersteigt, enthält.

In der Regel enthält der heterogene Kupferhaltige Katalysator ein Trägermaterial. Als Trägermaterial kommt Kohle, z. B. Aktivkohle, Graphit oder Ruß, oder ein poröses Me-talloxid in Betracht. Beispiele für geeignete poröse Metalloxide sind Aluminiumoxid, Siliciumdioxid, Alumosilicate, Titandioxid, Zirkoniumdioxid, Magnesiumoxid oder Gemische hiervon, vorzugsweise Aluminiumoxid, Titandioxid oder Zirkoniumdioxid. Es ist aber auch möglich als Trägermaterialien Aluminiumphosphate, Mullitte, Kieselguhr, Bauxite und Caliumaluminate einzusetzen.

Insbesondere beträgt das Gesamtgewicht der oben genannten katalytisch aktiven Me-talle und ggf. Promotoren des heterogenen Kupfer-haltigen Katalysators bezogen auf sein Gesamtgewicht höchstens 95 Gew.%, vorzugsweise höchstens 90 Gew.%.

In einer weiteren Ausgestaltungsform handelt es sich bei dem heterogene Kupfer-haltige Katalysator um einen Vollkontakt.

In einer Ausgestaltungsform enthält dieser heterogene Kupfer-haltige Katalysator, bezogen auf das Gesamtgewicht des Katalysators

| | |
|---|---|
| 1 - 90 Gew.% | Kupfer; |
| 0,1 - 80 Gew.% | mindestens ein Metall ausgewählt aus der Gruppe Nickel, Kobalt und Zink; |
| 0 - 15% Gew.% | mindestens eines Promotors ausgewählt aus der Gruppe Eisen, Rhodium, Ruthenium, Palladium, Platin, Iridium, Osmium, Silber, Gold, Molybdän, Wolfram, Rhenium, Cadmium, Blei, Mangan, Zinn, Chrom, Lithium, Natrium, Kalium, Cäsium, Magnesium, Barium, Phosphor, Arsen, Antimon, Wismut, Selen und Tellur. |

Insbesondere enthält dieser heterogene Kupfer-haltige Katalysator, bezogen auf das Gesamtgewicht des Katalysators

| | |
|---|---|
| 2 - 85 Gew.% | Kupfer; |
| 0,1 - 80 Gew.% | mindestens ein Metall ausgewählt aus der Gruppe Nickel, Kobalt und Zink; |
| 0 - 15% Gew.% | mindestens eines Promotors ausgewählt aus der Gruppe Eisen, Rhodium, Ruthenium, Palladium, Platin, Iridium, Osmium, Silber, Gold, Molybdän, Wolfram, Rhenium, Cadmium, Blei, Mangan, Zinn, Chrom, Lithium, Natrium, Kalium, Cäsium, Magnesium, Barium, Phosphor, Arsen, Antimon, Wismut, Selen und Tellur. |

In einer weiteren Ausgestaltungsform enthält dieser heterogene Kupfer-haltige Kataly-sator, bezogen auf das Gesamtgewicht des Katalysators

| | |
|---|---|
| 2 - 50 Gew.% | Kupfer; |
| 0 - 30 Gew.% | mindestens ein Metall ausgewählt aus der Gruppe Nickel und Kobalt; |
| 0,5 - 50 Gew. % | Zink; |
| 0 - 5% Gew.% | mindestens einen Promotor ausgewählt aus der Gruppe Eisen, Rhodium, Ruthenium, Palladium, Platin, Iridium, Osmium, Silber, Gold, Molybdän, Wolfram, Rhenium, Cadmium, Blei, Mangan, Zinn, Chrom, Lithium, Natrium, Kalium, Cäsium, Magnesium, Barium, Phosphor, Arsen, Antimon, Wismut, Selen und Tellur. |

Vorzugsweise enthält dieser heterogene Kupfer-haltige Katalysator, bezogen auf das Gesamtgewicht des Katalysators

| | |
|---|---|
| 5 - 40 Gew.% | Kupfer; |
| 0 - 30 Gew.% | mindestens ein Metall ausgewählt aus der Gruppe Nickel und Kobalt; |
| 5 - 50 Gew. % | Zink; |
| 0 - 5% Gew.% | mindestens einen Promotor ausgewählt aus der Gruppe Eisen, Rhodium, Ruthenium, Palladium, Platin, Iridium, Osmium, Silber, Gold, Molybdän, Wolfram, Rhenium, Cadmium, Blei, Mangan, Zinn, Chrom, Lithium, Natrium, Kalium, Cäsium, Magnesium, Barium, Phosphor, Arsen, Antimon, Wismut, Selen und Tellur. |

Insbesondere enthält dieser heterogene Kupfer-haltige Katalysator, bezogen auf das Gesamtgewicht des Katalysators

| | |
|---|---|
| 10 - 35 Gew.% | Kupfer; |
| 0 - 30 Gew.% | mindestens ein Metall ausgewählt aus der Gruppe Nickel und Kobalt; |
| 10 - 45 Gew. % | Zink; |
| 0 - 5% Gew.% | mindestens einen Promotor ausgewählt aus der Gruppe Eisen, Rhodium, Ruthenium, Palladium, Platin, Iridium, Osmium, Silber, Gold, Molybdän, Wolfram, Rhenium, Cadmium, Blei, Mangan, Zinn, Chrom, Lithium, Natrium, Kalium, Cäsium, Magnesium, Barium, Phosphor, Arsen, Antimon, Wismut, Selen und Tellur. |

Besonders bevorzugt enthält dieser heterogene Kupfer-haltige Katalysator, bezogen auf das Gesamtgewicht des Katalysators

| | |
|---|---|
| 10 - 35 Gew.% | Kupfer; |
| 10 - 40 Gew. % | Zink; |
| 0 - 5% Gew.% | mindestens einen Promotor ausgewählt aus der Gruppe Eisen, Rhodium, Ruthenium, Palladium, Platin, Iridium, Osmium, Silber, Gold, Molybdän, Wolfram, Rhenium, Cadmium, Blei, Mangan, Zinn, Chrom, Lithium, Natrium, Kalium, Cäsium, Magnesium, Barium, Phosphor, Arsen, Antimon, Wismut, Selen und Tellur. |

Insbesondere bevorzugt enthält dieser Kupfer-haltige Katalysator, als katalytisch aktive Metalle nur Kupfer und Zink, insbesondere jeweils (aber unabhängig voneinander) 5 bis 50 Gew.%, besonders bevorzugt 10 bis 45 Gew.%, insbesondes bevorzugt 20 bis 40 Gew. %, bezogen auf das Gesamtgewicht des Katalysators. Als Trägermaterial kommt vorzugsweise ein poröses Metalloxid, insbesondere Aluminiumoxid, Titandioxid oder Zirkoniumdioxid, in Betracht.

In einer weiteren Ausgestaltungsform enthält dieser heterogene Kupfer-haltige Kataly-sator, bezogen auf das Gesamtgewicht des Katalysators

| | |
|---|---|
| 2 - 50 Gew.% | Kupfer; |
| 0,1 - 70 Gew.% | Nickel |
| 0 - 30 Gew.% | mindestens ein Metall ausgewählt aus der Gruppe Kobalt und Zink; |
| 0 - 5% Gew.% | mindestens einen Promotor ausgewählt aus der Gruppe Eisen, Rhodium, Ruthenium, Palladium, Platin, Iridium, Osmium, Silber, Gold, Molybdän, Wolfram, Rhenium, Cadmium, Blei, Mangan, Zinn, Chrom, Lithium, Natrium, Kalium, Cäsium, Magnesium, Barium, Phosphor, Arsen, Antimon, Wismut, Selen und Tellur. |

Vorzugsweise enthält dieser heterogene Kupfer-haltige Katalysator, bezogen auf das Gesamtgewicht des Katalysators

| | |
|---|---|
| 2 - 40 Gew.% | Kupfer; |
| 1 - 65 Gew.% | Nickel; |
| 0 - 10 Gew.% | mindestens ein Metall ausgewählt aus der Gruppe Kobalt und Zink; |
| 0 - 5% Gew.% | mindestens einen Promotor ausgewählt aus der Gruppe Eisen, Rhodium, Ruthenium, Palladium, Platin, Iridium, Osmium, Silber, Gold, Molybdän, Wolfram, Rhenium, Cadmium, Blei, Mangan, Zinn, Chrom, Lithium, Natrium, Kalium, Cäsium, Magnesium, Barium, Phosphor, Arsen, Antimon, Wismut, Selen und Tellur. |

Insbesondere enthält dieser heterogene Kupfer-haltige Katalysator, bezogen auf das Gesamtgewicht des Katalysators

| | |
|---|---|
| 2 - 25 Gew.% | Kupfer; |
| 3 - 60 Gew.% | Nickel; |
| 0 - 10 Gew.% | mindestens ein Metall ausgewählt aus der Gruppe Gruppe Kobalt und Zink; |
| 0 - 5% Gew.% | mindestens einen Promotor ausgewählt aus der Gruppe Eisen, Rhodium, Ruthenium, Palladium, Platin, Iridium, Osmium; Silber, Gold, Molybdän, Wolfram, Rhenium, Cadmium, Blei, Mangan, Zinn, Chrom, Lithium, Natrium, Kalium, Cäsium, Magnesium, Barium, Phosphor, Arsen, Antimon, Wismut, Selen und Tellur. |

Besonders bevorzugt enthält dieser heterogene Kupfer-haltige Katalysator, bezogen auf das Gesamtgewicht des Katalysators

| | |
|---|---|
| 2 - 25 Gew.% | Kupfer; |
| 3 - 50 Gew. % | Nickel; |
| 0 - 5% Gew.% | mindestens einen Promotor ausgewählt aus der Gruppe Eisen, Rhodium, Ruthenium, Palladium, Platin, Iridium, Osmium, Silber, Gold, Molybdän, Wolfram, Rhenium, Cadmium, Blei, Mangan, Zinn, Chrom, Lithium, Natrium, Kalium, Cäsium, Magnesium, Barium, Phosphor, Arsen, Antimon, Wismut, Selen und Tellur, vorzugsweise Molybdän. |

Vorzugsweise enthält dieser Kupfer-haltige Katalysator, als katalytisch aktive Metalle nur Kupfer und Nickel, insbesondere jeweils (aber unabhängig voneinander) 2 bis 15 Gew.%, besonders bevorzugt 2 bis 10 Gew.%, insbesonders bevorzugt 3 bis 8 Gew.%, bezogen auf das Gesamtgewicht des Katalysators. Als Trägermaterial kommt vorzugsweise ein poröses Metalloxid, insbesondere Aluminiumoxid, Titandioxid oder Zirkoniumdioxid, in Betracht.

Ebenso vorzugsweise enthält dieser Kupfer-haltige Katalysator, als katalytisch aktive Metalle bzw. Promotoren nur Kupfer, Nickel und Molybdän, insbesondere 2 bis 25 Gew.% Kupfer, 20 bis 60 Gew.% Nickel, 0,01 bis 5 Gew.% Molybdän, besonders bevorzugt 5 bis 20 Gew.% Kupfer, 30 bis 50 Gew.% Nickel, 0,1 bis 2 Gew.% Molybdän, insbesondes bevorzugt 10 bis 15 Gew.% Kupfer, 35 bis 45 Gew.% Nickel, 0,5 bis 1,5 Gew.% Molybdän bezogen auf das Gesamtgewicht des Katalysators. Als Trägermate-rial kommt vorzugsweise ein poröses Metalloxid, insbesondere Aluminiumoxid, Titandioxid oder Zirkoniumdioxid, in Betracht.

In einer weiteren Ausgestaltungsform enthält dieser heterogene Kupfer-haltige Kataly-sator, bezogen auf das Gesamtgewicht des Katalysators

| | |
|---|---|
| 2 - 40 Gew.% | Kupfer; |
| 0,1 - 80 Gew.% | mindestens ein Metall ausgewählt aus der Gruppe Nickel und Kobalt; |
| 0 - 5% Gew.% | mindestens eines Promotors ausgewählt aus der Gruppe Eisen, Rhodium, Ruthenium, Palladium, Platin, Iridium, Osmium, Silber, Gold, Molybdän, Wolfram, Rhenium, Cadmium, Blei, Mangan, Zinn, Chrom, Lithium, Natrium, Kalium, Cäsium, Magnesium, Barium, Phosphor, Arsen, Antimon, Wismut, Selen und Tellur. |

Vorzugsweise enthält dieser heterogene Kupfer-haltige Katalysator, bezogen auf das Gesamtgewicht des Katalysators

| | |
|---|---|
| 2 - 40 Gew.% | Kupfer; |
| 0,1 - 40 Gew.% | Nickel; |
| 0,1 - 40 Gew.% | Kobalt; |
| 0 - 5% Gew.% | mindestens eines Promotors ausgewählt aus der Gruppe Eisen, Rhodium, Ruthenium, Palladium, Platin, Iridium, Osmium, Silber, Gold, Molybdän, Wolfram, Rhenium, Cadmium, Blei, Mangan, Zinn, Chrom, Lithium, Natrium, Kalium, Cäsium, Magnesium, Barium, Phosphor, Arsen, Antimon, Wismut, Selen und Tellur. |

Insbesondere enthält dieser heterogene Kupfer-haltige Katalysator, bezogen auf das Gesamtgewicht des Katalysators

| | |
|---|---|
| 2 - 30 Gew.% | Kupfer; |
| 0,5 - 35 Gew.% | Nickel; |
| 0,5 - 35 Gew.% | Kobalt; |
| 0 - 5% Gew.% | mindestens einen Promotor ausgewählt aus der Gruppe Eisen, Rhodium, Ruthenium, Palladium, Platin, Iridium, Osmium, Silber, Gold, Molybdän, Wolfram, Rhenium, Cadmium, Blei, Mangan, Zinn, Chrom, Lithium, Natrium, Kalium, Cäsium, Magnesium, Barium, Phosphor, Arsen, Antimon, Wismut, Selen und Tellur. |

Besonders bevorzugt enthält dieser heterogene Kupfer-haltige Katalysator, bezogen auf das Gesamtgewicht des Katalysators

| | |
|---|---|
| 2 - 20 Gew.% | Kupfer; |
| 1 - 30 Gew. % | Nickel; |
| 1 - 30 Gew. % | Kobalt; |
| 0 - 5% Gew.% | mindestens einen Promotor ausgewählt aus der Gruppe Eisen, Rhodium, Ruthenium, Palladium, Platin, Iridium, Osmium, Silber, Gold, Molybdän, Wolfram, Rhenium, Cadmium, Blei, Mangan, Zinn, Chrom, Lithium, Natrium, Kalium, Cäsium, Magnesium, Barium, Phosphor, Arsen, Antimon, Wismut, Selen und Tellur, vorzugsweise Molybdän. |

Vorzugsweise enthält dieser Kupfer-haltige Katalysator, als katalytisch aktive Metalle nur Kupfer, Nickel und Kobalt, insbesondere 2 bis 25 Gew.% Kupfer und jeweils unabhängig voneinander 1 bis 35 Gew.% Nickel bzw. Kobalt, besonders bevorzugt 2 bis 20 Gew.% Kupfer und jeweils unabhängig voneinander 10 bis 30 Gew.% Nickel bzw. Ko-balt, insbesondes bevorzugt 5 bis 15 Gew. % Kupfer und jeweils unabhängig voneinander 15 bis 25 Gew.% Nickel bzw. Kobalt, bezogen auf das Gesamtgewicht des Katalysators. Als Trägermaterial kommt vorzugsweise ein poröses Metalloxid, insbesondere Aluminiumoxid, Titandioxid oder Zirkoniumdioxid, in Betracht.

Ebenso insbesondere enthält dieser Kupfer-haltige Katalysator, als katalytisch aktive Metalle nur Kupfer, Nickel und Kobalt, besonders bevorzugt 2 bis 10 Gew.% Kupfer und jeweils unabhängig voneinander 1 bis 10 Gew.% Nickel bzw. Kobalt, insbesondes bevorzugt 2 bis 5 Gew.% Kupfer und jeweils unabhängig voneinander 2 bis 5 Gew.% Nickel bzw. Kobalt, bezogen auf das Gesamtgewicht des Katalysators. Als Trägerma-terial kommt vorzugsweise ein poröses Metalloxid, insbesondere Aluminiumoxid, Titandioxid oder Zirkoniumdioxid, in Betracht.

In einer weiteren Ausgestaltungsform enthält dieser heterogene Kupfer-haltige Kataly-sator, bezogen auf das Gesamtgewicht des Katalysators

| | |
|---|---|
| 2 - 40 Gew.% | Kupfer; |
| 0,1 - 80 Gew.% | Kobalt; |
| 0 - 15% Gew.% | mindestens eines Promotors ausgewählt aus der Gruppe Eisen, Rhodium, Ruthenium, Palladium, Platin, Iridium, Osmium, Silber, Gold, Molybdän, Wolfram, Rhenium, Cadmium, Blei, Mangan, Zinn, Chrom, Lithium, Natrium, Kalium, Cäsium, Magnesium, Barium, Phosphor, Arsen, Antimon, Wismut, Selen und Tellur. |

Insbesonders enthält dieser heterogene Kupfer-haltige Katalysator, bezogen auf das Gesamtgewicht des Katalysators

| | |
|---|---|
| 2 - 20 Gew.% | Kupfer; |
| 2 - 20 Gew.% | Kobalt; |
| 0 - 5% Gew.% | mindestens eines Promotors ausgewählt aus der Gruppe Eisen, Rhodium, Ruthenium, Palladium, Platin, Iridium, Osmium, Silber, Gold, Molybdän, Wolfram, Rhenium, Cadmium, Blei, Mangan, Zinn, Chrom, Lithium, Natrium, Kalium, Cäsium, Magnesium, Barium, Phosphor, Arsen, Antimon, Wismut, Selen und Tellur. |

Besonders bevorzugt enthält dieser heterogene Kupfer-haltige Katalysator, bezogen auf das Gesamtgewicht des Katalysators

| | |
|---|---|
| 2 - 15 Gew.% | Kupfer; |
| 2 - 15 Gew.% | Kobalt; |
| 0 - 5% Gew.% | mindestens einen Promotor ausgewählt aus der Gruppe Eisen, Rhodium, Ruthenium, Palladium, Platin, Iridium, Osmium, Silber, Gold, Molybdän, Wolfram, Rhenium, Cadmium, Blei, Mangan, Zinn, Chrom, Lithium, Natrium, Kalium, Cäsium, Magnesium, Barium, Phosphor, Arsen, Antimon, Wismut, Selen und Tellur. |

Vorzugsweise enthält dieser Kupfer-haltige Katalysator, als katalytisch aktive Metalle nur Kupfer und Kobalt, insbesondere jeweils (aber unabhängig voneinander) 2 bis 15 Gew.%, besonders bevorzugt 3 bis 10 Gew.%, insbesonders bevorzugt 3 bis 8 Gew. %, bezogen auf das Gesamtgewicht des Katalysators. Als Trägermaterial kommt vor-zugsweise ein poröses Metalloxid, insbesondere Aluminiumoxid, Titandioxid oder Zirkoniumdioxid, in Betracht.

In einer weiteren Ausgestaltungsform enthält dieser heterogene Kupfer-haltige Kataly-sator, bezogen auf das Gesamtgewicht des Katalysators

| | |
|---|---|
| 5 - 40 Gew.% | Kupfer; |
| 20 - 80 Gew.% | Kobalt; |
| 0 - 15 Gew.% | mindestens eines Promotors ausgewählt aus der Gruppe Eisen, Rhodium, Ruthenium, Palladium, Platin, Iridium, Osmium, Silber, Gold, Molybdän, Wolfram, Rhenium, Cadmium, Blei, Mangan, Zinn, Chrom, Lithium, Natrium, Kalium, Cäsium, Magnesium, Barium, Phosphor, Arsen, Antimon, Wismut, Selen und Tellur. |

Besonders bevorzugt enthält dieser heterogene Kupfer-haltige Katalysator, bezogen auf das Gesamtgewicht des Katalysators

| | |
|---|---|
| 10 - 25 Gew.% | Kupfer; |
| 40 - 70 Gew.% | Kobalt; |
| 0,1 - 15 Gew.% | mindestens eines Promotors ausgewählt aus der Gruppe Eisen, Rhodium, Ruthenium, Palladium, Platin, Iridium, Osmium, Silber, Gold, Molybdän, Wolfram, Rhenium, Cadmium, Blei, Mangan, Zinn, Chrom, Lithium, Natrium, Kalium, Cäsium, Magnesium, Barium, Phosphor, Arsen, Antimon, Wismut, Selen und Tellur, vorzugsweise Molybdän, Mangan und Phosphor. |

Vorzugsweise enthält dieser Kupfer-haltige Katalysator, als katalytisch aktive Metalle bzw. Promotoren nur Kupfer, Kobalt, Molybdän und Mangan, insbesondere 5 bis 40 Gew.% Kupfer, 30 bis 80 Gew.% Kobalt und jeweils unabhängig voneinander 0,1 bis 15 Gew.% Molybdän, Mangan und Phosphor, besonders bevorzugt 10 bis 35 Gew.% Kupfer, 40 bis 75 Gew.% Kobalt und jeweils unabhängig voneinander 0,5 bis 15 Gew.% Molybdän, Mangan und Phosphor, insbesondes bevorzugt 12 bis 25 Gew.% Kupfer, 45 bis 60 Gew.% Kobalt und jeweils unabhängig voneinander 0,5 bis 15 Gew.% Molybdän, Mangan und Phosphor bezogen auf das Gesamtgewicht des Katalysators. In einer besonderen Ausführungsform handelt es sich hierbei um einen Voll-kontakt.

Der Katalysator weist üblicherweise eine BET-Oberfläche (bestimmt nach DIN 66131) von 50 bis zu 150 m²/g, vorzugsweise von 70 bis 130 m²/g, insbesondere von 75 bis 120 m²/g auf. In der Regel liegt das Porenvolumen des Katalysators (bestimmt mittel Hg-Porosimetrie nach DIN 66133) bei 0,1 bis 0,4 ml/g, vorzugsweise bei 0,15 bis 0,35 ml/g, insbesondere bei 0,15 bis 0,3 ml/g.

Die Herstellung des Katalysators kann aber auch nach üblichen Verfahren erfolgen (A. Farkas, in Ullmann's Encyclopedia of Industrial Chemistry, Electronic Release 2000, Kapitel 5,3, 5,4, 5,6 bis 5,10).

Beispielsweise ist es möglich den Träger aus entsprechenden Verbindungen herzustellen, die sich beim Calcinieren in das Oxid des jeweiligen Trägers umwandeln. Hierfür sind insbesondere Hydroxide, Carbonate und Carboxylate geeignet. Das Oxid bzw. der entsprechende Precursor, der beim Calcinieren in das Oxid des jeweiligen Trägers umgewandelt wird, kann nach an sich bekannten Verfahren, wie z.B. nach dem Sol-Gel-Verfahren, durch Fällung, Entwässern der entsprechenden Carboxylate, Trockenmischen, Aufschlämmen oder Sprühtrocknen, hergestellt werden. Bei der Fällung werden üblicherweise lösliche Salze von Aluminium, Titan, Zirconium etc. eingesetzt, wie z.B. die entsprechenden Halogenide, vorzugsweise Chlorid, Alkoxide, Nitrat etc., vorzugsweise Nitrat von Aluminium, Titan, Zirconium etc. Weiterhin ist es möglich Stabili-satoren in den Träger nach üblichen Verfahren einzubauen. Ebenso können Hilfsmittel in den Träger eingearbeitet werden, welche die Formgebung des Trägers erleichtern, wie z.B. Graphit oder Stearinsäure. Anschließend erfolgt die Formgebung. In der Regel werden Stränge, Tabletten, Kugeln, Splitt, Monolithe etc., nach den üblichen Verfahren hergestellt.

Die Calcinierung erfolgt üblicherweise mit Luft oder einem Gemisch aus Luft und Stickstoff, bei einer Temperatur von 300 bis 800°C, bevorzugt bei 500 bis 600°C. Es kann von Vorteil sein der Luft bzw. dem Luft/Stickstoff-Gemisch Wasserdampf beizumengen.

Auf den Träger können nun die erfindungsgemäßen katalytisch aktiven Metalle bzw. Promotoren aufgebracht werden. Üblicherweise wird der Träger mit einer Lösung eines entsprechenden Metall-Precursors bzw. Promoter-Precursor imprägniert oder in dieser getränkt. Die Imprägnierung kann nach der incipient-wetness Methode erfolgen, wobei das poröse Volumen des Trägers durch in etwa gleiches Volumen an Imprägnierlösung aufgefüllt wird und man - ggf. nach einer Reifung - den Träger trocknet; oder man arbeitet mit einem Überschuss an Lösung, wobei das Volumen dieser Lösung größer ist als das poröse Volumen des Trägers. Hierbei wird der Träger mit in der Imprägnierlösung gemischt und ausreichend lange gerührt. Die überschüssige Imprägnierlösung wird abgeschüttelt, abzentrifugiert oder durch Fitration abgetrennt. Von Fall zu Fall kann auch der Zusatz von Säuren, Neutralsalzen oder Basen die Imprägnierung/Tränkung erleichtern. Eine durchgehende Imprägnierung des Trägers kann von Fall zu Fall erreicht werden, indem z.B. die Lösung während des Imprägnierens/Tränkens erwärmt wird, oberflächenaktive Substanzen zugegeben werden oder der Träger evakuiert wird. Weiterhin ist es möglich den Träger mit einer Lösung des entsprechenden Precursors zu besprühen. Hierbei wird der entsprechende Träger mit einer Lösung des entsprechenden Metall-Precursors bzw. Promoter-Precursor, welche so bemessen ist, dass der Träger die Lösung aufnimmt, behandelt.

Aber auch andere dem Fachmann bekannte Herstellmethoden, wie z.B. Chemical Vapor Deposition, Soltränkung etc. sind möglich.

Geeignete Metall-Precursoren bzw. Promotor-Precursoren sind entsprechende lösliche Metallsalze, u.a. Halogenide, insbesondere Chlorid, Nitrat, Acetat, alkalische Carbonate, Formiat, Oxalat, Citrat, Tartrat.

Die Aufbringung der Metall- bzw. Promotor-Precursoren nach den voranstehend genannten Methoden kann gemeinsam oder nacheinander erfolgen. Es kann auch von Vorteil sein, hierbei eine gewisse Reihenfolge einzuhalten.

Aber auch andere dem Fachmann bekannte Herstellmethoden, wie z.B. Chemical Vapor Deposition, Soltränkung etc. sind möglich.

Der Träger, auf dem die erfindungsgemäßen katalytisch aktiven Metall-Precursoren, aufgetragen wird, wird nun calciniert. Die Calcinierung erfolgt üblicherweise mit Luft oder einem Gemisch aus Luft und Stickstoff, bei einer Temperatur von 300 bis 800°C, bevorzugt bei 400 bis 600 °C. Es kann von Vorteil sein der Luft bzw. dem Luft/Stickstoff-Gemisch Wasserdampf beizumengen.

Nach der Calcinierung wird der heterogene Kupfer-haltige Katalysator zweckmäßiger-weise konditioniert, sei es, dass man ihn durch Vermahlen auf eine bestimmte Komgröße einstellt oder dass man ihn nach seiner Vermahlung mit Formhilfsmitteln wie Graphit oder Stearinsaure vermischt, mittels einer Tablettenpresse zu Formlingen verpreßt und tempert (wärmebehandelt). Die Tempertemperaturen entsprechen dabei im allgemeinen den Temperaturen bei der Calcinierung.

Es können aber auch zur Herstellung der heterogenen Kupfer-haltigen Katalysatoren Fällmethoden angewendet werden. So können diese beispielsweise durch eine gemeinsame Fällung der Metall-/bzw. Promotorprecursoren aus einer diese Metal-le/Promotoren enthaltenden, wässrigen Salzlosung mittels Mineralbasen in Gegenwart einer Aufschlämmung einer schwerlöslichen, sauerstoffhaltigen Trägerprecursorverbindung oder des Trägers selbst und anschließendes Waschen, Trocknen und Calci-nieren des erhaltenen Prazipitats erhalten werden.

Als schwerlösliche, sauerstoffhaltige Trägerprecursorverbindungen bzw. Träger selbst können beispielsweise Oxide, Oxihydrate, Phosphate, Borate und Silikate verwendet werden, beispielsweise Oxide, Oxihydrate, Phosphate, Borate und Silicate, wie z.B. Zirkoniumdioxid, Zirkoniumoxidhydrat, Zirkoniumphosphate, -borate und -silikate, Siliciumdioxid, Aluminiumoxid, Aluminiumoxihydrat, Titandioxid sowie weitere, dem Fachmann bekannte und hierfür geeignete Verbindungen. Die Aufschlammungen der schwerloslichen Trägerprecursorverbindungen bzw. Träger selbst können durch Suspendieren feinkörniger Pulver dieser Trägerprecursorverbindungen bzw. Träger selbst in Wasser unter kräftigem Rühren hergestellt werden. Vorteilhaft werden diese Aufschlämmungen durch Ausfällen der schwerlöslichen Trägerprecursorverbindungen aus wässrigen Salzlösungen mittels Mineralbasen erhalten.

Insbesondere werden die erfindungsgemäßen heterogenen Kupfer-haltigen Katalysatoren über eine gemeinsame Fällung (Mischfällung) aller ihrer Komponenten hergestellt. Dazu wird zweckmäßigerweise eine die Katalysatorkomponenten enthaltende, wässrige Salzlösung in der Wärme und unter Rühren so lange mit einer wässrigen Mineralbase, insbesondere einer Alkalimetallbase - beispielsweise Natriumcarbonat, Natriumhydroxid, Kaliumcarbonat oder Kaliumhydroxid - versetzt, bis die Fällung vollständig ist. Die Art der verwendeten Salze ist im allgemeinen nicht kritisch - da es bei dieser Vorgehensweise vornehmlich auf die Wasserlöslichkeit der Salze ankommt, ist ein Kriterium ihre zur Herstellung dieser verhaltnismäßig stark konzentrierten Salzlösungen erforderliche, gute Wasserlöslichkeit. Es wird als selbstverständlich erachtet, dass bei der Auswahl der Salze der einzelnen Komponenten natürlich nur Salze mit solchen Anionen gewählt werden, die nicht zu Störungen führen, sei es, indem sie unerwünschte Fällungen verursachen oder indem sie durch Komplexbildung die Fällung erschweren oder verhindern.

Die bei diesen Fallungsreaktionen erhaltenen Niederschläge sind im allgemeinen chemisch uneinheitlich und bestehen u.a. aus Mischungen der Oxide, Oxidhydrate, Hydroxide, Carbonate und unlöslichen und basischen Salze der eingesetzten Metal-le/Promotoren. Es kann sich für die Filtrierbarkeit der Niederschläge als günstig erweisen, wenn sie gealtert werden, d.h. wenn man sie noch einige Zeit nach der Fällung, gegebenenfalls in Wärme oder unter Durchleiten von Luft, sich selbst überläßt.

Die nach diesen Fallungsverfahren erhaltenen Niederschläge werden wie üblich zu den erfindungsgemäßen heterogenen Kupfer-haltigen Katalysatoren weiterverarbeitet. Nach dem Waschen werden sie im allgemeinen bei 80 bis 200 °C, vorzugsweise bei 100 bis 150 °C, getrocknet und danach calciniert. Die Calcinierung (Wärmebehand-lung) wird im allgemeinen bei Temperaturen zwischen 300 und 800 °C, vorzugsweise bei 400 bis 600 °C, insbesondere bei 450 bis 550 °C ausgeführt.

Nach der Calcinierung wird der heterogene Kupfer-haltige Katalysator zweckmäßigerweise konditioniert, sei es, dass man ihn durch Vermahlen auf eine bestimmte Korngröße einstellt oder dass man ihn nach seiner Vermahlung mit Formhilfsmitteln wie Graphit oder Stearinsaure vermischt, mittels einer Tablettenpresse zu Formlingen verpreßt und tempert (wärmebehandelt). Die Tempertemperaturen entsprechen dabei im allgemeinen den Temperaturen bei der Calcinierung.

Die auf diese Weise hergestellten heterogen Kupfer-haltigen Katalysatoren enthalten die katalytisch aktiven Metalle/Promotoren in Form eines Gemisches ihrer sauerstoff-haltigen Verbindungen, d.h. insbesondere als Oxide und Mischoxide.

Die auf diese Weise hergestellten heterogen Kupfer-haltigen Katalysatoren können als solche gelagert werden.

Der so erhaltene Katalysator kann vor seinem Einsatz, in der diaselektiven Hydrierung von Verbindungen der Formel I, aktiviert werden. Hierzu wird er mit Wasserstoff oder einem Gemisch aus Wasserstoff und Stickstoff bei Temperaturen von 100 bis 300°C, behandelt. Hierbei kann es von Vorteil sein, mit einem niedrigen Wasserstoffanteil in dem Wasserstoff/Stickstoff-Gemisch zu beginnen und den Wasserstoffanteil kontinuierlich oder stufenweise im Verlauf des Aktivierungsprozesses zu erhöhen. Die Vorreduktion kann z.B. zunächst bei 150 bis 200°C über einen Zeitraum von 12 bis 20 Stunden in einer Stickstoff/Wasserstoffatmosphäre durchgeführt werden und anschließend noch bis ca. 24 Stunden bei 200 bis 300°C in einer Wasserstoffatmosphäre weitergeführt werden.

Die Aktivierung des Katalysators wird in der Regel in dem Reaktor, in der die erfindungsgemäße Hydrierung erfolgen soll, durchgeführt. Es ist aber auch möglich die Aktivierung des Katalysators vor dem Einbau in den entsprechenden Reaktor vorzunehmen.

Üblicherweise wird der Katalysator in reduzierter Form in die erfindungsgemäße Hydrierung eingesetzt. Hierbei kann es von Vorteil sein, den in reduzierter Form vorliegenden Katalysator nochmals zu aktivieren. Hierzu wird er mit Wasserstoff oder einem Gemisch aus Wasserstoff und einem Inertgas, wie z.B. Stickstoff, bei Temperaturen von Raumtemperatur bis 300 °C, vorzugsweise bei 150 bis 300 °C, und einem Wasserstoffdruck von 10 bis 60 bar, vorzugsweise bei max. 50 bar, behandelt. Hierbei kann es von Vorteil sein, mit Wasserstoff ohne Inertgas zu aktivieren. Es kann aber auch von Vorteil sein mit einem Gemisch von Wasserstoff und Inertgas zu aktivieren, wobei mit dem Wasserstoff/Inertgas-Gemisch zu beginnen und den Wasserstoffanteil kontinuierlich im Verlauf des Aktivierungsprozesses zu erhöhen.

Es ist aber auch möglich den Katalysator, in seiner oxidischen Form oder auch in seiner reduzierten Form, ohne weitere vorangehende Aktivierung in die diaselektiven Hydrierung von Iminen der Formel I einzusetzen.

Das erfindungsgemäße Verfahren kann diskontinuierlich, semikontinuierlich oder kontinuierlich durchgeführt werden.

Bei einer diskontinuierlichen Verfahrensweise wird das Rektionsgemisch nach üblichen Verfahren aufgearbeitet, indem man z.B. den Katalysator abtrennt, beispielsweise durch Filtration, Absetzen lassen und Abtrennen der flüssigen Phase oder durch Zentrifugation, und von dem so erhaltenen Fitrat, Überstand bzw. Zentrifugat wird das Lösungsmittel, z.B. durch abdestillieren, entfernt.

Die erfindungsgemäße Hydrierung wird in den den Fachmann bekannten Hydrierreaktoren durchgeführt. Beispiele hierfür sind u.a. sogenannte Slurry-Reaktoren, Tricklebed Reaktoren und Blasensäulen (P.N. Rylander, Ullmann's Encyclopedia, Electronic Relese 2007, Kapitel Hydrogenation and Dehydrogenation, p. 2-3).

In der Regel wird die Hydrierung der Imine der Formel I in der Flüssigphase, beispielsweise in einem Rührautoklaven, einer Blasensäule, einem Umlaufreaktor, wie etwa einem Schlaufenreaktor oder einem Festbettreaktor, erfolgen. Der Festbettreaktor kann sowohl in der Sumpf- als auch in der Rieselfahrweise betrieben werden.

Es ist aber auch möglich, insbesondere wenn das Imin der Formel I eine gewisse Flüchtigkeit zeigt, die Hydrierung ohne Lösungsmittel in der Gasphase durchzuführen. Hierfür eignen sich beispielsweise Festbettreaktoren oder Wirbelbettreaktoren.

Der Reaktionsaustrag kann nach den üblichen Methoden aufgearbeitet und gereinigt werden. Hierfür kommen beispielsweise Destillation, Flüssigextraktion und/oder Kristallisation in Betracht.

Die mit dem erfindungsgemäßen Verfahren erhaltenen Verbindungen der Formel II haben einen Diastereomerenverhätnis von > 0,70, vorzugsweise > 0,9, insbesondere > 0,95, außerordentlich bevorzugt von > 0,98 (wobei das Diasteromerenverhätnis das mol-Verhältnis von gewünschtem Diastereomeren : unerwünschten Diastereomeren ist). Falls es wünschenswert ist einen noch höheresDiasterostereomerenverhätnis zu erzielen, so kann das Diasterostereomerenverhätnis der mit dem erfindungsgemäßen Verfahren erhaltenen Verbindungen der Formel II durch bekannte Verfahren, wie z.B. Umkristallisation, erhöht werden.

Das Diastereomerenverhältnis kann nach dem Fachmann üblichen Verfahren bestimmt werden, üblicherweise wird dies indirekt über den Drehwert oder direkt durch Gas- bzw. Flüssigchromatographie bestimmt. Die Bestimmung kann direkt oder über entsprechende Derivate der Zielverbindung erfolgen.

Der Katalysator kann wieder in das erfindungsgemäße Verfahren eingesetzt werden.

Die Verbindungen der Formel I sind bekannt oder können nach literaturbekannten Verfahren hergestellt werden.

Beispielsweise ist es möglich, die Verbindungen der Formel I durch Umsetzung von Ketonen der Formel III mit Aminen der Formel IV, wobei die Reste R¹ bis R⁴, die bei den Verbindungen der Formel I angegebene Bedeutung haben, zu erhalten.

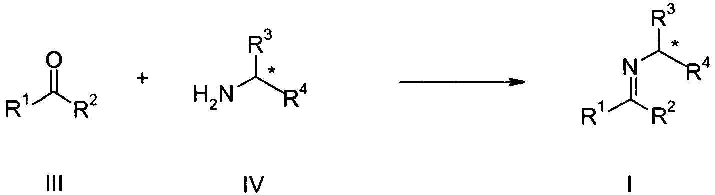

Üblicherweise werden hierbei das Keton der Formel III und das Amin der Formel IV in stöchiometrischen Mengen eingesetzt. Von Fall zu Fall kann es auch von Vorteil sein das eine oder das andere Edukt im Überschuss einzusetzen. In der Regel wird die Umsetzung in einem Lösungsmittel durchgeführt. Als Lösungsmittel eignen sich inerte Lösungsmittel, wie beispielsweise Alkohole, Ether, Kohlenwasserstoffe, halogenierte Kohlenwasserstoffe etc., insbesondere solche, welche mit Wasser ein Azeotrop, wie z.B. Toluol oder Ethylbenzol, ausbilden und so das bei der Reaktion entstehende Wasser abgetrennt werden kann. Bildet eines der eingesetzten Edukte mit Wasser ein azeotropes Gemisch, so kann dieses Edukt im Überschuss eingesetzt werden, und das gebildete Wasser azeotrop abgetrennt werden. Dies kann in Gegenwart oder in Abwesenheit eines zusätzlichen Lösungsmittels erfolgen. Weiterhin kann es von Fall zu Fall vorteilhaft sein katalytische Mengen von Säure, wie z.B. p-Toluolsulfonsäure, zuzusetzen. Die Umsetzung kann auch in Gegenwart von heterogenen Katalysatoren, wie zum Beispiel Aluminiumoxide, Titandioxid, Zirkondioxid, Siliciumoxide oder Tonmineralien wie Montmorillonit, durchgeführt werden.

Von Fall zu Fall kann es auch vorteilhaft sein das bei der Umsetzung freiwerdende Wasser mit Molekularsieb abzufangen. Alternativ kann es auch von Vorteil sein, das bei der Reaktion gebildete Wasser abzudestilieren. Die Umsetzung findet üblicherweise bei einer Temperatur von Raumtemperatur bis Rückflußtemperatur des Reaktionsgemisches statt. Die Aufarbeitung des Reaktionsgemisches erfolgt nach den dem Fachmann bekannten Methoden.

Bei der voranstehend genannten Umsetzung kann anstelle des chiralen Amins der Formel IV auch ein entsprechendes Racemat eingesetzt werden. Nach erfolgter Umsetzung und ggf. Aufarbeitung wird eine Racemat-Trennung nach den dem Fachmann bekannten Methoden durchgeführt.

Die Verbindungen der Formel I können auch erhalten werden, indem man ein Alkin der Formel V mit einem Amin der Formel IV umsetzt.

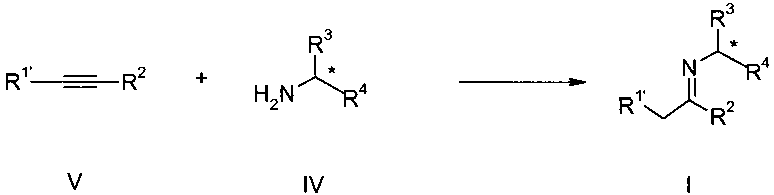

Die Bedeutungen der Reste R² bis R⁴ entsprechen denjenigen, welche für die Verbindungen der Formel I angegeben sind. Und R^{1'}-CH₂ steht für die Bedeutungen von R¹ welche kompatibel hiermit sind. Entsprechendes gilt für R^{1'}.

Üblicherweise werden das Alkin der Formel V und das Amin der Formel IV im stöchiometrischen Verhältnis eingesetzt. Es kann aber von Vorteil sein das Alkin der Formel V im Überschuß einzusetzten. Die Umsetzung wird in der Regel in einem inerten Lösungsmittel, wie z.B. einem Ether, einem Kohlenwasserstoff, einen halogenierten Kohlenwasserstoff etc., oder Gemischen hiervon, bei Raumtemperatur bis Rückflußtemperatur des Reaktionsgemisches durchgeführt. In der Regel wird die Umsetzung bei Normaldruck durchgeführt. Von Fall zu Fall kann es aber auch von Vorteil sein die Umsetzung bei erhöhten Druck, vorzugsweise im Bereich von 10 bis 200 bar durchzuführen. Nach erfolgter Umsetzung wird nach den für den Fachmann bekannten Methoden aufgearbeitet.

Bei der voranstehend genannten Umsetzung kann anstelle des chiralen Amins der Formel IV auch ein entsprechendes Racemat eingesetzt werden. Nach erfolgter Umsetzung und ggf. Aufarbeitung wird eine Racemat-Trennung nach den dem Fachmann bekannten Methoden durchgeführt.

Ebenso ist es möglich die Imine der Formel I durch Umsetzung von Nitroso-Verbindungen der Formel VII mit Phosphoryliden der Formel VI herzustellen.

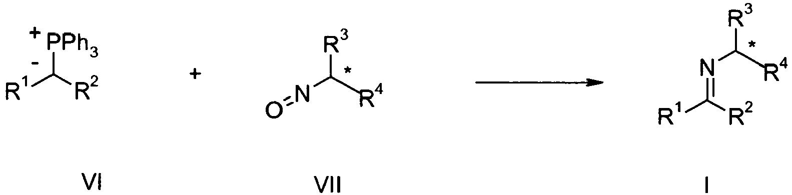

Die Reste R¹ bis R⁴ haben die unter den Verbindungen der Formel I genannte Bedeutung.

Üblicherweise werden das Phosphorylid der Formel VI und die Nitrosoverbindung der der Formel VII im stöchiometrischen Verhältnis eingesetzt. Von Fall zu Fall kann es jedoch auch von Vorteil sein die eine oder andere Reaktionskomponente im Überschuß oder im Unterschuß einzusetzten. Die Umsetzung wird in der Regel in einem inerten Lösungsmittel, wie z.B. einem Ether, einem Kohlenwasserstoff, einen halogenierten Kohlenwasserstoff etc., oder Gemischen hiervon, bei Raumtemperatur bis Rückflußtemperatur des Reaktionsgemisches durchgeführt. In der Regel wird die Umsetzung bei Normaldruck durchgeführt. Nach erfolgter Umsetzung wird nach den für den Fachmann bekannten Methoden aufgearbeitet.

Bei der voranstehend genannten Umsetzung kann anstelle der chiralen Nitrosoverbindung der Formel VII auch ein entsprechendes Racemat eingesetzt werden. Nach erfolgter Umsetzung und ggf. Aufarbeitung wird eine Racemat-Trennung nach den dem Fachmann bekannten Methoden durchgeführt.

Die Darstellung der Imine der Formel I kann kontinuierlich, semi-kontinuierlich oder diskontinuierlich durchgeführt werden.

Vorzugsweise werden bei dem erfindungsgemäßen Verfahren Imine der Formel I eingesetzt, bzw. Amine der Formel II dargestellt, wobei die Reste unabhängig voneinander folgende Bedeutungen haben:
- R¹, R²: C₁-C₆-Alkyl, C₃-C₆-Cycloalkyl, C₂-C₆-Alkenyl, C₂-C₆-Alkinyl, C₂-C₆-Halogen-alkenyl, C₂-C₆-Halogenalkinyl oder C₁-C₆-Alkylcarbonyl-C₁-C₆-alkyl, wobei die genannten Alkyl-, Cycloalkyl- und Alkoxyreste partiell oder vollständig halogeniert sein können und/oder eine bis drei der folgenden Gruppen tragen können: Cyano, Hydroxy, C₁-C₄-Alkyl, C₃-C₆-Cycloalkyl, C₁-C₆-Alkoxy-C₁-C₄-alkyl, C₁-C₄-Alkoxy-C₁-C₄-alkoxy-C₁-C₄-alkyl, C₁-C₄-Alkoxy, C₁-C₄-Alkylthio, Amino, C₁-C₄-alkyl-amino, Di(C₁-C₄-alkyl)amino-,
Aryl, Aryl-C₁-C₄-alkyl, Aryl-C₂-C₄-alkenyl, Aryl-C₂-C₄-alkinyl, Aryl-C₁-C₄-halogenalkyl, Aryl-C₂-C₄-halogenalkenyl, Aryl-C₃-C₄-halogenalkinyl, Aryl-C₁-C₄-hydroxyalkyl, Arylcarbonyl-C₁-C₄-alkyl, Aryl-C₁-C₄-alkyl carbonyl-C₁-C₄-alkyl, Ary-loxy-C₁-C₄-alkyl, Arylamino-C₁-C₄-alkyl, Arylthio-C₁-C₄-alkyl, Arylsulfinyl-C₁-C₄-alkyl, Arylsulfonyl-C₁-C₄-alkyl,
Heterocyclyl, Heterocyclyl-C₁-C₄-alkyl, Heterocyclyl-C₂-C₄-alkenyl, Heterocyclyl-C₂-C₄-alkinyl, Heterocyclyl-C₁-C₄-halogenalkyl, Heterocyclyl-C₂-C₄-halogen-alkenyl, Heterocyclyl-C₃-C₄-halogenalkinyl, Heterocyclyl-C₁-C₄-hydroxyalkyl, He-terocydylcarbonyl-C₁-C₄-alkyl, Heterocyclyl-C₁-C₄-alkylcarbonyl-C₁-C₄-alkyl, He-terocyclyloxy-C₁-C₄-alkyl, Heterocyclylamino-C₁-C₄-alkyl, Heterocyclylthio-C₁-C₄-alkyl, Heterocyclylsulfinyl-C₁-C₄-alkyl, Heterocyclylsulfonyl-C₁-C₄-alkyl,
wobei die vorstehend genannten Reste partiell oder vollständig halogeniert sein können und/oder ein bis drei Reste aus der Gruppe Cyano, Nitro, C₁-C₆-Alkyl, C₁-C₆-Halogenalkyl, Hydroxy, C₁-C₆-Hydroxyalkyl, C₁-C₆-Alkoxy, C₁-C₆-Halogenalkoxy, C₁-C₆-Alkoxycarbonyl, Di(C₁-C₆-alkyl)aminocarbonyl, Amino, C₁-C₆-Alkylamino, Di(C₁-C₆-alkyl)amino, C₁-C₆-Alkylsulfonylamino, C₁-C₆-Halogenalkylsulfonylamino, Aryl und Aryl(C₁-C₆-alkyl) tragen können;
- R³: C₁-C₄-Alkyl, vorzugsweise Methyl; und
- R⁴: Aryl, welches partiell oder vollständig halogeniert sein kann und/oder ein bis drei Reste aus der Gruppe Cyano, Nitro, C₁-C₆-Alkyl, C₁-C₆-Halogenalkyl, C₁-C₆-Alkoxy, C₁-C₆-Halogenalkoxy, Aryl und Aryl(C₁-C₆-alkyl) tragen kann; vorzugsweise Phenyl oder 1-Naphthyl;

Insbesondere werden bei dem erfindungsgemäßen Verfahren Imine der Formel I ein-gesetzt, bzw. Amine der Formel II dargestellt, wobei die Reste unabhängig voneinander folgende Bedeutungen haben:
- R¹, R²: C₁-C₆-Alkyl oder C₃-C₆-Cycloalkyl, wobei die genannten Alkyl- oder Cyclo-alkylreste partiell oder vollständig halogeniert sein können und/oder eine bis drei der folgenden Gruppen tragen können: Cyano, Hydroxy, C₃-C₆-Cycloalkyl, C₁-C₆-Alkoxy-C₁-C₄-alkyl, C₁-C₄-Alkoxy-C₁-C₄-alkoxy-C₁-C₄-alkyl, C₁-C₄-Alkoxy, C₁-C₄-Alkylthio, Amino, C₁-C₄-alkyl-amino, Di(C₁-C₄-alkyl)amino, C₁-C₄-Alkyl carbo-nylamino, Hydroxycarbonyl, C₁-C₄-Alkoxycarbonyl, Aminocarbonyl, C₁-C₄-Alkylaminocarbonyl, Di(C₁-C₄-alkyl)aminocarbonyl oder C₁-C₄-Alkylcarbonyloxy; insbesondere C₁-C₆-Alkyl oder C₃-C₆-Cycloalkyl, wobei die genannten Alkyl- o-der Cycloalkylreste partiell oder vollständig halogeniert sein können und/oder ei-ne der folgenden Gruppen tragen können: Cyano, C₁-C₄-Alkoxy, C₁-C₄-Alkylthio, Di(C₁-C₄-alkyl)amino, C₁-C₄-Alkoxycarbonyl, Di(C₁-C₄-alkyl)aminocarbonyl oder C₁-C₄-Alkylcarbonyloxy; ebenso insbesondere C₁-C₆-Alkyl oder C₃-C₆-Cycloalkyl, wobei die genannten Alkyl- oder Cycloalkylreste partiell oder vollständig halogeniert sein können und/oder eine bis drei der folgenden Gruppen tragen können: Cyano, C₁-C₄-Alkoxycarbonyl, Di(C₁-C₄-alkyl)aminocarbonyl oder C₁-C₄-Alkylcarbonyloxy; besonders bevorzugt C₁-C₆-Alkyl, wobei der genannten Alkyl-Rest partiell oder vollständig halogeniert sein kann und/oder eine bis drei der folgenden Gruppen tragen können: Cyano, Di(C₁-C₄-alkyl)amino, C₁-C₄-Alkoxycarbonyl, Di(C₁-C₄-alkyl)aminocarbonyl oder C₁-C₄-Alkylcarbonyloxy; außerordentlich bevorzugt C₁-C₆-Alkyl;
Phenyl, Phenyl-C₁-C₄-alkyl, Phenyl-C₂-C₄-alkenyl, Phenyl-C₂-C₄-alkinyl, Phenyl-C₁-C₄-halogenalkyl, Phenyl-C₂-C₄-halogenalkenyl, Phenyl-C₃-C₄-halogenalkinyl, Phenyl-C₁-C₄-hydroxyalkyl, Phenylcarbonyl-C₁-C₄-alkyl, Phenyl-C₁-C₄-alkyl-carbonyl-C₁-C₄-alkyl, Phenylcarbonyloxy-C₁-C₄-alkyl, Phenyloxycarbonyl-C₁-C₄-alkyl, Phenyloxy-C₁-C₄-alkyl, Phenylamino-C₁-C₄-alkyl, Phenylthio-C₁-C₄-alkyl, Phenylsulfinyl-C₁-C₄-alkyl, Phenylsulfonyl-C₁-C₄-alkyl, Heterocyclyl, Heterocyclyl-C₁-C₄-alkyl, Heterocyclyl-C₂-C₄-alkenyl, Heterocyclyl-C₂-C₄-alkinyl, Heterocyclyl-C₁-C₄-halogenalkyl, Heterocyclyl-C₂-C₄-halogen-alkenyl, Heterocyclyl-C₃-C₄-halogenalkinyl, Heterocyclyl-C₁-C₄-hydroxyalkyl, He-terocyclylcarbonyl-C₁-C₄-alkyl, Heterocyclyl-C₁-C₄-alkylcarbonyl-C₁-C₄-alkyl, He-terocyclylcarbonyloxy-C₁-C₄-alkyl, Heterocyclyloxycarbonyl-C₁-C₄-alkyl, Heterocyclyloxy-C₁-C₄-alkyl, Heterocyclylamino-C₁-C₄-alkyl, Heterocyclylthio-C₁-C₄-alkyl, Heterocyclylsulfinyl-C₁-C₄-alkyl, Heterocyclylsulfonyl-C₁-C₄-alkyl, wobei die vorstehend genannten Reste partiell oder vollständig halogeniert sein können und/oder ein bis drei Reste aus der Gruppe Cyano, Nitro, C₁-C₆-Alkyl, C₁-C₆-Halogenalkyl, Hydroxy, C₁-C₆-Hydroxyalkyl, C₁-C₆-Alkoxy, C₁-C₆-Halogenalkoxy, Hydroxycarbonyl, C₁-C₆-Alkoxycarbonyl, Aminocarbonyl, (C₁-C₆-Alkyl)aminocarbonyl, Di(C₁-C₆-alkyl)aminocarbonyl, Hydroxycarbonyl-C₁-C₆-alkoxy, C₁-C₆-Alkoxycarbonyl-C₁-C₆-alkoxy, Amino, C₁-C₆-Alkylamino, Di(C₁-C₆-alkyl)amino, C₁-C₆-Alkylsulfonylamino, C₁-C₆-Halogenalkylsulfonylamino, (C₁-C₆-Alkyl)aminocarbonylamino, Di(C₁-C₆-alkyl)aminocarbonylamino, Aryl und Aryl(C₁-C₆-alkyl) tragen können;
insbesondere Phenyl, Phenyl-C₁-C₄-alkyl, Phenyl-C₂-C₄-alkenyl, Phenyl-C₁-C₄-halogenalkyl, Phenyl-C₁-C₄-hydroxyalkyl, Phenylcarbonyl-C₁-C₄-alkyl, Phenyl-C₁-C₄-alkylcarbonyl-C₁-C₄-alkyl, Phenylcarbonyloxy-C₁-C₄-alkyl, Phenyloxycarbonyl-C₁-C₄-alkyl, Phenyloxy-C₁-C₄-alkyl, Phenylamino-C₁-C₄-alkyl, Phenylthio-C₁-C₄-alkyl, Phenylsulfonyl-C₁-C₄-alkyl,
Heterocyclyl, Heterocyclyl-C₁-C₄-alkyl, Heterocyclyl-C₂-C₄-alkenyl, Heterocyclyl-C₁-C₄-halogenalkyl, Heterocyclyl-C₁-C₄-hydroxyalkyl, Heterocyclylcarbonyl-C₁-C₄-alkyl, Heterocyclyl-C₁-C₄-alkylcarbonyl-C₁-C₄-alkyl, Heterocyclylcarbonyloxy-C₁-C₄-alkyl, Heterocyclyloxycarbonyl-C₁-C₄-alkyl, Heterocyclyloxy-C₁-C₄-alkyl, Heterocyclylamino-C₁-C₄-alkyl, Heterocyclylthio-C₁-C₄-alkyl, Heterocyclylsulfonyl-C₁-C₄-alkyl,
wobei die vorstehend genannten Reste partiell oder vollständig halogeniert sein können und/oder ein bis drei Reste aus der Gruppe Cyano, Nitro, C₁-C₆-Alkyl, C₁-C₆-Halogenalkyl, Hydroxy, C₁-C₆-Hydroxyalkyl, C₁-C₆-Alkoxy, C₁-C₆-Halogenalkoxy, Hydroxycarbonyl, C₁-C₆-Alkoxycarbonyl, Aminocarbonyl, (C₁-C₆-Alkyl)aminocarbonyl, Di(C₁-C₆-alkyl)aminocarbonyl, Hydroxycarbonyl-C₁-C₆-alkoxy, Di(C₁-C₆-alkyl)amino, Di(C₁-C₆-alkyl)aminocarbonylamino, Aryl und A-ryl(C₁-C₆-alkyl) tragen können; besonders bevorzugt Phenyl, Phenyl-C₁-C₄-alkyl, Phenyl-C₁-C₄-halogenalkyl, Phenylcarbonyloxy-C₁-C₄-alkyl, Phenyloxycarbonyl-C₁-C₄-alkyl, Phenyloxy-C₁-C₄-alkyl,
Heterocyclyl, Heterocyclyl-C₁-C₄-alkyl, Heterocyclyl-C₁-C₄-halogenalkyl, , Heterocyclylcarbonyloxy-C₁-C₄-alkyl, Heterocyclyloxycarbonyl-C₁-C₄-alkyl, Heterocyclyloxy-C₁-C₄-alkyl,
wobei die vorstehend genannten Reste partiell oder vollständig halogeniert sein können und/oder ein bis drei Reste aus der Gruppe Cyano, C₁-C₆-Alkyl, C₁-C₆-Halogenalkyl, C₁-C₆-Alkoxy, C₁-C₆-Halogenalkoxy, Hydroxycarbonyl, C₁-C₆-Alkoxycarbonyl, Aminocarbonyl, (C₁-C₆-Alkyl)aminocarbonyl, Di(C₁-C₆-alkyl)-aminocarbonyl, Di(C₁-C₆-alkyl)amino, Aryl und Aryl(C₁-C₆-alkyl) tragen können;
- R³: C₁-C₄-Alkyl, vorzugsweise Methyl; und
- R⁴: Aryl, welches partiell oder vollständig halogeniert sein kann und/oder ein bis drei Reste aus der Gruppe Cyano, Nitro, C₁-C₆-Alkyl, C₁-C₆-Halogenalkyl, C₁-C₆-Alkoxy, C₁-C₆-Halogenalkoxy, Aryl und Aryl(C₁-C₆-alkyl) tragen kann; vorzugsweise Phenyl oder 1-Naphthyl;

Besonders bevorzugt werden bei dem erfindungsgemäßen Verfahren Imine der Formel I eingesetzt, bzw. Amine der Formel II dargestellt, wobei die Reste unabhängig voneinander folgende Bedeutungen haben:
- R¹, R²: C₁-C₆-Alkyl oder C₃-C₆-Cycloalkyl, wobei die genannten Alkyl- oder Cyclo-alkylreste partiell oder vollständig halogeniert sein können und/oder eine bis drei der folgenden Gruppen tragen können: Cyano, Hydroxy, C₃-C₆-Cycloalkyl, C₁-C₆-Alkoxy-C₁-C₄-alkyl, C₁-C₄-Alkoxy-C₁-C₄-alkoxy-C₁-C₄-alkyl, C₁-C₄-Alkoxy, C₁-C₄-Alkylthio, Amino, C₁-C₄-alkyl-amino, Di(C₁-C₄-alkyl)amino, C₁-C₄-Alkyl-carbonylamino, Hydroxycarbonyl, C₁-C₄-Alkoxycarbonyl, Aminocarbonyl, C₁-C₄-Alkylaminocarbonyl, Di(C₁-C₄-alkyl)aminocarbonyl oder C₁-C₄-Alkylcarbonyloxy; insbesondere C₁-C₆-Alkyl oder C₃-C₆-Cycloalkyl, wobei die genannten Alkyl- o-der Cycloalkylreste partiell oder vollständig halogeniert sein können und/oder ei-ne der folgenden Gruppen tragen können: Cyano, C₁-C₄-Alkoxy, C₁-C₄-Alkylthio, Di(C₁-C₄-alkyl)amino, C₁-C₄-Alkoxycarbonyl, Di(C₁-C₄-alkyl)aminocarbonyl oder C₁-C₄-Alkylcarbonyloxy;
ebenso insbesondere C₁-C₆-Alkyl oder C₃-C₆-Cycloalkyl, wobei die genannten Alkyl- oder Cycloalkylreste partiell oder vollständig halogeniert sein können und/oder eine bis drei der folgenden Gruppen tragen können: Cyano, C₁-C₄-Alkoxycarbonyl, Di(C₁-C₄-alkyl)aminocarbonyl oder C₁-C₄-Alkylcarbonyloxy; besonders bevorzugt C₁-C₆-Alkyl, wobei der genannten Alkyl-Rest partiell oder vollständig halogeniert sein kann und/oder eine bis drei der folgenden Gruppen tragen können: Cyano, Di(C₁-C₄-alkyl)amino, C₁-C₄-Alkoxycarbonyl, Di(C₁-C₄-alkyl)aminocarbonyl oder C₁-C₄-Alkylcarbonyloxy; außerordentlich bevorzugt C₁-C₆-Alkyl;
- R³: C₁-C₄-Alkyl, vorzugsweise Methyl; und
- R⁴: Aryl, welches partiell oder vollständig halogeniert sein kann und/oder ein bis drei Reste aus der Gruppe Cyano, Nitro, C₁-C₆-Alkyl, C₁-C₆-Halogenalkyl, C₁-C₆-Alkoxy, C₁-C₆-Halogenalkoxy, Aryl und Aryl(C₁-C₆-alkyl) tragen kann; vorzugsweise Phenyl oder 1-Naphthyl, welches partiell oder vollständig haloge-niert sein kann und/oder ein bis drei Reste aus der Gruppe Cyano, Nitro, C₁-C₆-Alkyl, C₁-C₆-Halogenalkyl, C₁-C₆-C₁-C₆-Alkoxy, C₁-C₆-Halogenalkoxy, Aryl und Aryl(C₁-C₆-alkyl) tragen kann; insbesondere Phenyl oder 1-Naphthyl.

Ebenso besonders bvorzugt werden bei dem erfindungsgemäßen Verfahren Imine der Formel I eingesetzt, bzw. Amine der Formel II dargestellt, wobei die Reste unabhängig voneinander folgende Bedeutungen haben:
- R¹: C₁-C₆-Alkyl oder C₃-C₆-Cycloalkyl, wobei die genannten Alkyl- oder Cycloalkyl-reste partiell oder vollständig halogeniert sein können und/oder eine bis drei der folgenden Gruppen tragen können: Cyano, Hydroxy, C₃-C₆-Cycloalkyl, C₁-C₆-Alkoxy-C₁-C₄-alkyl, C₁-C₄-Alkoxy-C₁-C₄-alkoxy-C₁-C₄-alkyl, C₁-C₄-Alkoxy, C₁-C₄-Alkylthio, Amino, C₁-C₄-alkyl-amino, Di(C₁-C₄-alkyl)amino, C₁-C₄-Alkyl ycarbony-lamino, Hydroxycarbonyl, C₁-C₄-Alkoxycarbonyl, Aminocarbonyl, C₁-C₄-Alkyl-aminocarbonyl, Di(C₁-C₄-alkyl)aminocarbonyl oder C₁-C₄-Alkylcarbonyloxy, insbesondere C₁-C₆-Alkyl oder C₃-C₆-Cycloalkyl, wobei die genannten Alkyl- o-der Cycloalkylreste partiell oder vollständig halogeniert sein können und/oder ei-ne der folgenden Gruppen tragen können: Cyano, C₁-C₄-Alkoxy, C₁-C₄-Alkylthio, Di(C₁-C₄-alkyl)amino, C₁-C₄-Alkoxycarbonyl, Di(C₁-C₄-alkyl)aminocarbonyl oder C₁-C₄-Alkylcarbonyloxy;
ebenso insbesondere C₁-C₆-Alkyl oder C₃-C₆-Cycloalkyl, wobei die genannten Alkyl- oder Cycloalkylreste partiell oder vollständig halogeniert sein können und/oder eine bis drei der folgenden Gruppen tragen können: Cyano, C₁-C₄-Alkoxycarbonyl, Di(C₁-C₄-alkyl)aminocarbonyl oder C₁-C₄-Alkylcarbonyloxy; besonders bevorzugt C₁-C₆-Alkyl, wobei der genannten Alkyl-Rest partiell oder vollständig halogeniert sein kann und/oder eine bis drei der folgenden Gruppen tragen können: Cyano, Di(C₁-C₄-alkyl)amino, C₁-C₄-Alkoxycarbonyl, Di(C₁-C₄-alkyl)aminocarbonyl oder C₁-C₄-Alkylcarbonyloxy; außerordentlich bevorzugt C₁-C₆-Alkyl;
- R²: Aryl, Aryl-C₁-C₄-alkyl, Aryl-C₂-C₄-alkenyl, Aryl-C₂-C₄-alkinyl, Aryl-C₁-C₄-halogen-alkyl, Aryl-C₂-C₄-halogenalkenyl, Aryl-C₃-C₄-halogenalkinyl, Aryl-C₁-C₄-hydroxy-alkyl, Arylcarbonyl-C₁-C₄-alkyl, Aryl-C₁-C₄-alkyl carbonyl-C₁-C₄-alkyl, Arylcar-bonyloxy-C₁-C₄-alkyl, Aryloxycarbonyl-C₁-C₄-alkyl, Aryloxy-C₁-C₄-alkyl, Aryl-amino-C₁-C₄-alkyl, Arylthio-C₁-C₄-alkyl, Arylsulfinyl-C₁-C₄-alkyl, Arylsulfonyl-C₁-C₄-alkyl,
Heterocyclyl, Heterocyclyl-C₁-C₄-alkyl, Heterocyclyl-C₂-C₄-alkenyl, Heterocyclyl-C₂-C₄-alkinyl, Heterocyclyl-C₁-C₄-halogenalkyl, Heterocyclyl-C₂-C₄-halogen-alkenyl, Heterocyclyl-C₃-C₄-halogenalkinyl, Heterocyclyl-C₁-C₄-hydroxyalkyl, He-terocyclyl carbonyl-C₁-C₄-alkyl, Heterocyclyl-C₁-C₄-alkyl carbonyl-C₁-C₄-alkyl, Heterocyclylcarbonyloxy-C₁-C₄-alkyl, Heterocyclyloxycarbonyl-C₁-C₄-alkyl, Hete-rocyclyloxy-C₁-C₄-alkyl, Heterocyclylamino-C₁-C₄-alkyl Heterocyclylthio-C₁-C₄-alkyl, Heterocyclylsulfinyl-C₁-C₄-alkyl, Heterocyclylsulfonyl-C₁-C₄-alkyl, wobei die vorstehend genannten Reste partiell oder vollständig halogeniert sein können und/oder ein bis drei Reste aus der Gruppe Cyano, Nitro, C₁-C₆-Alkyl, C₁-C₆-Halogenalkyl, Hydroxy, C₁-C₆-Hydroxyalkyl, Hydroxycarbonyl-C₁-C₆-alkyl, C₁-C₆-Alkoxycarbonyl-C₁-C₆-alkyl, C₁-C₆-Alkoxy, C₁-C₆-Halogenalkoxy, Hydroxy-carbonyl, C₁-C₆-Alkoxycarbonyl, Aminocarbonyl, (C₁-C₆-Alkyl)aminocarbonyl, Di(C₁-C₆-alkyl)aminocarbonyl, Hydroxycarbonyl-C₁-C₆-alkoxy, C₁-C₆-Alkoxy-carbonyl-C₁-C₆-alkoxy, Amino, C₁-C₆-Alkylamino, Di(C₁-C₆-alkyl)amino, C₁-C₆-Alkylsulfonylamino, C₁-C₆-Halogenalkylsulfonylamino, (C₁-C₆-Alkyl)amino-carbonylamino, Di(C₁-C₆-alkyl)aminocarbonylamino, Aryl und Aryl(C₁-C₆-alkyl) tragen können;
vorzugsweise Phenyl, Phenyl-C₁-C₄-alkyl, Phenyl-C₂-C₄-alkenyl, Phenyl-C₂-C₄-alkinyl, Phenyl-C₁-C₄-halogenalkyl, Phenyl-C₂-C₄-halogenalkenyl, Phenyl-C₃-C₄-halogenalkinyl, Phenyl-C₁-C₄-hydroxyalkyl, Phenylcarbonyl-C₁-C₄-alkyl, Phenyl-C₁-C₄-alkyl-carbonyl-C₁-C₄-alkyl Phenylcarbonyloxy-C₁-C₄-alkyl, Phenyloxycar-bonyl-C₁-C₄-alkyl, Phenyloxy-C₁-C₄-alkyl, Phenylamino-C₁-C₄-alkyl, Phenylthio-C₁-C₄-alkyl, Phenylsulfinyl-C₁-C₄-alkyl, Phenylsulfonyl-C₁-C₄-alkyl, Heterocyclyl, Heterocyclyl-C₁-C₄-alkyl, Heterocyclyl-C₂-C₄-alkenyl, Heterocyclyl-C₂-C₄-alkinyl, Heterocyclyl-C₁-C₄-halogenalkyl, Heterocyclyl-C₂-C₄-halogen-alkenyl, Heterocyclyl-C₃-C₄-halogenalkinyl, Heterocyclyl-C₁-C₄-hydroxyalkyl, He-terocyclyl-carbonyl-C₁-C₄-alkyl, Heterocyclyl-C₁-C₄-alkyl-carbonyl-C₁-C₄-alkyl, Heterocyclylcarbonyloxy-C₁-C₄-alkyl, Heterocyclyloxycarbonyl-C₁-C₄-alkyl, Hete-rocyclyloxy-C₁-C₄-alkyl, Heterocyclylamino-C₁-C₄-alkyl, Heterocyclylthio-C₁-C₄-alkyl, Heterocyclylsulfinyl-C₁-C₄-alkyl, Heterocyclylsulfonyl-C₁-C₄-alkyl, wobei die vorstehend genannten Reste partiell oder vollständig halogeniert sein können und/oder ein bis drei Reste aus der Gruppe Cyano, Nitro, C₁-C₆-Alkyl, C₁-C₆-Halogenalkyl, Hydroxy, C₁-C₆-Hydroxyalkyl, C₁-C₆-Alkoxy, C₁-C₆-Halogenalkoxy, Hydroxycarbonyl, C₁-C₆-Alkoxycarbonyl, Aminocarbonyl, (C₁-C₆-Alkyl)aminocarbonyl, Di(C₁-C₆-alkyl)aminocarbonyl, Hydroxycarbonyl-C₁-C₆-alkoxy, C₁-C₆-Alkoxycarbonyl-C₁-C₆-alkoxy, Amino, C₁-C₆-Alkylamino, Di(C₁-C₆-alkyl)amino, C₁-C₆-Alkylsulfonylamino, C₁-C₆-Halogenalkylsulfonylamino, (C₁-C₆-Alkyl)aminocarbonylamino, Di(C₁-C₆-alkyl)aminocarbonylamino, Aryl und Aryl(C₁-C₆-alkyl) tragen können;
insbesondere Phenyl, Phenyl-C₁-C₄-alkyl, Phenyl-C₂-C₄-alkenyl, Phenyl-C₁-C₄-halogenalkyl, Phenyl-C₁-C₄-hydroxyalkyl, Phenylcarbonyl-C₁-C₄-alkyl, Phenyl-C₁-C₄-alkyl carbonyl-C₁-C₄-alkyl, Phenylcarbonyloxy-C₁-C₄-alkyl, Phenyloxycarbo-nyl-C₁-C₄-alkyl, Phenyloxy-C₁-C₄-alkyl, Phenylamino-C₁-C₄-alkyl, Phenylthio-C₁-C₄-alkyl, Phenylsulfonyl-C₁-C₄-alkyl, Heterocyclyl, Heterocyclyl-C₁-C₄-alkyl, Heterocyclyl-C₂-C₄-alkenyl, Heterocyclyl-C₁-C₄-halogenalkyl, Heterocyclyl-C₁-C₄-hydroxyalkyl, Heterocyclylcarbonyl-C₁-C₄-alkyl, Heterocyclyl-C₁-C₄-alkyl-carbonyl-C₁-C₄-alkyl, Heterocyclylcarbonyloxy-C₁-C₄-alkyl, Heterocyclyloxycarbonyl-C₁-C₄-alkyl, Heterocyclyloxy-C₁-C₄-alkyl, Heterocyclylamino-C₁-C₄-alkyl, Heterocyclylthio-C₁-C₄-alkyl, Heterocyclylsulfonyl-C₁-C₄-alkyl,
wobei die vorstehend genannten Reste partiell oder vollständig halogeniert sein können und/oder ein bis drei Reste aus der Gruppe Cyano, Nitro, C₁-C₆-Alkyl, C₁-C₆-Halogenalkyl, Hydroxy, C₁-C₆-Hydroxyalkyl, C₁-C₆-Alkoxy, C₁-C₆-Halogenalkoxy, Hydroxycarbonyl, C₁-C₆-Alkoxycarbonyl, Aminocarbonyl, (C₁-C₆-Alkyl)aminocarbonyl, Di(C₁-C₆-alkyl)aminocarbonyl, Hydroxycarbonyl-C₁-C₆-alkoxy, Di(C₁-C₆-alkyl)amino, Di(C₁-C₆-alkyl)aminocarbonylamino, Aryl und Aryl(C₁-C₆-alkyl) tragen können; besonders bevorzugt Phenyl, Phenyl-C₁-C₄-alkyl, Phenyl-C₁-C₄-halogenalkyl, Phenylcarbonyloxy-C₁-C₄-alkyl,
Phenyloxycarbonyl-C₁-C₄-alkyl, Phenyloxy-C₁-C₄-alkyl,
Heterocyclyl, Heterocyclyl-C₁-C₄-alkyl, Heterocyclyl-C₁-C₄-halogenalkyl,, Heterocyclylcarbonyloxy-C₁-C₄-alkyl, Heterocyclyloxycarbonyl-C₁-C₄-alkyl, Heterocyclyloxy-C₁-C₄-alkyl, wobei die vorstehend genannten Reste partiell oder vollständig halogeniert sein können und/oder ein bis drei Reste aus der Gruppe Cyano, C₁-C₆-Alkyl, C₁-C₆-Halogenalkyl, C₁-C₆-Alkoxy, C₁-C₆-Halogenalkoxy, Hydroxycarbonyl, C₁-C₆-Alkoxycarbonyl, Di(C₁-C₆-alkyl)amino, Aryl und Aryl(C₁-C₆-alkyl) tragen können;
- R³: C₁-C₄-Alkyl, vorzugsweise Methyl; und
- R⁴: Aryl, welches partiell oder vollständig halogeniert sein kann und/oder ein bis drei Reste aus der Gruppe Cyano, Nitro, C₁-C₆-Alkyl, C₁-C₆-Halogenalkyl, C₁-C₆-Alkoxy, C₁-C₆-Halogenalkoxy, Aryl und Aryl(C₁-C₆-alkyl) tragen kann; vorzugsweise Phenyl oder 1-Naphthyl.

Die Amine der Formel II können hydrogenolytisch zu den chiralen Aminen der Formel VIII, 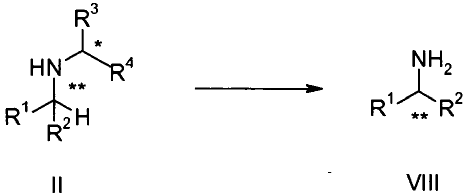 wobei die Reste R¹ und R² die bei den Verbindungen der Formel I gegebene Bedeutung haben, nach an sich bekannten Verfahren, gespalten werden.

Üblicherweise wird diese Hydrogenolyse in einem inerten Lösungsmittel, wie z.B. einem Alkohol, wie Methanol, Ethanol, Isopropanol oder Butanol, einem Ether, wie z.B. Tetrahydrofuran, Dioxan, einem Kohlenwasserstoff, wie z.B. Benzol, Toluol, Ethylbenzol, oder Xylol, oder Gemischen hiervon durchgeführt. Die Hydrogenolyse kann mittels Wasserstoff in Gegenwart einer katalytischen Menge eines Platin-Gruppen-Metallelements, vorzugsweise an Pt/C, Pd/C oder Pd/Al₂O₃, besonders bevorzugt an Pd/C oder Pd/Al₂O₃, durchgeführt werden. Hierbei wird in der Regel der Wasserstoff im Überschuß eingesetzt. Die Umsetzung erfolgt in der Regel bei Raumtemperatur bis Rückflußtemperatur des Reaktionsgemisches und Normaldruck bis zu einem Druck von 200 bar. Nach Beendigung der Umsetzung wird das Reaktionsgemisch nach dem Fachmann bekannten Methoden aufgearbeitet.

Es ist aber auch möglich, die Hydrogenolyse mittels Metallhydriden, wie z.B. Lithium-aluminiumhydrid, Natriumboranat, Natriumcycanoborant, Diboran etc. durchzuführen. Hierbei werden die Edukte in der Regel im stöchiometrischen Verhältnis eingesetzt. Von Fall zu Fall kann es auch von Vorteil sein, das Metallhydrid im Überschuß einzusetzten. Die Umsetzung erfolgt in der Regel bei Raumtemperatur bis Rückflußtemperatur des Reaktionsgemisches, bei Normaldruck. Nach Beendigung der Umsetzung wird das Reaktionsgemisch nach dem Fachmann bekannten Methoden aufgearbeitet.

Die Hydrogenolyse der Amine der Formel II kann kontinuierlich, semi-kontinuierlich oder diskontinuierlich durchgeführt werden.

Die Reste R¹ und R² der Verbindungen der Formel I, II, III, VI und VIII sowie der Rest R^{1'} der Verbindungen der Formel V können je nach Substitutionsmuster noch weitere Chiralitätszentren tragen. Auch diese Verbindungen fallen unter den Gegenstand der vorliegenden Erfindung.

Die für die Substituenten R¹-R⁴ oder als Reste an Phenyl-, Aryl-, Heteroaryl- oder Hetrocyclylringen etc. genannten organischen Molekülteile stellen Sammelbegriffe für individuelle Aufzählungen der einzelnen Gruppenmitglieder dar.

Sämtliche Kohlenwasserstoffketten können geradkettig oder verzweigt sein.

Sofern nicht anders angegeben tragen halogenierte Substituenten vorzugsweise ein bis fünf gleiche oder verschiedene Halogenatome. Die Bedeutung Halogen steht jeweils für Fluor, Chlor, Brom oder lod.

Ferner bedeuten beispielsweise:
- Aryl: ein- bis dreikerniger aromatischer Carbocyclus mit 6 bis 14 Ringgliedern, wie z.B. Phenyl, Naphthyl und Anthracenyl, vorzugsweise Phenyl, Naphthyl;
- Heterocycyl: monocyclische, gesättigte oder partiell ungesättigte Kohlenwasserstoffe mit drei bis sechs Ringgliedern, welche neben Kohlenstoffatomen ein bis vier Stickstoffatome, oder ein bis drei Stickstoffatome und ein Sauerstoff- oder Schwefelatom, oder ein bis drei Sauerstoffatome, oder ein bis drei Schwefelatome enthalten können, und welche über ein C-Atom oder ein N-Atom verknüpft sein können, z.B.
   z.B. 2-Oxiranyl, 2-Oxetanyl, 3-Oxetanyl, 2-Aziridinyl, 3-Thiethanyl, 1-Azetidinyl, 2-Azetidinyl,
   z.B. 2-Tetrahydrofuranyl, 3-Tetrahydrofuranyl, 2-Tetrahydrothienyl, 3-Tetrahydrothienyl, 2-Pyrrolidinyl, 3-Pyrrolidinyl, 3-Isoxazolidinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl, 1,2,4-Triazolidin-3-yl, 1,3,4-Oxadiazolidin-2-yl, 1,3,4-Thiadiazolidin-2-yl, 1,3,4-Triazolidin-2-yl, 1,2,3,4-Tetrazolidin-5-yl;
   z.B. 1-Pyrrolidinyl, 2-Isothiazolidinyl, 2-Isothiazolidinyl, 1-Pyrazolidinyl, 3-Oxazolidinyl, 3-Thiazolidinyl, 1-Imidazolidinyl, 1,2,4-Triazolidin-1-yl, 1,2,4-Oxadiazolidin-2-yl, 1,2,4-Oxadiazolidin-4-yl, 1,2,4-Thiadiazolidin-2-yl, 1,2,4-Thiadiazolidin-4-yl, 1,2,3,4-Tetrazolidin-1-yl,
   z.B. 2,3-Dihydrofur-2-yl, 2,3-Dihydrofur-3-yl, 2,4-Dihydrofur-2-yl, 2,4-Dihydrofur-3-yl, 2,3-Dihydrothien-2-yl, 2,3-Dihydrothien-3-yl, 2,4-Dihydrothien-2-yl, 2,4-Dihydrothien-3-yl, 4,5-Dihydropyrrol-2-yl, 4,5-Dihydropyrrol-3-yl, 2,5-Dihydropyrrol-2-yl, 2,5-Dihydropyrrol-3-yl, 4,5-Dihydroisoxazol-3-yl, 2,5-Dihydroisoxazol-3-yl, 2,3-Di-hydroisoxazol-3-yl, 4,5-Dihydroisoxazol-4-yl, 2,5-Dihydroisoxazol-4-yl, 2,3-Dihydro-isoxazol-4-yl, 4,5-Dihydroisoxazol-5-yl, 2,5-Dihydroisoxazol-5-yl, 2,3-Dihydro-isoxazol-5-yl, 4,5-Dihydroisothiazol-3-yl, 2,5-Dihydroisothiazol-3-yl, 2,3-Dihydro-isothiazol-3-yl, 4,5-Dihydroisothiazol-4-yl, 2,5-Dihydroisothiazol-4-yl, 2,3-Dihydro-isothiazol-4-yl, 4,5-Dihydroisothiazol-5-yl, 2,5-Dihydroisothiazol-5-yl, 2,3-Dihydro-isothiazol -5-yl, 2,3-Dihydropyrazol-2-yl, 2,3-Dihydropyrazol-3-yl, 2,3-Dihydro-pyrazol-4-yl, 2,3-Dihydropyrazol-5-yl, 3,4-Dihydropyrazol-3-yl, 3,4-Dihydropyrazol-4-yl, 3,4-Dihydropyrazol-5-yl, 4,5-Dihydropyrazol-3-yl, 4,5-Dihydropyrazol-4-yl, 4,5-Dihydropyrazol-5-yl, 2,3-Dihydroimidazol-2-yl, 2,3-Dihydroimidazol-3-yl ,2,3-Dihydroimidazol-4-yl, 2,3-Dihydroimidazol-5-yl, 4,5-Dihydroimidazol-2-yl, 4,5-Di-hydroimidazol-4-yl, 4,5-Dihydroimidazol-5-yl, 2,5-Dihydroimidazol-2-yl, 2,5-Di-hydroimidazol-4-yl, 2,5-Dihydroimidazol-5-yl, 2,3-Dihydrooxazol-3-yl, 2,3-Dihydro-oxazol-4-yl, 2,3-Dihydrooxazol-5-yl, 3,4-Dihydrooxazol-3-yl, 3,4-Dihydrooxazol-4-yl, 3,4-Dihydrooxazol-5-yl, 2,3-Dihydrothiazol-3-yl, 2,3-Dihydrothiazol-4-yl, 2,3-Di-hydrothiazol-5-yl, 3,4-Dihydrothiazol-3-yl, 3,4-Dihydrothiazol-4-yl, 3,4-Dihydro-thiazol-5-yl, 3,4-Dihydrothiazol-2-yl, 3,4-Dihydrothiazol-3-yl, 3,4-Dihydrothiazol-4-yl,
   z.B. 4,5-Dihydropyrrol-1-yl, 2,5-Dihydropyrrol-1-yl, 4,5-Dihydroisoxazol-2-yl, 2,3-Dihydroisoxazol-1-yl, 4,5-Dihydroisothiazol-1-yl, 2,3-Dihydroisothiazol-1-yl, 2,3-Di-hydropyrazol-1-yl, 4,5-Dihydropyrazol-1-yl, 3,4-Dihydropyrazol-1-yl, 2,3-Dihydro-imidazol-1-yl, 4,5-Dihydroimidazol-1-yl, 2,5-Dihydroimidazol-1-yl, 2,3-Dihydro-oxazol2-yl, 3,4-Dihydrooxazol-2-yl, 2,3-Dihydrothiazol-2-yl, 3,4-Dihydrothiazol-2-yl;
   z.B. 2-Piperidinyl, 3-Piperidinyl, 4-Piperidinyl, 1,3-Dioxan-2-yl 1,3-Dioxan-4-yl, 1,3-Dioxan-5-yl, 1,4-Dioxan-2-yl, 1,3-Dithian-2-yl, 1,4-Dithian-3-yl, 1,3-Dithian-4-yl, 1,4-Dithian-2-yl, 2-Tetrahydropyranyl, 3-Tetrahydropyranyl, 4-Tetrahydropyranyl, 2-Tetrahydrothiopyranyl, 3-Tetrahydrothiopyranyl, 4-Tetrahydro-thiopyranyl 3-Hexahydropyridazinyl, 4-Hexahydropyridazinyl, 2-Hexahydropyrimidinyl, 4-Hexahydropyrimidinyl, 5-Hexahydropyrimidinyl, 2-Piperazinyl, 1,3,5-Hexa-hydrotriazin-2-yl, 1,2,4-Hexahydrotriazin-3-yl, Tetrahydro-1,3-oxazin-2-yl, Tetra-hydro-1,3-oxazin-6-yl, 2-Morpholinyl, 3-Morpholinyl, 1,3,5-Trioxan-2-yl;
   z.B. 1-Piperidinyl, 1-Hexahydropyridazinyl, 1-Hexahydropyrimidinyl, 1-Piperazinyl, 1,3,5-Hexahydrotriazin-1-yl, 1,2,4-Hexahydrotriazin-1-yl, Tetrahydro-1,3-oxazin-1-yl, 1-Morpholinyl;
   z.B. 2H-Pyran-2-yl, 2H-Pyran-3-yl, 2H-Pyran-4-yl, 2H-Pyran-5-yl, 2H-Pyran-6-yl, 3,6-Dihydro-2H-pyran-2-yl, 3,6-Dihydro-2H-pyran-3-yl, 3,6-Dihydro-2H-pyran-4-yl, 3,6-Dihydro-2H-pyran-5-yl, 3,6-Dihydro-2H-pyran-6-yl, 3,4-Dihydro-2H-pyran-3-yl, 3,4-Dihydro-2H-pyran-4-yl, 3,4-Dihydro-2H-pyran-6-yl, 2H-Thiopyran-2-yl, 2H-Thiopyran-3-yl, 2H-Thiopyran-4-yl, 2H-Thiopyran-5-yl, 2H-Thiopyran-6-yl, 5,6-Dihydro-4H-1,3-oxazin-2-yl;
   sowie Heteroaryl.
- Heteroaryl: mono- oder bicyclisches aromatisches Heteroaryl mit 5 bis 10 Ringgliedem, welches neben Kohlenstoffatomen 1 bis 4 Stickstoffatome, oder 1 bis 3 Stickstoffatome und ein Sauerstoff- oder ein Schwefelatom, oder ein Sauerstoff- oder ein Schwefelatom enthält,
   z.B. Monocyclen wie Furyl (z.B. 2-Furyl, 3-Furyl), Thienyl (z.B. 2-Thienyl, 3-Thienyl), Pyrrolyl (z.B. Pyrrol-2-yl, Pyrrol-3-yl), Pyrazolyl (z.B. Pyrazol-3-yl, Pyrazol-4-yl), Iso-xazolyl (z.B. Isoxazol-3-yl, Isoxazol-4-yl, Isoxazol-5-yl), Isothiazolyl (z.B. Isothiazol-3-yl, Isothiazol-4-yl, Isothiazol-5-yl), Imidazolyl (z.B. Imidazol-2-yl, Imidazol-4-yl), Oxazolyl (z.B. Oxazol-2-yl, Oxazol-4-yl, Oxazol-5-yl), Thiazolyl (z.B. Thiazol-2-yl, Thiazol-4-yl, Thiazol-5-yl), Oxadiazolyl (z.B. 1,2,3-Oxadiazol-4-yl, 1,2,3-Oxadiazol-5-yl, 1,2,4-Oxadiazol-3-yl, 1,2,4-Oxadiazol-5-yl, 1,3,4-Oxadiazol-2-yl), Thiadiazolyl (z.B. 1,2,3-Thiadiazol-4-yl, 1,2,3-Thiadiazol-5-yl, 1,2,4-Thiadiazol-3-yl, 1,2,4-Thiadiazol-5-yl, 1,3,4-Thiadiazolyl-2-yl), Triazolyl (z.B. 1,2,3-Triazol-4-yl, 1,2,4-Triazol-3-yl), Tetrazol-5-yl, Pyridyl (z.B. Pyridin-2-yl, Pyridin-3-yl, Pyridin-4-yl), Pyrazinyl (z.B. Pyridazin-3-yl, Pyridazin-4-yl), Pyrimidinyl (z.B. Pyrimidin-2-yl, Pyrimidin-4-yl, Pyrimidin-5-yl), Pyrazin-2-yl, Triazinyl (z.B. 1,3,5-Triazin-2-yl, 1,2,4-Triazin-3-yl, 1,2,4-Triazin-5-yl, 1,2,4-Triazin-6-yl), Tetrazinyl (z.B. 1,2,4,5-Tetrazin-3-yl); sowie
   Bicyclen wie die benzanellierten Derivate der vorgenannten Monocyclen, z.B. Chinolinyl, Isochinolinyl, Indolyl, Benzthienyl, Benzofuranyl, Benzoxazolyl, Benzthiazolyl, Benzisothiazolyl, Benzimidazolyl, Benzopyrazolyl, Benzthiadiazolyl, Benzotriazolyl;
   vorzugsweise
   5- oder 6-gliedriges Heteroaryl mit ein bis vier Stickstoffatomen, oder ein bis drei Stickstoffatomen und einem Sauerstoff- oder Schwefelatom, oder mit einem Sauerstoff- oder Schwefelatom:
   z.B. über ein C-Atom verknüpfte aromatische 5-Ring-Heterocyclen, welche neben Kohlenstoffatomen ein bis vier Stickstoffatome, oder ein bis drei Stickstoffatome und ein Schwefel- oder Sauerstoffatom, oder ein Schwefel- oder Sauerstoffatom als Ringglieder enthalten können, z.B. 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl und 1,3,4-Triazol-2-yl;
   z.B. über ein C-Atom verknüpfte aromatische 6-Ring Heterocyclen, welche neben Kohlenstoffatomen ein bis vier, vorzugsweise ein bis drei Stickstoffatome als Ringglieder enthalten können, z.B. 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl und 1,2,4-Triazin-3-yl.

### Beispiele:

Die in den nachfolgenden Beispielen eingesetzten Kupfer-haltigen Katalysatoren wurden wie in folgenden Schriften beschrieben hergestellt:
DE 19826396, DE 4428004, EP 383132 bzw. DE 3717111

Das Diastereomerenverhältnis der in den nachfolgenden Beispielen I und II dargestellten Verbindungen wurde wie folgt bestimmt: Derivatisierung mit Trifluoressigsäure, gaschromatographische Trennung auf Säule BGB 175.

Das Diastereomerenverhältnis der in den nachfolgenden Beispielen III und VI dargestellten Verbindungen wurde wie folgt bestimmt: Derivatisierung mit Trifluoressigsäure, gaschromatographische Trennung auf Säule Hydrodex beta 6-TBDM.

Das Diastereomerenverhältnis der in dem nachfolgenden Beispiel VII dargestellten Verbindungen wurde wie folgt bestimmt: gaschromatographische Trennung auf Säule CP-SIL 19 CB.

Das Diastereomerenverhältnis der in dem nachfolgenden Beispiel VIII dargestellten Verbindungen wurde wie folgt bestimmt: gaschromatographische Trennung auf Säule OV1701.

Das Diastereomerenverhältnis der in dem nachfolgenden Beispiel IX dargestellten Verbindungen wurde wie folgt bestimmt: gaschromatographische Trennung auf Säule RTX-5-Amin.

### I. Allgemeine Vorschrift A - Diaselektive Hydrierung von (R)-sec-Butyliden-(1-phenyl-ethyl)-amin (Schiffsche Base von (R)-(1-Phenyl-ethyl)-amin mit 2-Butanon)

540 mg Katalysator wurden in einen 15 mL-Autoklaven gegeben und mit Stickstoff inertisiert. Anschließend wurde der Katalysator für 2 Stunden bei einem Druck von 50 bar bei der in Tabelle 1 angegebenen Temperatur mit Wasserstoff voraktiviert. Dann wurde wiederum mit Stickstoff gespült, hierbei auf Raumtemperatur abgekühlt, und dann unter Stickstoff ein Gemisch von 5,97 mL (R)-sec-Butyliden-(1-phenyl-ethyl)-amin und 1,03 mL Methanol zugefügt. Anschließend wurde Wasserstoff aufgepresst bis ein Druck von etwa 20 bar erreicht wurde und das Reaktionsgemisch auf 100 °C erwärmt. Nach Erreichen dieser Temperatur wurde der Druck auf 70 bar mit Wasserstoff erhöht und der Rührer mit 1000 U/min gestartet. Nach 6 Stunden unter diesen Reaktionsbedingungen wurde eine Probe entnommen und diese gaschromatographisch untersucht. Die Ergebnisse sind in der nachfolgenden Tabelle 1 angegeben.

**Tabelle 1**

| Beispiel | Katalysator [Metallbeladung in Gew.-%] | Träger | Aktivierung [°C] | Umsatz [bzgl. Imin] | Verhältnis RR/RS |
|---|---|---|---|---|---|
| 1.1 | Cu/Ni (5/5) | TiO₂ | 200 | 91 | 84/16 |
| 1.2 | Cu/Ni (5/5) | TiO₂ | 300 | >99 | 82/18 |
| 1.3 | Cu/Ni/Mo (13/40/1) | ZrO₂ | 200 | 99 | 85/15 |
| 1.4 | Cu/Co (5/5) | TiO₂ | 200 | >99 | 84/16 |
| 1.5 | Cu/Co (5/5) | TiO₂ | 300 | >99 | 84/16 |
| 1.6 | Cu/Ni/Co (3,3/3,3/3,3) | TiO₂ | 200 | 98 | 83/17 |
| 1.7 | Cu/Ni/Co (3,3/3,3/3,3) | TiO₂ | 300 | >99 | 85/15 |
| 1.8 | Cu/Ni/Co (11/21/21) | ZrO₂ | 200 | 99 | 85/15 |
| 1.9 | Cu/Zn (32/32) | Al₂O₃ | 200 | 98 | 83/17 |

Zum Vergleich wurden unter den gleichen Bedingungen die Umsetzung mit einem Eisen-Vollkontakt (100% Eisenoxid), einem Kupfer auf Titandioxid-Katalysator, einem Nickel auf Titandioxid-Katalysator und einem Nickel/Kobalt auf Titandioxid-Katalysator durchgeführt. Die Ergebnisse sind in Tabelle 2 aufgeführt.

**Tabelle 2 - Vergleichsbeispiele**

| Beispiel | Katalysator [Metallbeladung in Gew.-%] | Träger | Aktivierung [°C] | Umsatz [bzgl. Imin] | Verhältnis RR/RS |
|---|---|---|---|---|---|
| 2.1 | Fe | - | 200 | 10 | 59/41 |
| 2.2 | Cu (10) | TiO₂ | 200 | 23 | 76/24 |
| 2.3 | Cu (10) | TiO₂ | 300 | 21 | 78/22 |
| 2.4 | Ni (10) | TiO₂ | 200 | >99 | 75/25 |
| 2.5 | Co (10) | TiO₂ | 200 | 99 | 83/15 |
| 2.6 | Ni/Co (5/5) | TiO₂ | 200 | 90 | 77/23 |
| 2.7 | Ni/Co (5/5) | TiO₂ | 300 | >99 | 75/25 |

Der Vergleich der Ergebnisse von Tabelle 1 und 2 zeigt deutlich, dass der Zusatz von Kupfer zu den jeweiligen nicht Kupferhaltigen-Katalysatoren eine Steigerung der Diastereoselktivität bewirkt.

### II. Allgemeine Vorschrift B - Diaselektive Hydrierung von (R)-sec-Butyliden-(1-phenyl-ethyl)-amin (Schiffsche Base von (R)-(1-Phenyl-ethyl)-amin mit 2-Butanon)

Ein Gemisch von (R)-sec-Butyliden-(1-phenyl-ethyl)-amin (Imin); Lösungsmittel (LM) und passivierter Katalysator, wie in Tabelle 3 angegeben, wurden in jeweils in einem 300 ml Autoklaven vorgelegt. Anschließend wurde mit Stickstoff inertisiert und auf 100 °C erwärmt. Anschließend wurde bei dieser Temperatur Wasserstoff bis zu dem gewünschten Druck, wie ebenfalls jeweils in Tabelle 3 angegeben, aufgepresst und bei Abfall des Innendrucks erneut auf den gewünschten Druck gebracht. Nach den jeweils in Tabelle 3 angegebenen Laufzeiten, gemessen ab Aufpressen von Wasserstoff, wurde eine Probe entnommen und diese gaschromatographisch untersucht. Die Ergebnisse sind in der nachfolgenden Tabelle 3 angegeben.

**Tabelle 3**

| Beispiel | lmin [g] | Katalysator [Metall beladung in Gew.-%] | Träger | Kat.: Imin [Gew%] | LM | Gew.-% Imin in LM | p [bar] | Laufzeit [h] | Umsatz [bzgl. Imin] | Ver-hältnis RR/R S |
|---|---|---|---|---|---|---|---|---|---|---|
| 3.1 | 41 | Cu/Ni/Co (3,3/3,3/3,3) | TiO₂ | 2,4 | Methanol | 6 | 70 | 6 | 95 | 92/8 |
| 3.2 | 110 | Cu/Ni/Co (11/21/21) | ZrO₂ | 3 | Ethylbenz ol | 60 | 70 | 12 | >99 | 91/9 |
| 3.3 | 110 | Cu/Ni/Co (11/21/21) | ZrO₂ | 3 | Methanol | 60 | 70 | 12 | >99 | 91/9 |
| 3.4 | 110 | Cu/Ni/Mo (13/40/1) | ZrO₂ | 3 | Ethylbenz ol | 60 | 70 | 12 | >99 | 91/9 |
| 3.5 | 110 | Cu/Ni/Mo (13/40/1) | ZrO₂ | 3 | Methanol | 60 | 70 | 12 | >99 | 91/9 |
| 3.6 | 11 | Cu/Ni/Mo (13/40/1) | ZrO₂ | 1 | Methanol | 8,5 | 100 | 24 | >99 | 91/9 |

Zum Vergleich wurden unter den gleichen Bedingungen die Umsetzung mit einem Pt/C-Katalysator durchgeführt. Die Ergebnisse sind in Tabelle 4 aufgeführt. Das Beispiel zeigt, dass die erfindungsgemäßen Versuche eine höhere Diaselektivität zeigen.

**Tabelle 4 - Vergleichsbeispiel**

| Beispiel | Imin [g] | Katalysator [Metallbeladung in Gew.-%] | Träger | Kat. : Imin [Gew%] | LM | Gew.-% Imin in LM | p [bar] | Laufzeit [h] | Umsatz [bzgl. lmin] | Verhältnis RR/R S |
|---|---|---|---|---|---|---|---|---|---|---|
| 4.1 | 11 | Pt (10) | C | 1 | Methanol | 8,5 | 100 | 24 | >99 | 80/20 |

### III. Allgemeine Vorschrift C - Diaselektive Hydrierung von (R) 1,2-Dimethyl-propyliden)-(phenyl-ethyl)-amin (Schiffsche Base von (R)-(1-Phenyl-ethyl)-amin mit 3-Methyl-2-butanon)

540 mg Katalysator wurden in einen 15 mL-Autoklaven gegeben und mit Stickstoff inertisiert. Anschließend wurde der Katalysator für 2 Stunden bei einem Druck von 50 bar bei der in Tabelle 5 angegebenen Temperatur mit Wasserstoff voraktiviert. Dann wurde wiederum mit Stickstoff gespült, hierbei auf Raumtemperatur abgekühlt, und dann unter Stickstoff ein Gemisch von 6,0 mL (R)-1,2-Dimethylpropyliden-(1-phenyl-ethyl)-amin und 1,0 mL Ethylbenzol zugefügt. Anschließend wurde Wasserstoff aufgepresst bis ein Druck von etwa 20 bar erreicht wurde und das Reaktionsgemisch auf 100 °C erwärmt. Nach Erreichen dieser Temperatur wurde der Druck auf 70 bar mit Wasserstoff erhöht und der Rührer mit 1000 U/min gestartet. Nach 3 Stunden unter diesen Reaktionsbedingungen wurde eine Probe entnommen und diese gaschromatographisch untersucht. Die Ergebnisse sind in der nachfolgenden Tabelle 5 angegeben.

**Tabelle 5**

| Beispiel | Katalysator [Metallbeladung in %] | Träger | Aktivierung [°C] | Umsatz [bzgl. Imin] | Verhältnis RR/RS |
|---|---|---|---|---|---|
| 5.1 | Cu/Ni (5/5) | TiO₂ | 200 | 72 | 94/6 |
| 5.2 | Cu/Ni/Mo (13/40/1) | ZrO₂ | 200 | >99 | 98/2 |
| 5.3 | Cu/Co (5/5) | TiO₂ | 200 | 80 | 98/2 |
| 5.4 | Cu/Co/Mn/Mo/P (20/50/7/3/3) | Vollkon takt | 200 | 13 | 98/2 |
| 5.5 | Cu/Co/Mn/Mo/P (20/50/7/3/3) | Vollkon takt | 300 | 20 | 98/2 |
| 5.6 | Cu/Ni/Co (3,3/3,3/3,3) | TiO₂ | 300 | 88 | 98/2 |
| 5.7 | Cu/Ni/Co (11/21/21) | ZrO₂ | 200 | 95 | 98/2 |
| 5.8 | Cu/Zn (32/32) | Al₂O₃ | 200 | 49 | 98/2 |

Zum Vergleich wurden unter den gleichen Bedingungen die Umsetzung mit einem Eisen-Vollkontakt durchgeführt. Die Ergebnisse sind in Tabelle 6 aufgeführt. Alle Beispiele zeigen, dass die erfindungsgemäßen Versuche eine höhere Diaselektivität zeigen.

**Tabelle 6 - Vergleichsbeispiele**

| Beispiel | Katalysator [Metallbeladung in %] | Träger | Aktivierung [°C] | Umsatz [bzgl. Imin] | Verhältnis RR/RS |
|---|---|---|---|---|---|
| 6.1 | Fe (10) | TiO₂ | 200 | 5 | 62/38 |
| 6.2 | Fe (10) | TiO₂ | 300 | 5 | 66/34 |
| 6.3 | Cu (10) | TiO₂ | 200 | 6 | 91/9 |
| 6.4 | Ni (10) | TiO₂ | 200 | 78 | 91/9 |

### IV. Allgemeine Vorschrift D - Diaselektive Hydrierung von (R)-1,2-Dimethyl-propyliden)-(phenyl-ethyl)-amin (Schiffsche Base von (R)-(1-Phenyl-ethyl)-amin mit 3-Methyl-2-butanon)

Ein Gemisch von (R)-1,2-Dimethyl-propyliden)-1-phenyl-ethyl)-amin (Imin), Lösungsmittel (LM) und passiviertem Katalysator, wie in Tabelle 7 angegeben, wurden in jeweils in einem Autoklaven vorgelegt. Anschließend wurde mit Stickstoff inertisiert und auf die gewünschte Temperatur erwärmt. Anschließend wurde bei dieser Temperatur Wasserstoff bis zu dem gewünschten Druck, wie ebenfalls jeweils in Tabelle 7 angegeben, aufgepresst und bei Abfall des Innendrucks erneut auf den gewünschten Druck gebracht. Nach den jeweils in Tabelle 7 angegebenen Laufzeiten,gemessen ab dem Aufpressen von Wasserstoff, wurde eine Probe entnommen und diese gaschromatographisch untersucht. Die Ergebnisse sind in der nachfolgenden Tabelle 7 angegeben.

**Tabelle 7**

| Beispiel | Imin [g] | Katalysator [Metallbe ladung in %] | Träger | Katalysator: Imin [Gew%] | LM | Gew.-% Imin in LM | Auto klaven-größe [ml] | T [°C] | p [bar] | Laufzeit [h] | Umsatz [bzgl. Imin] | Verhältnis RR/RS |
|---|---|---|---|---|---|---|---|---|---|---|---|---|
| 7.1 | 2000 | Cu/Ni/Co (11/21/21) | ZrO₂ | 10 | Ethlbenzol | 50 | 9000 | 120 | 100 | 42 | >99 | 99/1 |
| 7.2 | 40 | Cu/Ni/Co (3,3/3,3/3,3) | TiO₂ | 2,5 | Methanol | 6 | 300 | 100 | 70 | 6 | 63 | 94/6 |
| 7.3 | 95 | Cu/Ni/Co (11/21/21) | ZrO₂ | 1,5 | Methanol | 60 | 300 | 100 | 70 | 12 | >99 | 99/1 |
| 7.4 | 6800 | Cu/Ni/Co (11/21/21) | ZrO₂ | 7,5 | Toluol/ Ethylbenzol | 63 | 20000 | 120 | 100 | 48 | >99 | 98/2 |

### V. Allgemeine Vorschrift E - Diaselektive Hydrierung von (R)-1,2-Dimethyl-propyliden)-(1-phenyl-ethyl)-amin (Schiffsche Base von (R)-(1-Phenyl-ethyl)-amin mit 3-Methyl-2-butanon)

In einen 250 ml Schlaufenreaktor (Länge = 48,5 cm, Durchmesser = 2,5 cm, Füllhöhe = 46,5 cm), welcher kontinuierlich betrieben wurde, wurde ein passivierter Cu/Ni/Co-Katalysator auf ZrO₂ mit einer Metallbeladung von 11/21/21 Gew.-% bezogen auf das Gesamtgewicht des Katalysators, vorgelegt und eine 30 Gew.%-ige Lösung von (R)-1,2-Dimethyl-propyliden)-(1-phenyl-ethyl)-amin in dem in Tabelle 8 angegebenen Lösungsmittel mit Wasserstoff unter den in Tabelle 8 angegebenen Reaktionsbedingungen in kontinuierlicher Fahrweise hydriert. Von dem Austrag wurde eine Probe entnommen und per Gaschromatographie untersucht. Die Ergebnisse hiervon sind in Tabelle 8 aufgeführt.

**Tabelle 8**

| Beispiel | Lösungsmittel | Belastung [g/h*g Kat] | T [°C] | p [bar] | Umsatz [bzgl. Imin] | Verhältnis RR/RS |
|---|---|---|---|---|---|---|
| 8.1 | Ethylbenzol | 0,05 | 100 | 100 | >99 | 98/2 |
| 8.2 | Ethylbenzol | 0,07 | 100 | 100 | >99 | 98/2 |
| 8.3 | Ethylbenzol | 0,05 | 80 | 100 | >99 | 98/2 |
| 8.4 | Ethylbenzol | 0,05 | 60 | 100 | >99 | 98/2 |
| 8.5 | Ethylbenzol | 0,05 | 40 | 100 | >99 | 98/2 |
| 8.6 | Ethylbenzol | 0,05 | 40 | 70 | >99 | 98/2 |
| 8.7 | Ethylbenzol | 0,05 | 50 | 70 | >99 | 98/2 |
| 8.8 | Ethylbenzol | 0,05 | 50 | 50 | >99 | 98/2 |
| 8.9 | Ethylbenzol | 0,09 | 50 | 50 | 97 | 98/2 |
| 8.10 | Ethylbenzol | 0,09 | 70 | 50 | 98 | 98/2 |
| 8.11 | Ethylbenzol | 0,09 | 70 | 70 | >99 | 98/2 |
| 8.12 | Ethylbenzol | 0,09 | 100 | 100 | >99 | 98/2 |
| 8.13 | Ethylbenzol | 0,14 | 100 | 100 | >99 | 98/2 |
| 8.14 | Ethylbenzol | 0,18 | 100 | 100 | >99 | 98/2 |
| 8.15 | Ethylbenzol | 0,18 | 80 | 100 | >99 | 98/2 |
| 8.16 | Methanol | 0,04 | 100 | 100 | >99 | 98/2 |
| 8.17 | Methanol | 0,06 | 100 | 100 | >99 | 98/2 |
| 8.18 | Methanol | 0,09 | 100 | 100 | >99 | 98/2 |
| 8.19 | Methanol | 0,04 | 100 | 70 | >99 | 98/2 |
| 8.20 | Methanol | 0,04 | 100 | 50 | >99 | 98/2 |
| 8.21 | Methanol | 0,04 | 80 | 50 | >99 | 98/2 |
| 8.22 | Methanol | 0,04 | 60 | 50 | >99 | 98/2 |
| 8.23 | Methanol | 0,04 | 40 | 50 | >99 | 98/2 |

### VI. Allgemeine Vorschrift F - Diaselektive Hydrierung von (S)-1,2-Dimethyl-propyliden)-(1-phenyl-ethyl)-amin (Schiffsche Base von (S)-(1-Phenyl-ethyl)-amin mit 3-Methyl-2-butanon)

In einen 250 ml Schlaufenreaktor (Länge = 48,5 cm, Durchmesser = 2,5 cm, Füllhöhe = 46,5 cm), welcher kontinuierlich betrieben wurde, wurde ein passivierter Cu/Ni/Mo-Katalysator auf ZrO₂ mit einer Metallbeladung von 13/40/1 Gew.-% bezogen auf das Gesamtgewicht des Katalysators, vorgelegt und eine 30 Gew.%-ige Lösung von (S)-1,2-Dimethyl-propyliden)-(1-phenyl-ethyl)-amin in Methanol mit Wasserstoff unter den in Tabelle 9 angegebenen Reaktionsbedingungen in kontinuierlicher Fahrweise hydriert. Von dem Austrag wurde eine Probe entnommen und per Gaschromatographie untersucht. Die Ergebnisse hiervon sind in Tabelle 9 aufgeführt.

**Tabelle 9**

| Beispiel | Belastung [g/h*g Kat] | T [°C] | p [bar] | Umsatz [bzgl. Imin] | Verhältnis SS/SR |
|---|---|---|---|---|---|
| 9.1 | 0,06 | 100 | 120 | 99 | 95/5 |
| 9.2 | 0,10 | 100 | 120 | 99 | 95/5 |
| 9.3 | 0,19 | 100 | 120 | 98 | 95/5 |
| 9.4 | 0,16 | 100 | 100 | >99 | 96/4 |
| 9.5 | 0,16 | 90 | 100 | >99 | 96/4 |
| 9.6 | 0,16 | 80 | 100 | >99 | 96/4 |
| 9.7 | 0,19 | 100 | 120 | 98 | 95/5 |
| 9.8 | 0,25 | 100 | 120 | 97 | 95/5 |

### VII. Allgemeine Vorschrift G - Diaselektive Hydrierung von (R)-(1-Cyclopropyliden)-(1-phenyl-ethyl)-amin (Schiffsche Base von (R)-(1-Phenyl-ethyl)-amin mit 1-Cyclopropyl-1-ethanon)

315 mg Katalysator wurden in einen 15 mL-Autoklaven gegeben und mit Stickstoff inertisiert. Anschließend wurde der Katalysator für 2 Stunden bei einem Druck von 50 bar bei der in Tabelle 10 angegebenen Temperatur mit Wasserstoff voraktiviert.. Dann wurde wiederum mit Stickstoff gespült, hierbei auf Raumtemperatur abgekühlt und dann unter Stickstoff ein Gemisch von 3,5 mL (R)-(1-Cyclopropyliden)-(1-phenyl-ethyl)-amin und 3,5 mL Ethylbenzol zugefügt. Anschließend wurde Wasserstoff aufgepresst bis ein Druck von etwa 20 bar erreicht wurde und das Reaktionsgemisch auf 100 °C erwärmt. Nach Erreichen dieser Temperatur wurde der Druck auf 70 bar mit Wasserstoff erhöht und der Rührer mit 1000 U/min gestartet. Nach 3 Stunden unter diesen Reaktionsbedingungen wurde eine Probe entnommen und diese gaschromatographisch untersucht. Die Ergebnisse sind in der nachfolgenden Tabelle 10 angegeben.

**Tabelle 10**

| Beispiel | Katalysator [Metallbeladung in Gew.-%] | Träger | Aktivierung [°C] | Umsatz [bzgl. Imin] | Verhältnis RR/RS |
|---|---|---|---|---|---|
| 10.1 | Cu/Ni/Mo (13/40/1) | ZrO₂ | 200 | 94 | 67/33 |
| 10.2 | Cu/Co/Mn/Mo/P (20/50/7/3/3) | Vollkontakt | 300 | 32 | 67/33 |
| 10.3 | Cu/Ni/Co (3,3/3,3/3,3) | TiO₂ | 300 | 62 | 67/33 |
| 10.4 | Cu/Ni/Co (11/21/21) | ZrO₂ | 200 | 99 | 67/33 |
| 10.5 | Cu/Zn(32/32) | Al₂O₃ | 200 | 89 | 64/36 |

Zum Vergleich wurden unter den gleichen Bedingungen die Umsetzung mit einem Ni-ckel/Kobalt auf Titandioxid-Katalysator durchgeführt. Die Ergebnisse sind in Tabelle 11 aufgeführt.

**Tabelle 11 - Vergleichsbeispiele**

| Beispiel | Katalysator [Metallbeladung in Gew.-%] | Träger | Aktivierung [°C] | Umsatz [bzgl. Imin] | Verhältnis RR/RS |
|---|---|---|---|---|---|
| 11.1 | Ni/Co (5/5) | TiO₂ | 300 | 30 | 67/33 |

Der Vergleich der Ergebnisse von Tabelle 10 und 11 zeigt deutlich, dass der Zusatz von Kupfer zu den jeweiligen nicht Kupferhaltigen-Katalysatoren bei vergleichbarer Diastereoselektivität ein höherer Umsatz erreicht werden kann.

### VIII. Allgemeine Vorschrift H - Diaselektive Hydrierung von (R)-(1-Phenylethyliden)-(1-phenyl-ethyl)-amin (Schiffsche Base von (R)-(1-Phenyl-ethyl)-amin mit 1-Phenyl-1-ethanon

315 mg Katalysator wurden in einen 15 mL-Autoklaven gegeben und mit Stickstoff inertisiert. Anschließend wurde der Katalysator für 2 Stunden bei einem Druck von 50 bar bei der in Tabelle 12 angegebenen Temperatur mit Wasserstoff voraktiviert. Dann wurde wiederum mit Stickstoff gespült, hierbei auf Raumtemperatur abgekühlt und dann unter Stickstoff ein Gemisch von 3,5 mL (R)-(1-Phenylethyliden)-(1-phenyl-ethyl)-amin und 3,5 mL Ethylbenzol zugefügt. Anschließend wurde Wasserstoff aufgepresst bis ein Druck von etwa 20 bar erreicht wurde und das Reaktionsgemisch auf 100 °C erwärmt. Nach Erreichen dieser Temperatur wurde der Druck auf 70 bar mit Wasserstoff erhöht und der Rührer mit 1000 U/min gestartet. Nach 3 Stunden unter diesen Reaktionsbedingungen eine Probe entnommen und diese gaschromatographisch untersucht. Die Ergebnisse sind in der nachfolgenden Tabelle 12 angegeben.

**Tabelle 12**

| Beispiel | Katalysator [Metallbeladung in Gew.-%] | Träger | Aktivierung [°C] | Umsatz [bzgl. Imin] | Verhältnis RR/RS |
|---|---|---|---|---|---|
| 12.1 | Cu/Ni/Mo (13/40/1) | ZrO₂ | 200 | >99 | 94/6 |
| 12.2 | Cu/Co/Mn/Mo/P (20/50/7/3/3) | Vollkontakt | 300 | 53 | 98/2 |
| 12.3 | Cu/Ni/Co (3,3/3,3/3,3) | TiO₂ | 300 | 48 | 98/2 |
| 12.4 | Cu/Ni/Co (11/21/21) | ZrO₂ | 200 | >99 | 98/2 |
| 12.5 | Cu/Zn(32/32) | Al₂O₃ | 200 | >99 | 99/1 |

Zum Vergleich wurden unter den gleichen Bedingungen die Umsetzung mit einem Kupfer auf Titandioxid und einem Nickel/Kobalt auf Titandioxid-Katalysator durchgeführt. Die Ergebnisse sind in Tabelle 13 aufgeführt.

**Tabelle 13 - Vergleichsbeispiele**

| Beispiel | Katalysator [Metallbeladung in Gew.-%] | Träger | Aktivierung [°C] | Umsatz [bzgl. Imin] | Verhältnis RR/RS |
|---|---|---|---|---|---|
| 13.1 | Cu (10) | TiO₂ | 200 | 1 | 75/25 |
| 13.2 | Ni/Co (5/5) | TiO₂ | 300 | 47 | 96/4 |

Der Vergleich der Ergebnisse von Tabelle 12 und 13 zeigt deutlich, dass der Zusatz von Kupfer zu den jeweiligen nicht Kupferhaltigen-Katalysatoren zu einer Erhöhung der Diastereoselktivität führt.

### IX. Allgemeine Vorschrift I - Diaselektive Hydrierung von (R)-1-Phenylbutyliden)-(1-phenyl-ethyl)-amin (Schiffsche Base von (R)-(1-Phenyl-ethyl)-amin mit 1-Phenyl-1-butanon)

Ein Gemisch von 35 Gew.-% (R)-1-Phenylbutyliden)-(1-phenyl-ethyl)-amin (Imin) in Methanol und passiviertem Katalysator, wie in Tabelle 14 angegeben, wurden in je-weils in einem 300 ml- Autoklaven vorgelegt. Dann wurde mit Stickstoff inertisiert und auf 100 °C erwärmt. Anschließend wurde bei dieser Temperatur Wasserstoff bis zu einem Druck von 100 bar aufgepresst und bei Abfall des Innendrucks erneut auf den gewünschten Druck gebracht. Nach 24 Stunden, gemessen ab dem Aufpressen von Wasserstoffwurde eine Probe entnommen und diese gaschromatographisch untersucht. Die Ergebnisse sind in der nachfolgenden Tabelle 14 angegeben.

**Tabelle 14**

| Beispiel | Imin [g] | Katalysator [Metallbeladung in %] | Träger | Katalysator : Imin [Gew.-%] | Umsatz [bzgl. Imin] | Verhältnis RR/RS |
|---|---|---|---|---|---|---|
| 14.1 | 48 | Cu/Ni/Co (11/21/21) | ZrO₂ | 5 | 98 | 98/2 |
| 14.2 | 48 | Cu/Ni/Mo (13/40/1) | ZrO₂ | 5 | 98 | 98/2 |

### X. Allgemeine Vorschrift J - Synthese der Imine

Die Imine (Schiffsche Basen) können nach einer modifizierten Vorschrift von Charles et al, Bull. Soc. Chim. Fr. 1970, 12, 4439-4446 synthetisiert werden.

Das Keton wird mit 1.25 eq. deines Enantiomers von (1-Phenyl-ethyl)-amin und 0.01 eq. p-Toluolsulfonsäure in Toluol vorgelegt. Das entstehende Reaktionswasser wird kontinuierlich mittels azeoptroper Destillation entfernt. Wenn kein weiterer Umsatz mittels GC zu erkennen ist, wird das Lösungsmittel abgezogen und das Produkt mittels einer fraktionierten Destillation gereinigt.

## Patentansprüche

1. Diastereoselektive Umsetzung von chiralen Iminen der Formel I zu Aminen der Formel II, 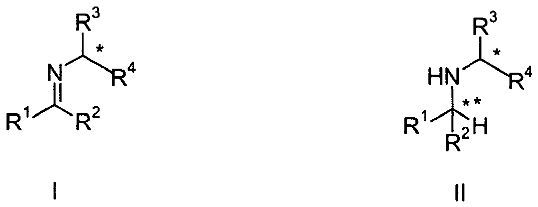 wobei
R¹, R² C₁-C₆-Alkyl, C₃-C₆-Cycloalkyl, C₂-C₆-Alkenyl, C₂-C₆-Alkinyl, C₂-C₆-Halogenalkenyl, C₂-C₆-Halogenalkinyl, C₁-C₆-Alkoxycarbonyl, C₃-C₆-Alkenyloxycarbonyl, C₃-C₆-Alkinyloxycarbonyl, Aminocarbonyl, C₁-C₆-Alkylaminocarbonyl, C₃-C₆-Alkenylaminocarbonyl, C₃-C₆-Alkinylamino-carbonyl, C₁-C₆-Alkylsulfonylaminocarbonyl, Di(C₁-C₆-alkyl)aminocarbonyl, N-(C₃-C₆-Alkenyl)-N-(C₁-C₆-alkyl)aminocarbonyl, N-(C₃-C₆-Alkinyl)-N-(C₁-C₆-alkyl)-aminocarbonyl, N-(C₁-C₆-Alkoxy)-N-(C₁-C₆-alkyl)-amino-carbonyl, N-(C₃-C₆-Alkenyl)-N-(C₁-C₆-alkoxy)aminocarbonyl, N-(C₃-C₆-Alkinyl)-N-(C₁-C₆-alkoxy)-aminocarbonyl, (C₁-C₆-alkyl)aminothiocarbonyl, Di(C₁-C₆-alkyl)aminothiocarbonyl oder C₁-C₆-Alkylcarbonyl-C₁-C₆-alkyl, wobei die genannten Alkyl-, Cycloalkyl- und Alkoxyreste partiell oder vollständig halogeniert sein können und/oder eine bis drei der folgenden Gruppen tragen können: Cyano, Hydroxy, C₁-C₄-Alkyl, C₃-C₆-Cycloalkyl, C₁-C₆-Alkoxy-C₁-C₄-alkyl, C₁-C₄-Alkoxy-C₁-C₄-alkoxy-C₁-C₄-alkyl, C₁-C₄-Alkoxy, C₁-C₄-Alkylthio, Amino, C₁-C₄-alkyl-amino, Di(C₁-C₄-alkyl)amino, C₁-C₄-Alkyl-carbonylamino, Hydroxycarbonyl, C₁-C₄-Alkoxycarbonyl, Aminocarbonyl, C₁-C₄-Alkylaminocarbonyl, Di(C₁-C₄-alkyl)aminocarbonyl oder C₁-C₄-Alkylcarbonyloxy;
Aryl, Aryl-C₁-C₄-alkyl, Aryl-C₂-C₄-alkenyl, Aryl-C₂-C₄-alkinyl, Aryl-C₁-C₄-halogenalkyl, Aryl-C₂-C₄-halogenalkenyl, Aryl-C₃-C₄-halogenalkinyl, Aryl-C₁-C₄-hydroxyalkyl, Arylcarbonyl-C₁-C₄-alkyl, Aryl-C₁-C₄-alkyl carbonyl-C₁-C₄-alkyl, Arylcarbonyloxy-C₁-C₄-alkyl, Aryloxycarbonyl-C₁-C₄-alkyl, Aryloxy-C₁-C₄-alkyl, Arylamino-C₁-C₄-alkyl, Arylthio-C₁-C₄-alkyl, Arylsulfinyl-C₁-C₄-alkyl, Arylsulfonyl-C₁-C₄-alkyl,
Heterocyclyl, Heterocyclyl-C₁-C₄-alkyl, Heterocyclyl-C₂-C₄-alkenyl, Heterocyclyl-C₂-C₄-alkinyl, Heterocyclyl-C₁-C₄-halogenalkyl, Heterocyclyl-C₂-C₄-halogenalkenyl, Heterocyclyl-C₃-C₄-halogenalkinyl, Heterocyclyl-C₁-C₄-hydroxyalkyl, Heterocyclyl-carbonyl-C₁-C₄-alkyl, Heterocyclyl-C₁-C₄-alkyl-carbonyl-C₁-C₄-alkyl, Heterocyclylcarbonyloxy-C₁-C₄-alkyl, Heterocyclyloxycarbonyl-C₁-C₄-alkyl, Heterocyclyloxy-C₁-C₄-alkyl, Heterocyclylamino-C₁-C₄-alkyl Heterocyclylthio-C₁-C₄-alkyl, Heterocyclylsulfinyl-C₁-C₄-alkyl, Heterocyclylsulfonyl-C₁-C₄-alkyl,
wobei die vorstehend genannten Reste partiell oder vollständig halogeniert sein können und/oder ein bis drei Reste aus der Gruppe Cyano, Nitro, C₁-C₆-Alkyl, C₁-C₆-Halogenalkyl, Hydroxycarbonyl-C₁-C₆-alkyl,C₁-C₄-Alkoxy-carbonyl-C₁-C₆-alkyl, Hydroxy, C₁-C₆-Hydroxyalkyl, C₁-C₆-Alkoxy, C₁-C₆-Halogenalkoxy, Hydroxycarbonyl, C₁-C₆-Alkoxycarbonyl, Aminocarbonyl, (C₁-C₆-Alkyl)aminocarbonyl, Di(C₁-C₆-alkyl)aminocarbonyl, Hydroxycarbonyl-C₁-C₆-alkoxy, C₁-C₆-Alkoxycarbonyl-C₁-C₆-alkoxy, Amino, C₁-C₆-Alkyl-amino, Di(C₁-C₆-alkyl)amino, C₁-C₆-Alkylsulfonylamino, C₁-C₆-Halogenalkyl-sulfonylamino, (C₁-C₆-Alkyl)aminocarbonylamino, Di(C₁-C₆-alkyl)amino-carbonylamino, Aryl und Aryl(C₁-C₆-alkyl) tragen können;
wobei die Reste R¹ und R² voneinander verschieden sind;
R³ C₁-C₆-Alkyl;
R⁴ Aryl, welches partiell oder vollständig halogeniert sein kann und/oder ein bis drei Reste aus der Gruppe Cyano, Nitro, C₁-C₆-Alkyl, C₁-C₆-Halogen-alkyl, Hydroxy, C₁-C₆-Hydroxyalkyl, C₁-C₆-Alkoxy, C₁-C₆-Halogenalkoxy, Hydroxycarbonyl, C₁-C₆-Alkoxycarbonyl, C₁-C₆-Alkylamino, Di(C₁-C₆-alkyl)-amino, Aryl und Aryl(C₁-C₆-alkyl) tragen kann;
bedeuten;
und
* für die S oder R-Konfiguration, und
** für die S und/oder R-Konfiguration;
stehen;
indem man das Imin der Formel I in Gegenwart von Wasserstoff und einem heterogenen Kupfer-haltigen Katalysator umsetzt, **dadurch gekennzeichnet, dass** der heterogene Kupfer-haltigen Katalysator, bezogen auf das Gesamtgewicht des Katalysators
| | |
|---|---|
| 0,1 - 95 Gew.% | Kupfer, |
| 0,1 - 85 Gew.% | mindestens ein Metall ausgewählt aus der Gruppe Nickel, Ko-balt und Zink; |
| 0 - 15% Gew.% | mindestens einen Promotor ausgewählt aus der Gruppe Eisen, Rhodium, Ruthenium, Palladium, Platin, Iridium, Osmium, Silber, Gold, Molybdän, Wolfram, Rhenium, Cadmium, Blei, Mangan, Zinn, Chrom, Lithium, Natrium, Kalium, Cäsium, Magnesium, Barium, Phosphor, Arsen, Antimon, Wismut, Selen und Tellur; |
wobei die Summe der Gew.% 100% nicht übersteigt;
enthält.

2. Verfahren nach Anspruch 1, **dadurch gekennzeichnet, dass** der Kupfer-haltige Katalysator ein Trägermaterial enthält.

3. Verfahren nach den Ansprüchen 1 bis 2, **dadurch gekennzeichnet, dass** der heterogene Kupfer-haltigen Katalysator als Trägermaterial Kohle oder ein poröses Metalloxid ausgewählt aus der Gruppe Aluminiumoxid, Siliciumdioxid, Alumosilicaten, Titandioxid, Zirkoniumdioxid, Magnesiumoxid oder Gemische hiervon, enthält.

4. Verfahren nach Anspruch 1, **dadurch gekennzeichnet, dass** der Kupfer-haltige Katalysator ein Vollkontakt ist.

5. Verfahren nach den Ansprüchen 1 bis 4, **dadurch gekennzeichnet, dass** die Umsetzung in Gegenwart eines Lösungsmittels oder in Substanz durchgeführt wird.

6. Verfahren nach den Ansprüchen 1 bis 5, **dadurch gekennzeichnet, dass** die Umsetzung bei Normaldruck bis 200 bar durchgeführt wird.

7. Verfahren nach den Ansprüchen 1 bis 6, **dadurch gekennzeichnet, dass** die Umsetzung bei Raumtemperatur bis Rückflußtemperatur des Reaktionsgemisches durchgeführt wird.

8. Verfahren nach den Ansprüchen 1 bis 7, **dadurch gekennzeichnet, dass**
R³ C₁-C₄-Alkyl, und
R⁴ Aryl, welches partiell oder vollständig halogeniert sein kann und/oder ein bis drei Reste aus der Gruppe Cyano, Nitro, C₁-C₆-Alkyl, C₁-C₆-Halogenalkyl, C₁-C₆-Alkoxy, C₁-C₆-Halogenalkoxy, Aryl und Aryl(C₁-C₆-alkyl) tragen kann;
bedeuten.

9. Verfahren nach den Ansprüchen 1 bis 8, **dadurch gekennzeichnet, dass**
R¹, R² C₁-C₆-Alkyl, C₃-C₆-Cycloalkyl, C₂-C₆-Alkenyl, C₂-C₆-Alkinyl, C₂-C₆-Halogenalkenyl, C₂-C₆-Halogenalkinyl oder C₁-C₆-Alkylcarbonyl-C₁-C₆-alkyl, wobei die genannten Alkyl-, Cycloalkyl- und Alkoxyreste partiell oder vollständig halogeniert sein können und/oder eine bis drei der folgenden Gruppen tragen können: Cyano, Hydroxy, C₁-C₄-Alkyl, C₃-C₆-Cycloalkyl, C₁-C₆-Alkoxy-C₁-C₄-alkyl, C₁-C₄-Alkoxy-C₁-C₄-alkoxy-C₁-C₄-alkyl, C₁-C₄-Alkoxy, C₁-C₄-Alkylthio, Amino, C₁-C₄-alkyl-amino, Di(C₁-C₄-alkyl)amino;
Aryl, Aryl-C₁-C₄-alkyl, Aryl-C₂-C₄-alkenyl, Aryl-C₂-C₄-alkinyl, Aryl-C₁-C₄-halogenalkyl, Aryl-C₂-C₄-halogenalkenyl, Aryl-C₃-C₄-halogenalkinyl, Aryl-C₁-C₄-hydroxyalkyl, Arylcarbonyl-C₁-C₄-alkyl, Aryl-C₁-C₄-alkyl carbonyl-C₁-C₄-alkyl, Aryloxy-C₁-C₄-alkyl, Arylamino-C₁-C₄-alkyl, Arylthio-C₁-C₄-alkyl, Arylsulfinyl-C₁-C₄-alkyl, Arylsulfonyl-C₁-C₄-alkyl,
Heterocyclyl, Heterocyclyl-C₁-C₄-alkyl, Heterocyclyl-C₂-C₄-alkenyl, Heterocyclyl-C₂-C₄-alkinyl, Heterocyclyl-C₁-C₄-halogenalkyl, Heterocyclyl-C₂-C₄-halogenalkenyl, Heterocyclyl-C₃-C₄-halogenalkinyl, Heterocyclyl-C₁-C₄-hydroxyalkyl, Heterocyclylcarbonyl-C₁-C₄-alkyl, Heterocyclyl-C₁-C₄-alkyl-carbonyl-C₁-C₄-alkyl, Heterocyclyloxy-C₁-C₄-alkyl, Heterocyclylamino-C₁-C₄-alkyl, Heterocyclylthio-C₁-C₄-alkyl, Heterocyclylsulfinyl-C₁-C₄-alkyl, Heterocydylsulfonyl-C₁-C₄-alkyl,
wobei die vorstehend genannten Reste partiell oder vollständig halogeniert sein können und/oder ein bis drei Reste aus der Gruppe Cyano, Nitro, C₁-C₆-Alkyl, C₁-C₆-Halogenalkyl, Hydroxy, C₁-C₆-Hydroxyalkyl, C₁-C₆-Alkoxy, C₁-C₆-Halogenalkoxy, C₁-C₆-Alkoxycarbonyl, Di(C₁-C₆-alkyl)amino-carbonyl, Amino, C₁-C₆-Alkylamino, Di(C₁-C₆-alkyl)amino, C₁-C₆-Alkyl-sulfonylamino, C₁-C₆-Halogenalkylsulfonylamino, Aryl und Aryl(C₁-C₆-alkyl) tragen können;
bedeuten.

10. Verfahren zur Herstellung von Aminen der Formel II, umfassend folgende Schritte
a) Umsetzung eines Ketons der Formel III mit einen Amin der Formel IV, 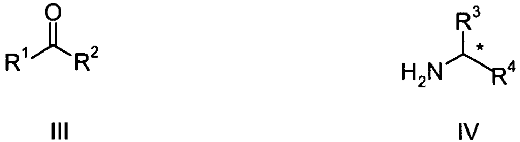 zum Imin der Formel I 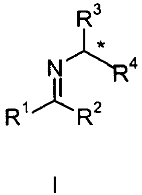 wobei
R¹, R² C₁-C₆-Alkyl, C₃-C₆-Cycloalkyl, C₂-C₆-Alkenyl, C₂-C₆-Alkinyl, C₂-C₆-Halogenalkenyl, C₂-C₆-Halogenalkinyl, C₁-C₆-Alkoxycarbonyl, C₃-C₆-Alkenyloxycarbonyl, C₃-C₆-Alkinyloxycarbonyl, Aminocarbonyl, C₁-C₆-Alkylaminocarbonyl, C₃-C₆-Alkenylaminocarbonyl, C₃-C₆-Alkinylamino-carbonyl, C₁-C₆-Alkylsulfonylaminocarbonyl, Di(C₁-C₆-alkyl)aminocarbonyl, N-(C₃-C₆-Alkenyl)-N-(C₁-C₆-alkyl)aminocarbonyl, N-(C₃-C₆-Alkinyl)-N-(C₁-C₆-alkyl)-aminocarbonyl, N-(C₁-C₆-Alkoxy)-N-(C₁-C₆-alkyl)-amino-carbonyl, N-(C₃-C₆-Alkenyl)-N-(C₁-C₆-alkoxy)aminocarbonyl, N-(C₃-C₆-Alkinyl)-N-(C₁-C₆-alkoxy)-aminocarbonyl, (C₁-C₆-alkyl)aminothiocarbonyl, Di(C₁-C₆-alkyl)aminothiocarbonyl oder C₁-C₆-Alkylcarbonyl-C₁-C₆-alkyl, wobei die genannten Alkyl-, Cycloalkyl- und Alkoxyreste partiell oder vollständig halogeniert sein können und/oder eine bis drei der folgenden Gruppen tragen können: Cyano, Hydroxy, C₁-C₄-Alkyl, C₃-C₆-Cycloalkyl, C₁-C₆-Alkoxy-C₁-C₄-alkyl, C₁-C₄-Alkoxy-C₁-C₄-alkoxy-C₁-C₄-alkyl, C₁-C₄-Alkoxy, C₁-C₄-Alkylthio, Amino, C₁-C₄-alkyl-amino, Di(C₁-C₄-alkyl)amino, C₁-C₄-Alkyl-carbonylamino, Hydroxycarbonyl, C₁-C₄-Alkoxycarbonyl, Aminocarbonyl, C₁-C₄-Alkylaminocarbonyl, Di(C₁-C₄-alkyl)aminocarbonyl oder C₁-C₄-Alkylcarbonyloxy;
Aryl, Aryl-C₁-C₄-alkyl, Aryl-C₂-C₄-alkenyl, Aryl-C₂-C₄-alkinyl, Aryl-C₁-C₄-halogenalkyl, Aryl-C₂-C₄-halogenalkenyl, Aryl-C₃-C₄-halogenalkinyl, Aryl-C₁-C₄-hydroxyalkyl, Arylcarbonyl-C₁-C₄-alkyl, Aryl-C₁-C₄-alkyl carbonyl-C₁-C₄-alkyl, Arylcarbonyloxy-C₁-C₄-alkyl, Aryloxycarbonyl-C₁-C₄-alkyl, Aryloxy-C₁-C₄-alkyl, Arylamino-C₁-C₄-alkyl, Arylthio-C₁-C₄-alkyl, Arylsulfinyl-C₁-C₄-alkyl, Arylsulfonyl-C₁-C₄-alkyl,
Heterocyclyl, Heterocyclyl-C₁-C₄-alkyl, Heterocyclyl-C₂-C₄-alkenyl, Heterocyclyl-C₂-C₄-alkinyl, Heterocyclyl-C₁-C₄-halogenalkyl, Heterocyclyl-C₂-C₄-halogenalkenyl, Heterocydyl-C₃-C₄-halogenalkinyl, Heterocyclyl-C₁-C₄-hydroxyalkyl, Heterocyclyl-carbonyl-C₁-C₄-alkyl, Heterocyclyl-C₁-C₄-alkyl-carbonyl-C₁-C₄-alkyl, Heterocyclylcarbonyloxy-C₁-C₄-alkyl, Heterocyclyloxycarbonyl-C₁-C₄-alkyl, Heterocyclyloxy-C₁-C₄-alkyl, Heterocyclylamino-C₁-C₄-alkyl Heterocyclylthio-C₁-C₄-alkyl, Heterocyclylsulfinyl-C₁-C₄-alkyl, Heterocyclylsulfonyl-C₁-C₄-alkyl,
wobei die vorstehend genannten Reste partiell oder vollständig halogeniert sein können und/oder ein bis drei Reste aus der Gruppe Cyano, Nitro, C₁-C₆-Alkyl, C₁-C₆-Halogenalkyl, Hydroxycarbonyl-C₁-C₆-alkyl,C₁-C₄-Alkoxycarbonyl-C₁-C₆-alkyl, Hydroxy, C₁-C₆-Hydroxyalkyl, C₁-C₆-Alkoxy, C₁-C₆-Halogenalkoxy, Hydroxycarbonyl, C₁-C₆-Alkoxycarbonyl, Aminocarbonyl, (C₁-C₆-Alkyl)aminocarbonyl, Di(C₁-C₆-alkyl)aminocarbonyl, Hydroxycarbonyl-C₁-C₆-alkoxy, C₁-C₆-Alkoxycarbonyl-C₁-C₆-alkoxy, Amino, C₁-C₆-Alkylamino, Di(C₁-C₆-alkyl)amino, C₁-C₆-Alkylsulfonylamino, C₁-C₆-Halogenalkylsulfonylamino, (C₁-C₆-Alkyl)aminocarbonylamino, Di(C₁-C₆-alkyl)aminocarbonylamino, Aryl und Aryl(C₁-C₆-alkyl) tragen können;
wobei die Reste R¹ und R² voneinander verschieden sind;
R³ C₁-C₆-Alkyl;
R⁴ Aryl, welches partiell oder vollständig halogeniert sein kann und/oder ein bis drei Reste aus der Gruppe Cyano, Nitro, C₁-C₆-Alkyl, C₁-C₆-Halogenalkyl, Hydroxy, C₁-C₆-Hydroxyalkyl, C₁-C₆-Alkoxy, C₁-C₆-Halogenalkoxy, Hydroxycarbonyl, C₁-C₆-Alkoxycarbonyl, C₁-C₆-Alkylamino, Di(C₁-C₆-alkyl)amino, Aryl und Aryl(C₁-C₆-alkyl) tragen kann;
bedeuten;
und
* für die S oder R-Konfiguration, und
** für die S und/oder R-Konfiguration;
stehen;
und anschließend
b) Umsetzung des aus Schritt a) erhaltenen Imins der Formel I mit Wasserstoff und einem heterogenen Kupfer-haltigen Katalysator, **dadurch gekennzeichnet, dass** der heterogene Kupfer-haltigen Katalysator, bezogen auf das Gesamtgewicht des Katalysators
| | |
|---|---|
| 0,1 - 95 Gew.% | Kupfer, |
| 0,1 - 85 Gew.% | mindestens ein Metall ausgewählt aus der Gruppe Nickel, Kobalt und Zink; |
| 0 - 15% Gew.% | mindestens einen Promotor ausgewählt aus der Gruppe Eisen, Rhodium, Ruthenium, Palladium, Platin, Iridium, Osmium, Silber, Gold, Molybdän, Wolfram, Rhenium, Cadmium, Blei, Mangan, Zinn, Chrom, Lithium, Natrium, Kalium, Cäsium, Magnesium, Barium, Phosphor, Arsen, Antimon, Wismut, Selen und Tellur; |
wobei die Summe der Gew.% 100% nicht übersteigt;
enthält.

11. Verfahren nach Anspruch 10, **dadurch gekennzeichnet, dass** die Herstellung des Imins der Formel I in Gegenwart einer Säure oder eines heterogenen Katalysators ausgewählt aus der Gruppe Aluminiumoxid, Titandioxid, Zirkondioxid, Siliciumoxid und Tonmineral, und Gemischen hiervon, erfolgt.

12. Verfahren zur Herstellung von chiralen Aminen der Formel VIII, 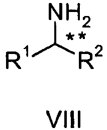 mit
R¹, R² C₁-C₆-Alkyl, C₃-C₆-Cycloalkyl, C₂-C₆-Alkenyl, C₂-C₆-Alkinyl, C₂-C₆-Halogenalkenyl, C₂-C₆-Halogenalkinyl, C₁-C₆-Alkoxycarbonyl, C₃-C₆-Alkenyloxycarbonyl, C₃-C₆-Alkinyloxycarbonyl, Aminocarbonyl, C₁-C₆-Alkylaminocarbonyl, C₃-C₆-Alkenylaminocarbonyl, C₃-C₆-Alkinylaminocarbonyl, C₁-C₆-Alkylsulfonylaminocarbonyl, Di(C₁-C₆-alkyl)aminocarbonyl, N-(C₃-C₆-Alkenyl)-N-(C₁-C₆-alkyl)aminocarbonyl, N-(C₃-C₆-Alkinyl)-N-(C₁-C₆-alkyl)-aminocarbonyl, N-(C₁-C₆-Alkoxy)-N-(C₁-C₆-alkyl)-amino-carbonyl, N-(C₃-C₆-Alkenyl)-N-(C₁-C₆-alkoxy)aminocarbonyl, N-(C₃-C₆-Alkinyl)-N-(C₁-C₆-alkoxy)-aminocarbonyl, (C₁-C₆-alkyl)aminothiocarbonyl, Di(C₁-C₆-alkyl)aminothiocarbonyl oder C₁-C₆-Alkylcarbonyl-C₁-C₆-alkyl, wobei die genannten Alkyl-, Cycloalkyl- und Alkoxyreste partiell oder vollständig halogeniert sein können und/oder eine bis drei der folgenden Gruppen tragen können: Cyano, Hydroxy, C₁-C₄-Alkyl, C₃-C₆-Cycloalkyl, C₁-C₆-Alkoxy-C₁-C₄-alkyl, C₁-C₄-Alkoxy-C₁-C₄-alkoxy-C₁-C₄-alkyl, C₁-C₄-Alkoxy, C₁-C₄-Alkylthio, Amino, C₁-C₄-alkyl-amino, Di(C₁-C₄-alkyl)amino, C₁-C₄-Alkyl-carbonylamino, Hydroxycarbonyl, C₁-C₄-Alkoxycarbonyl, Aminocarbonyl, C₁-C₄-Alkylaminocarbonyl, Di(C₁-C₄-alkyl)aminocarbonyl oder C₁-C₄-Alkylcarbonyloxy;
Aryl, Aryl-C₁-C₄-alkyl, Aryl-C₂-C₄-alkenyl, Aryl-C₂-C₄-alkinyl, Aryl-C₁-C₄-halogenalkyl, Aryl-C₂-C₄-halogenalkenyl, Aryl-C₃-C₄-halogenalkinyl, Aryl-C₁-C₄-hydroxyalkyl, Arylcarbonyl-C₁-C₄-alkyl, Aryl-C₁-C₄-alkyl carbonyl-C₁-C₄-alkyl, Arylcarbonyloxy-C₁-C₄-alkyl, Aryloxycarbonyl-C₁-C₄-alkyl, Aryloxy-C₁-C₄-alkyl, Arylamino-C₁-C₄-alkyl, Arylthio-C₁-C₄-alkyl, Arylsulfinyl-C₁-C₄-alkyl, Arylsulfonyl-C₁-C₄-alkyl,
Heterocyclyl, Heterocyclyl-C₁-C₄-alkyl, Heterocyclyl-C₂-C₄-alkenyl, Heterocyclyl-C₂-C₄-alkinyl, Heterocyclyl-C₁-C₄-halogenalkyl, Heterocyclyl-C₂-C₄-halogenalkenyl, Heterocyclyl-C₃-C₄-halogenalkinyl, Heterocyclyl-C₁-C₄-hydroxyalkyl, Heterocyclyl-carbonyl-C₁-C₄-alkyl, Heterocyclyl-C₁-C₄-alkyl-carbonyl-C₁-C₄-alkyl, Heterocyclylcarbonyloxy-C₁-C₄-alkyl, Heterocyclyloxycarbonyl-C₁-C₄-alkyl, Heterocyclyloxy-C₁-C₄-alkyl, Heterocyclylamino-C₁-C₄-alkyl Heterocyclylthio-C₁-C₄-alkyl, Heterocyclylsulfinyl-C₁-C₄-alkyl, Heterocyclylsulfonyl-C₁-C₄-alkyl,
wobei die vorstehend genannten Reste partiell oder vollständig halogeniert sein können und/oder ein bis drei Reste aus der Gruppe Cyano, Nitro, C₁-C₆-Alkyl, C₁-C₆-Halogenalkyl, Hydroxycarbonyl-C₁-C₆-alkyl,C₁-C₄-Alkoxycarbonyl-C₁-C₆-alkyl, Hydroxy, C₁-C₆-Hydroxyalkyl, C₁-C₆-Alkoxy, C₁-C₆-Halogenalkoxy, Hydroxycarbonyl, C₁-C₆-Alkoxycarbonyl, Aminocarbonyl, (C₁-C₆-Alkyl)aminocarbonyl, Di(C₁-C₆-alkyl)aminocarbonyl, Hydroxycarbonyl-C₁-C₆-alkoxy, C₁-C₆-Alkoxycarbonyl-C₁-C₈-alkoxy, Amino, C₁-C₆-Alkylamino, Di(C₁-C₆-alkyl)amino, C₁-C₆-Alkylsulfonylamino, C₁-C₆-Halogenalkylsulfonylamino, (C₁-C₆-Alkyl)aminocarbonylamino, Di(C₁-C₆-alkyl)aminocarbonylamino, Aryl und Aryl(C₁-C₆-alkyl) tragen können;
wobei die Reste R¹ und R² voneinander verschieden sind;
umfassend folgende Schritte:
a) Umsetzung eines Ketons der Formel III mit einem Amin der Formel IV zur Herstellung des Amins nach Formel I mit
R³ C₁-C₆-Alkyl;
R⁴ Aryl, welches partiell oder vollständig halogeniert sein kann und/oder ein bis drei Reste aus der Gruppe Cyano, Nitro, C₁-C₆-Alkyl, C₁-C₆-Halogenalkyl, Hydroxy, C₁-C₆-Hydroxyalkyl, C₁-C₆-Alkoxy, C₁-C₆-Halogenalkoxy, Hydroxycarbonyl, C₁-C₆-Alkoxycarbonyl, C₁-C₆-Alkylamino, Di(C₁-C₆-alkyl)amino, Aryl und Aryl(C₁-C₆-alkyl) tragen kann;
b) Umsetzung der aus Schritt a) erhaltenen Imins der Formel I mit Wasserstoff und einem heterogenen kupfer-haltigen Katalysator **dadurch gekennzeichnet, dass** der heterogene kupfer-haltige Katalystor, bezogen auf das Gesamtgewicht des Katalysators
| | |
|---|---|
| 0,1 - 95 Gew.% | Kupfer, |
| 0,1 - 85 Gew.% | mindestens ein Metall ausgewählt aus der Gruppe Nickel, Kobalt und Zink; |
| 0 - 15% Gew.% | mindestens einen Promotor ausgewählt aus der Gruppe Eisen, Rhodium, Ruthenium, Palladium, Platin, Iridium, Osmium, Silber, Gold, Molybdän, Wolfram, Rhenium, Cadmium, Blei, Mangan, Zinn, Chrom, Lithium, Natrium, Kalium, Cäsium, Magnesium, Barium, Phosphor, Arsen, Antimon, Wismut, Selen und Tellur; |
wobei die Summe der Gew. % 100% nicht übersteigt;
enthält;
c) anschließende hydrogenolytische Spaltung des aus Schritt b) erhaltenen Amins der Formel II.

13. Verfahren nach Anspruch 12, wobei die Hydrogenolyse mittels Wasserstoff in Gegenwart eines heterogenen Katalysators ausgewählt aus der Gruppe der Platin-Metall-Elemente durchgeführt wird.

14. Verfahren nach Anspruch 13, wobei die Hydrogenolyse mittels Metallhydriden oder Gemischen von Metallhydriden durchgeführt wird.

15. Verfahren nach den Ansprüchen 1 bis 14, **dadurch gekennzeichnet, dass** diese bzw. die zugrundeliegenden Verfahrensstufen kontinuierlich, semi-kontinuierlich oder diskontinuierlich durchgeführt werden.

16. Verwendung des heterogenen Kupfer-haltigen Katalysators gemäß den Ansprüchen 1 bis 15 zur diastereoselektiven Umsetzung von chiralen Iminen, wobei das dem chiralen Imin zugrundeliegende Amin chiral ist und das dem chiralen Imin zugrundeliegende Keton prochiral ist, mit Wasserstoff.

## Claims

1. A diastereoselective conversion of chiral imines of the formula I to amines of the formula II 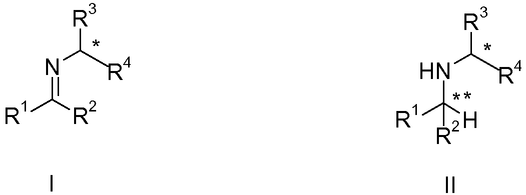 where
R¹, R² are each C₁-C₆-alkyl, C₃-C₆-cycloalkyl, C₂-C₆-alkenyl, C₂-C₆-alkynyl, C₂-C₆-haloalkenyl, C₂-C₆-haloalkynyl, C₁-C₆-alkoxycarbonyl, C₃-C₆-alkenyloxycarbonyl, C₃-C₆-alkynyloxycarbonyl, aminocarbonyl, C₁-C₆-alkylaminocarbonyl, C₃-C₆-alkenylaminocarbonyl, C₃-C₆-alkynylaminocarbonyl, C₁-C₆-alkylsulfonylaminocarbonyl, di(C₁-C₆-alkyl)aminocarbonyl, N-(C₃-C₆-alkenyl)-N-(C₁-C₆-alkyl)aminocarbonyl, N-(C₃-C₆-alkynyl)-N-(C₁C₆-alkyl)aminocarbonyl, N-(C₁-C₆-alkoxy)-N-(C₁-C₆-alkyl)aminocarbonyl, N-(C₃-C₆-alkenyl)-N-(C₁-C₆-alkoxy)aminocarbonyl, N-(C₃-C₆-alkynyl)-N-(C₁C₆-alkoxy)aminocarbonyl, (C₁-C₆-alkyl)aminothiocarbonyl, di(C₁-C₆alkyl)aminothiocarbonyl or C₁-C₆-alkylcarbonyl-C₁-C₆-alkyl,
where the alkyl, cycloalkyl and alkoxy radicals mentioned may be partially or fully halogenated and/or may bear from one to three of the following groups: cyano, hydroxyl, C₁-C₄-alkyl, C₃-C₆-cycloalkyl, C₁-C₆-alkoxy-C₁-C₄alkyl, C₁-C₄-alkoxy-C₁-C₄-alkoxy-C₁-C₄-alkyl, C₁-C₄-alkoxy, C₁-C₄-alkylthio, amino, C₁-C₄-alkylamino, di(C₁-C₄-alkyl)amino, C₁-C₄-alkylcarbonylamino, hydroxycarbonyl, C₁-C₄-alkoxycarbonyl, aminocarbonyl, C₁-C₄-alkylaminocarbonyl, di(C₁-C₄-alkyl)aminocarbonyl or C₁-C₄-alkylcarbonyloxy;
aryl, aryl-C₁-C₄-alkyl, aryl-C₂-C₄-alkenyl, aryl-C₂-C₄-alkynyl, aryl-C₁-C₄-haloalkyl, aryl-C₂-C₄-haloalkenyl, aryl-C₃-C₄-haloalkynyl, aryl-C₁-C₄-hydroxyalkyl, arylcarbonyl-C₁-C₄-alkyl, aryl-C₁-C₄-alkylcarbonyl-C₁-C₄-alkyl, arylcarbonyloxy-C₁-C₄-alkyl, aryloxycarbonyl-C₁-C₄-alkyl, aryloxy-C₁-C₄-alkyl, arylamino-C₁-C₄-alkyl, arylthio-C₁-C₄-alkyl, arylsulfinyl-C₁-C₄-alkyl, arylsulfonyl-C₁-C₄-alkyl,
heterocyclyl, heterocyclyl-C₁-C₄-alkyl, heterocyclyl-C₂-C₄-alkenyl, heterocyclyl-C₂-C₄-alkynyl, heterocyclyl-C₁-C₄-haloalkyl, heterocyclyl-C₂-C₄-haloalkenyl, heterocyclyl-C₃-C₄-haloalkynyl, heterocyclyl-C₁-C₄-hydroxyalkyl, heterocyclylcarbonyl-C₁-C₄-alkyl, heterocyclyl-C₁-C₄-alkyl-carbonyl-C₁-C₄-alkyl, heterocyclylcarbonyloxy-C₁-C₄-alkyl, heterocyclyloxycarbonyl-C₁-C₄-alkyl, heterocyclyloxy-C₁-C₄-alkyl, heterocyclylamino-C₁-C₄-alkyl, heterocyclylthio-C₁-C₄-alkyl, heterocyclylsulfinyl-C₁-C₄-alkyl, heterocyclylsulfonyl-C₁-C₄-alkyl, where the aforementioned radicals may be partially or fully halogenated and/or may bear from one to three radicals from the group of cyano, nitro, C₁-C₆-alkyl, C₁-C₆-haloalkyl, hydroxycarbonyl-C₁-C₆-alkyl, C₁-C₄-alkoxycarbonyl-C₁-C₆-alkyl, hydroxyl, C₁-C₆-hydroxyalkyl, C₁-C₆-alkoxy, C₁-C₆-haloalkoxy, hydroxycarbonyl, C₁-C₆-alkoxycarbonyl, aminocarbonyl, (C₁-C₆-alkyl)aminocarbonyl, di(C₁-C₆-alkyl)aminocarbonyl, hydroxycarbonyl-C₁-C₆-alkoxy, C₁-C₆-alkoxycarbonyl-C₁-C₆-alkoxy, amino, C₁-C₆-alkylamino, di(C₁-C₆-alkyl)amino, C₁-C₆-alkylsulfonylamino, C₁-C₆-haloalkylsulfonylamino, (C₁-C₆-alkyl)aminocarbonylamino, di(C₁-C₆-alkyl)-aminocarbonylamino, aryl and aryl(C₁-C₆-alkyl);
where the R¹ and R² radicals are different than one another;
R³ is C₁-C₆-alkyl;
R⁴ is aryl which may be partially or fully halogenated and/or may bear from one to three radicals from the group of cyano, nitro, C₁-C₆-alkyl, C₁-C₆-haloalkyl, hydroxyl, C₁-C₆-hydroxyalkyl, C₁-C₆-alkoxy, C₁-C₆-haloalkoxy, hydroxycarbonyl, C₁-C₆-alkoxycarbonyl, C₁-C₆-alkylamino, di(C₁-C₆-alkyl)-amino, aryl and aryl(C₁-C₆-alkyl);
and
* represents the S or R configuration, and
** represents the S and/or R configuration;
by converting the imine of the formula I in the presence of hydrogen and a heterogeneous copper-containing catalyst, wherein the heterogeneous copper-containing catalyst comprises, based on the total weight of the catalyst,
| | |
|---|---|
| 0.1 - 95% | by weight of copper, |
| 0.1 - 85% | by weight of at least one metal selected from the group of nickel, cobalt and zinc; |
| 0-15% | by weight of at least one promoter selected from the group of iron, rhodium, ruthenium, palladium, platinum, iridium, osmium, silver, gold, molybdenum, tungsten, rhenium, cadmium, lead, manganese, tin, chromium, lithium, sodium, potassium, cesium, magnesium, barium, phosphorus, arsenic, antimony, bismuth, selenium and tellurium; |
where the sum of the percentages by weight does not exceed 100%.

2. The process according to claim 1, wherein the copper-containing catalyst comprises a support material.

3. The process according to claims 1 to 2, wherein the heterogeneous copper-containing catalyst comprises, as the support material, carbon or a porous metal oxide selected from the group of aluminum oxide, silicon dioxide, aluminosilicates, titanium dioxide, zirconium dioxide, magnesium oxide or mixtures thereof.

4. The process according to claim 1, wherein the copper-containing catalyst is an unsupported catalyst.

5. The process according to claims 1 to 4, wherein the reaction is carried out in the presence of a solvent or in substance.

6. The process according to claims 1 to 5, wherein the reaction is carried out at from standard pressure to 200 bar.

7. The process according to claims 1 to 6, wherein the reaction is carried out at from room temperature to reflux temperature of the reaction mixture.

8. The process according to claims 1 to 7, wherein
R³ is C₁-C₄-alkyl, and
R⁴ is aryl which may be partially or fully halogenated and/or may bear from one to three radicals from the group of cyano, nitro, C₁-C₆-alkyl, C₁-C₆-haloalkyl, C₁-C₆-alkoxy, C₁-C₆-haloalkoxy, aryl and aryl(C₁-C₆-alkyl).

9. The process according to claims 1 to 8, wherein
R¹, R² are each C₁-C₆-alkyl, C₃-C₆-cycloalkyl, C₂-C₆-alkenyl, C₂-C₆-alkynyl, C₂-C₆-haloalkenyl, C₂-C₆-haloalkynyl or C₁-C₆-alkylcarbonyl-C₁-C₆-alkyl, where the alkyl, cycloalkyl and alkoxy radicals mentioned may be partially or fully halogenated and/or may bear from one to three of the following groups: cyano, hydroxyl, C₁-C₄-alkyl, C₃-C₆-cycloalkyl, C₁-C₆-alkoxy-C₁-C₄-alkyl, C₁-C₄-alkoxy-C₁-C₄-alkoxy-C₁-C₄-alkyl, C₁-C₄-alkoxy, C₁-C₄-alkylthio, amino, C₁-C₄-alkylamino, di(C₁-C₄-alkyl)amino;
aryl, aryl-C₁-C₄-alkyl, aryl-C₂-C₄-alkenyl, aryl-C₂-C₄-alkynyl, aryl-C₁-C₄-haloalkyl, aryl-C₂-C₄-haloalkenyl, aryl-C₃-C₄-haloalkynyl, aryl-C₁-C₄-hydroxyalkyl, arylcarbonyl-C₁-C₄-alkyl, aryl-C₁-C₄-alkyl, carbonyl-C₁-C₄-alkyl, aryloxy-C₁-C₄-alkyl, arylamino-C₁-C₄-alkyl, arylthio-C₁-C₄-alkyl, arylsulfinyl-C₁-C₄-alkyl, arylsulfonyl-C₁-C₄-alkyl, heterocyclyl, heterocyclyl-C₁-C₄-alkyl, heterocyclyl-C₂-C₄-alkenyl, heterocyclyl-C₂-C₄-alkynyl, heterocyclyl-C₁-C₄-haloalkyl, heterocyclyl-C₂-C₄-haloalkenyl, heterocyclyl-C₃-C₄-haloalkynyl, heterocyclyl-C₁-C₄-hydroxyalkyl, heterocyclylcarbonyl-C₁-C₄-alkyl, heterocyclyl-C₁-C₄-alkylcarbonyl-C₁-C₄-alkyl, heterocyclyloxy-C₁-C₄-alkyl, heterocyclylamino-C₁-C₄-alkyl, heterocyclylthio-C₁-C₄-alkyl, heterocyclylsulfinyl-C₁-C₄-alkyl, heterocyclylsulfonyl-C₁-C₄-alkyl,
where the aforementioned radicals may be partially or fully halogenated and/or may bear from one to three radicals from the group of cyano, nitro, C₁-C₆-alkyl, C₁-C₆-haloalkyl, hydroxyl, C₁-C₆-hydroxyalkyl, C₁-C₆-alkoxy, C₁-C₆-haloalkoxy, C₁-C₆-alkoxycarbonyl, di(C₁-C₆-alkyl)aminocarbonyl, amino, C₁-C₆-alkylamino, di(C₁-C₆-alkyl)amino, C₁-C₆-alkylsulfonylamino, C₁-C₆-haloalkylsulfonylamino, aryl and aryl(C₁-C₆-alkyl).

10. A process for preparing amines of the formula II, comprising the following steps
a) reacting a ketone of the formula III with an amine of the formula IV 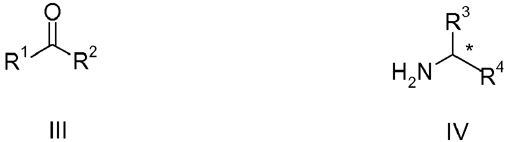 to give the imine of the formula I 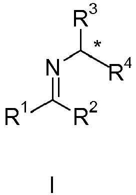 where
R¹, R² are each C₁-C₆-alkyl, C₃-C₆-cycloalkyl, C₂-C₆-alkenyl, C₂-C₆-alkynyl, C₂-C₆-haloalkenyl, C₂-C₆-haloalkynyl, C₁-C₆-alkoxycarbonyl, C₃-C₆-alkenyloxycarbonyl, C₃-C₆-alkynyloxycarbonyl, aminocarbonyl, C₁-C₆-alkylaminocarbonyl, C₃-C₆-alkenylaminocarbonyl, C₃-C₆-alkynylaminocarbonyl, C₁-C₆-alkylsulfonylaminocarbonyl, di(C₁-C₆-alkyl)aminocarbonyl, N-(C₃-C₆-alkenyl)-N-(C₁-C₆-alkyl)aminocarbonyl, N-(C₃-C₆-alkynyl)-N-(C₁-C₆-alkyl)aminocarbonyl, N-(C₁-C₆-alkoxy)-N-(C₁-C₆-alkyl)aminocarbonyl, N-(C₃-C₆-alkenyl)-N-(C₁-C₆-alkoxy)aminocarbonyl, N-(C₃-C₆-alkynyl)-N-(C₁-C₆-alkoxy)aminocarbonyl, (C₁-C₆-alkyl)aminothiocarbonyl, di(C₁-C₆-alkyl)aminothiocarbonyl or C₁-C₆-alkylcarbonyl-C₁-C₆-alkyl,
where the alkyl, cycloalkyl and alkoxy radicals mentioned may be partially or fully halogenated and/or may bear from one to three of the following groups: cyano, hydroxyl, C₁-C₄-alkyl, C₃-C₆-cycloalkyl, C₁-C₆-alkoxy-C₁-C₄-alkyl, C₁-C₄-alkoxy-C₁-C₄-alkoxy-C₁-C₄-alkyl, C₁-C₄-alkoxy, C₁-C₄-alkylthio, amino, C₁-C₄-alkylamino, di(C₁-C₄-alkyl)amino, C₁-C₄-alkylcarbonylamino, hydroxycarbonyl, C₁-C₄-alkoxycarbonyl, aminocarbonyl, C₁-C₄-alkylaminocarbonyl, di(C₁-C₄-alkyl)aminocarbonyl or C₁-C₄-alkylcarbonyloxy;
aryl, aryl-C₁-C₄-alkyl, aryl-C₂-C₄-alkenyl, aryl-C₂-C₄-alkynyl, aryl-C₁-C₄-haloalkyl, aryl-C₂-C₄-haloalkenyl, aryl-C₃-C₄-haloalkynyl, aryl-C₁-C₄-hydroxyalkyl, arylcarbonyl-C₁-C₄-alkyl, aryl-C₁-C₄-alkylcarbonyl-C₁-C₄-alkyl, arylcarbonyloxy-C₁-C₄-alkyl, aryloxycarbonyl-C₁-C₄-alkyl, aryloxy-C₁-C₄-alkyl, arylamino-C₁-C₄-alkyl, arylthio-C₁-C₄-alkyl, arylsulfinyl-C₁-C₄-alkyl, arylsulfonyl-C₁-C₄-alkyl,
heterocyclyl, heterocyclyl-C₁-C₄-alkyl, heterocyclyl-C₂-C₄-alkenyl, heterocyclyl-C₂-C₄-alkynyl, heterocyclyl-C₁-C₄-haloalkyl, heterocyclyl-C₂-C₄-haloalkenyl, heterocyclyl-C₃-C₄-haloalkynyl, heterocyclyl-C₁-C₄-hydroxyalkyl, heterocyclylcarbonyl-C₁-C₄-alkyl, heterocyclyl-C₁-C₄-alkyl-carbonyl-C₁-C₄-alkyl, heterocyclylcarbonyloxy-C₁-C₄-alkyl, heterocyclyloxycarbonyl-C₁-C₄-alkyl, heterocyclyloxy-C₁-C₄-alkyl, heterocyclylamino-C₁-C₄-alkyl, heterocyclylthio-C₁-C₄-alkyl, heterocyclylsulfinyl-C₁-C₄-alkyl, heterocyclylsulfonyl-C₁-C₄-alkyl, where the aforementioned radicals may be partially or fully halogenated and/or may bear from one to three radicals from the group of cyano, nitro, C₁-C₆-alkyl, C₁-C₆-haloalkyl, hydroxycarbonyl-C₁-C₆-alkyl, C₁-C₄-alkoxycarbonyl-C₁-C₆-alkyl, hydroxyl, C₁-C₆-hydroxyalkyl, C₁-C₆-alkoxy, C₁-C₆-haloalkoxy, hydroxycarbonyl, C₁-C₆-alkoxycarbonyl, aminocarbonyl, (C₁-C₆-alkyl)aminocarbonyl, di(C₁-C₆-alkyl)aminocarbonyl, hydroxycarbonyl-C₁-C₆-alkoxy, C₁-C₆-alkoxycarbonyl-C₁-C₆-alkoxy, amino, C₁-C₆-alkylamino, di(C₁-C₆-alkyl)amino, C₁-C₆-alkylsulfonylamino, C₁-C₆-haloalkylsulfonylamino, (C₁-C₆-alkyl)aminocarbonylamino, di(C₁-C₆-alkyl)-aminocarbonylamino, aryl and aryl(C₁-C₆-alkyl);
where the R¹ and R² radicals are different than one another;
R³ is C₁-C₆-alkyl;
R⁴ is aryl which may be partially or fully halogenated and/or may bear from one to three radicals from the group of cyano, nitro, C₁-C₆-alkyl, C₁-C₆-haloalkyl, hydroxyl, C₁-C₆-hydroxyalkyl, C₁-C₆-alkoxy, C₁-C₆-haloalkoxy, hydroxycarbonyl, C₁-C₆-alkoxycarbonyl, C₁-C₆-alkylamino, di(C₁-C₆-alkyl)-amino, aryl and aryl(C₁-C₆-alkyl);
and
* represents the S or R configuration, and
** represents the S and/or R configuration;
and then
b) reacting the imine of the formula I obtained from step a) with hydrogen and a heterogeneous copper-containing catalyst, where the heterogeneous copper-containing catalyst comprises, based on the total weight of the catalyst,
| | |
|---|---|
| 0.1 - 95% by weight | of copper, |
| 0.1 - 85% by weight | of at least one metal selected from the group of nickel, cobalt and zinc; |
| 0 - 15% by weight | of at least one promoter selected from the group of iron, rhodium, ruthenium, palladium, platinum, iridium, osmium, silver, gold, molybdenum, tungsten, rhenium, cadmium, lead, manganese, tin, chromium, lithium, sodium, potassium, cesium, magnesium, barium, phosphorus, arsenic, |
| | antimony, bismuth, selenium and tellurium; |
where the sum of the percentages by weight does not exceed 100%.

11. The process according to claim 10, wherein the imine of the formula I is prepared in the presence of an acid or of a heterogeneous catalyst selected from the group of aluminum oxide, titanium dioxide, zirconium dioxide, silicon oxide and clay mineral, and mixtures thereof.

12. A process for preparing chiral amines of the formula VIII 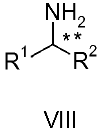 where
R¹, R² are each C₁-C₆-alkyl, C₃-C₆-cycloalkyl, C₂-C₆-alkenyl, C₂-C₆-alkynyl, C₂-C₆-haloalkenyl, C₂-C₆-haloalkynyl, C₁-C₆-alkoxycarbonyl, C₃-C₆-alkenyloxycarbonyl, C₃-C₆-alkynyloxycarbonyl, aminocarbonyl, C₁-C₆-alkylaminocarbonyl, C₃-C₆-alkenylaminocarbonyl, C₃-C₆-alkynylaminocarbonyl, C₁-C₆-alkylsulfonylaminocarbonyl, di(C₁-C₆-alkyl)aminocarbonyl, N-(C₃-C₆-alkenyl)-N-(C₁-C₆-alkyl)aminocarbonyl, N-(C₃-C₆-alkynyl)-N-(C₁-C₆-alkyl)aminocarbonyl, N-(C₁-C₆-alkoxy)-N-(C₁-C₆-alkyl)aminocarbonyl, N-(C₃-C₆-alkenyl)-N-(C₁-C₆-alkoxy)aminocarbonyl, N-(C₃-C₆-alkynyl)-N-(C₁-C₆-alkoxy)aminocarbonyl, (C₁-C₆-alkyl)aminothiocarbonyl, di(C₁-C₆-alkyl)aminothiocarbonyl or C₁-C₆-alkylcarbonyl-C₁-C₆-alkyl, where the alkyl, cycloalkyl and alkoxy radicals mentioned may be partially or fully halogenated and/or may bear from one to three of the following groups: cyano, hydroxyl, C₁-C₄-alkyl, C₃-C₆-cycloalkyl, C₁-C₆-alkoxy-C₁-C₄-alkyl, C₁-C₄-alkoxy-C₁-C₄-alkoxy-C₁-C₄-alkyl, C₁-C₄-alkoxy, C₁-C₄-alkylthio, amino, C₁-C₄-alkylamino, di(C₁-C₄-alkyl)amino, C₁-C₄-alkylcarbonylamino, hydroxycarbonyl, C₁-C₄-alkoxycarbonyl, aminocarbonyl, C₁-C₄-alkylaminocarbonyl, di(C₁-C₄-alkyl)aminocarbonyl or C₁-C₄-alkylcarbonyloxy;
aryl, aryl-C₁-C₄-alkyl, aryl-C₂-C₄-alkenyl, aryl-C₂-C₄-alkynyl, aryl-C₁-C₄-haloalkyl, aryl-C₂-C₄-haloalkenyl, aryl-C₃-C₄-haloalkynyl, aryl-C₁-C₄-hydroxyalkyl, arylcarbonyl-C₁-C₄-alkyl, aryl-C₁-C₄-alkylcarbonyl-C₁-C₄-alkyl, arylcarbonyloxy-C₁-C₄-alkyl, aryloxycarbonyl-C₁-C₄-alkyl, aryloxy-C₁-C₄-alkyl, arylamino-C₁-C₄-alkyl, arylthio-C₁-C₄-alkyl, arylsulfinyl-C₁-C₄-alkyl, arylsulfonyl-C₁-C₄-alkyl,
heterocyclyl, heterocyclyl-C₁-C₄-alkyl, heterocyclyl-C₂-C₄-alkenyl, heterocyclyl-C₂-C₄-alkynyl, heterocyclyl-C₁-C₄-haloalkyl, heterocyclyl-C₂-C₄-haloalkenyl, heterocyclyl-C₃-C₄-haloalkynyl, heterocyclyl-C₁-C₄-hydroxyalkyl, heterocyclylcarbonyl-C₁-C₄-alkyl, heterocyclyl-C₁-C₄-alkyl-carbonyl-C₁-C₄-alkyl, heterocyclylcarbonyloxy-C₁-C₄-alkyl, heterocyclyloxycarbonyl-C₁-C₄-alkyl, heterocyclyloxy-C₁-C₄-alkyl, heterocyclylamino-C₁-C₄-alkyl, heterocyclylthio-C₁-C₄-alkyl, heterocyclylsulfinyl-C₁-C₄-alkyl, heterocyclylsulfonyl-C₁-C₄-alkyl, where the aforementioned radicals may be partially or fully halogenated and/or may bear from one to three radicals from the group of cyano, nitro, C₁-C₆-alkyl, C₁-C₆-haloalkyl, hydroxycarbonyl-C₁-C₆-alkyl, C₁-C₄-alkoxycarbonyl-C₁-C₆-alkyl, hydroxyl, C₁-C₆-hydroxyalkyl, C₁-C₆-alkoxy, C₁-C₆-haloalkoxy, hydroxycarbonyl, C₁-C₆-alkoxycarbonyl, aminocarbonyl, (C₁-C₆-alkyl)aminocarbonyl, di(C₁-C₆-alkyl)aminocarbonyl, hydroxycarbonyl-C₁-C₆-alkoxy, C₁-C₆-alkoxycarbonyl-C₁-C₆-alkoxy, amino, C₁-C₆-alkylamino, di(C₁-C₆-alkyl)amino, C₁-C₆-alkylsulfonylamino, C₁-C₆-haloalkylsulfonylamino, (C₁-C₆-alkyl)aminocarbonylamino, di(C₁-C₆-alkyl)-aminocarbonylamino, aryl and aryl(C₁-C₆-alkyl);
where the R¹ and R² radicals are different than one another;
comprising the following steps:
a) reacting a ketone of the formula III with an amine of the formula IV to prepare the amine according to formula I where
R³ is C₁-C₆-alkyl;
R⁴ is aryl which may be partially or fully halogenated and/or may bear from one to three radicals from the group of cyano, nitro, C₁-C₆-alkyl, C₁-C₆-haloalkyl, hydroxyl, C₁-C₆-hydroxyalkyl, C₁-C₆-alkoxy, C₁-C₆-haloalkoxy, hydroxycarbonyl, C₁-C₆-alkoxycarbonyl, C₁-C₆-alkylamino, di(C₁-C₆-alkyl)amino, aryl and aryl(C₁-C₆-alkyl);
b) reacting the imine of the formula I obtained from step a) with hydrogen and a heterogeneous copper-containing catalyst, where the heterogeneous copper-containing catalyst comprises, based on the total weight of the catalyst,
| | |
|---|---|
| 0.1 - 95% by weight | of copper, |
| 0.1 - 85% by weight | of at least one metal selected from the group of nickel, cobalt and zinc; |
| 0 - 15% by weight | of at least one promoter selected from the group of iron, rhodium, ruthenium, palladium, platinum, iridium, osmium, silver, gold, molybdenum, tungsten, rhenium, cadmium, lead, manganese, tin, chromium, lithium, sodium, potassium, cesium, magnesium, barium, phosphorus, arsenic, antimony, bismuth, selenium and tellurium; |
where the sum of the percentages by weight does not exceed 100%;
c) subsequently hydrogenolytically cleaving the amine of the formula II obtained from step b).

13. The process according to claim 12, wherein the hydrogenolysis is carried out by means of hydrogen in the presence of a heterogeneous catalyst selected from the group of the platinum metal elements.

14. The process according to claim 13, wherein the hydrogenolysis is carried out by means of metal hydrides or mixtures of metal hydrides.

15. The process according to claims 1 to 14, wherein this or the underlying process stages are carried out continuously, semicontinuously or batchwise.

16. The use of the heterogeneous copper-containing catalyst according to claims 1 to 15 for diastereoselective conversion of chiral imines, where the parent amine of the chiral imine is chiral and the parent ketone of the chiral imine is prochiral, with hydrogen.

## Revendications

1. Transformation diastéréosélective d'imines chirales de formule I en amines de formule II 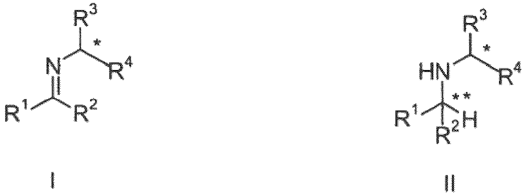 dans lesquelles
R¹, R² signifient alkyle en C₁-C₆, cycloalkyle en C₃-C₆, alcényle en C₂-C₆, alcynyle en C₂-C₆, halogénoalcényle en C₂-C₆, halogénoalcynyle en C₂-C₆, alcoxycarbonyle en C₁-C₆, alcényloxycarbonyle en C₃-C₆, alcynyloxycarbonyle en C₃-C₆, aminocarbonyle, alkylaminocarbonyle en C₁-C₆, alcénylaminocarbonyle en C₃-C₆, alcynylaminocarbonyle en C₃-C₆, alkylsulfonylaminocarbonyle en C₁-C₆, di(alkyle en C₁-C₆)aminocarbonyle, N-(alcényle en C₃-C₆) -N- (alkyle en C₁-C₆) aminocarbonyle, N- (alcynyle en C₃-C₆) -N- (alkyle en C₁-C₆) -aminocarbonyle, N- (alcoxy en C₁-C₆) -N- (alkyle en C₁-C₆)-aminocarbonyle, N-(alcényle en C₃-C₆)-N-(alcoxy en C₁-C₆) aminocarbonyle, N- (alcynyle en C₃-C₆) -N- (alcoxy en C₁-C₆)-aminocarbonyle, (alkyle en C₁-C₆)aminothiocarbonyle, di(alkyle en C₁-C₆)aminothiocarbonyle ou alkyle en C₁-C₆-carbonyl-alkyle en C₁-C₆, les radicaux alkyle, cycloalkyle et alcoxy cités pouvant être halogénés en partie ou en totalité et/ou pouvant porter un à trois des groupes suivants: cyano, hydroxy, alkyle en C₁-C₄, cycloalkyle en C₃-C₆, alcoxy en C₁-C₆-alkyle en C₁-C₄, alcoxy en C₁-C₄-alcoxy en C₁-C₄-alkyle en C₁-C₄, alcoxy en C₁-C₄, alkylthio en C₁-C₄, amino, alkyle en C₁-C₄-amino, di (alkyle en C₁-C₄)amino, alkyle en C₁-C₄-carbonylamino, hydroxycarbonyle, alcoxy en C₁-C₄-carbonyle, aminocarbonyle, alkyle en C₁-C₄-aminocarbonyle, di (alkyle en C₁-C₄)aminocarbonyle ou alkyle en C₁-C₄-carbonyloxy ;
aryle, aryl-alkyle en C₁-C₄, aryl-alcényle en C₂-C₄, aryl-alcynyle en C₂-C₄, aryl-halogénoalkyle en C₁-C₄, aryl-halogénoalcényle en C₂-C₄, aryl-halogénoalcynyle en C₃-C₄, aryl-hydroxyalkyle en C₁-C₄, arylcarbonyl-alkyle en C₁-C₄, aryl-alkyle en C₁-C₄-carbonyl-alkyle en C₁-C₄, arylcarbonyloxy-alkyle en C₁-C₄, aryloxycarbonyl-alkyle en C₁-C₄, aryloxy-alkyle en C₁-C₄, arylamino-alkyle en C₁-C₄, arylthio-alkyle en C₁-C₄, arylsulfinyl-alkyle en C₁-C₄, arylsulfonyl-alkyle en C₁-C₄, hétérocyclyle, hétérocyclyl-alkyle en C₁-C₄, hétérocyclyl-alcényle en C₂-C₄, hétérocyclyl-alcynyle en C₂-C₄, hétérocyclyl-halogénoalkyle en C₁-C₄, hétérocyclyl-halogénoalcényle en C₂-C₄, hétérocyclylhalogénoalcynyle en C₃-C₄, hétérocyclyl-hydroxyalkyle en C₁-C₄, hétérocyclyl-carbonyl-alkyle en C₁-C₄, hétérocyclyl-alkyle en C₁-C₄-carbonyl-alkyle en C₁-C₄, hétérocyclylcarbonyloxy-alkyle en C₁-C₄, hétérocyclyloxy-carbonyl-alkyle en C₁-C₄, hétérocyclyloxy-alkyle en C₁-C₄, hétérocyclylaminoalkyle en C₁-C₄, hétérocyclylthio-alkyle en C₁-C₄, hétérocyclylsulfinyl-alkyle en C₁-C₄, hétérocyclylsulfonyl-alkyle en C₁-C₄, les radicaux susmentionnés pouvant être halogénés en partie ou en totalité et/ou pouvant porter un à trois radicaux du groupe constitué par cyano, nitro, alkyle en C₁-C₆, halogénoalkyle en C₁-C₆, hydroxycarbonyl-alkyle en C₁-C₆, alcoxy en C₁-C₄-carbonyl-alkyle en C₁-C₆, hydroxy, hydroxyalkyle en C₁-C₆, alcoxy en C₁-C₆, halogénoalcoxy en C₁-C₆, hydroxycarbonyle, alcoxycarbonyle en C₁-C₆, aminocarbonyle, (alkyle en C₁-C₆)aminocarbonyle, di (alkyle en C₁-C₆)aminocarbonyle, hydroxycarbonyl-alcoxy en C₁-C₆, alcoxy en C₁-C₆-carbonyl-alcoxy en C₁-C₆, amino, alkyle en C₁-C₆-amino, di (alkyle en C₁-C₆)amino, alkyle en C₁-C₆-sulfonylamino, halogénoalkyle en C₁-C₆-sulfonylamino, (alkyle en C₁-C₆)aminocarbonylamino, di (alkyle en C₁-C₆)amino-carbonylamino, aryle et aryl(alkyle en C₁-C₆) ;
les radicaux R¹ et R² étant différents l'un de l'autre ;
R³ signifie alkyle en C₁-C₆ ;
R⁴ signifie aryle, qui peut être halogéné en partie ou en totalité et/ou qui peut porter un à trois radicaux du groupe constitué par cyano, nitro, alkyle en C₁-C₆, halogénoalkyle en C₁-C₆, hydroxy, hydroxyalkyle en C₁-C₆, alcoxy en C₁-C₆, halogénoalcoxy en C₁-C₆, hydroxycarbonyle, alcoxycarbonyle en C₁-C₆, alkylamino en C₁-C₆, di(alkyle en C₁-C₆)-amino, aryle et aryl(alkyle en C₁-C₆) ;
et
* représente la configuration S ou R et
** représente la configuration S et/ou R ;
selon laquelle l'imine de formule I est mise en réaction en présence d'hydrogène et d'un catalyseur hétérogène contenant du cuivre, **caractérisée en ce que** le catalyseur hétérogène contenant du cuivre contient, par rapport au poids total du catalyseur
0,1 à 95 % en poids de cuivre,
0,1 à 85 % en poids d'au moins un métal choisi dans le groupe constitué par le nickel, le cobalt et le zinc ;
0 à 15 % en poids d'au moins un promoteur choisi dans le groupe constitué par le fer, le rhodium, le ruthénium, le palladium, le platine, l'iridium, l'osmium, l'argent, l'or, le molybdène, le tungstène, le rhénium, le cadmium, le plomb, le manganèse, l'étain, le chrome, le lithium, le sodium, le potassium, le césium, le magnésium, le baryum, le phosphore, l'arsenic, l'antimoine, le bismuth, le sélénium et le tellure ;
la somme des % en poids ne dépassant pas 100 %.

2. Procédé selon la revendication 1, **caractérisé en ce que** le catalyseur contenant du cuivre contient un matériau support.

3. Procédé selon les revendications 1 à 2, **caractérisé en ce que** le catalyseur hétérogène contenant du cuivre contient en tant que matériau support du charbon ou un oxyde métallique poreux choisi dans le groupe constitué par l'oxyde d'aluminium, le dioxyde de silicium, les alumosilicates, le dioxyde de titane, le dioxyde de zirconium, l'oxyde de magnésium ou leurs mélanges.

4. Procédé selon la revendication 1, **caractérisé en ce que** le catalyseur contenant du cuivre est un contact entier.

5. Procédé selon les revendications 1 à 4, **caractérisé en ce que** la réaction est réalisée en présence d'un solvant ou en substance.

6. Procédé selon les revendications 1 à 5, **caractérisé en ce que** la réaction est réalisée de la pression normale à 200 bar.

7. Procédé selon les revendications 1 à 6, **caractérisé en ce que** la réaction est réalisée de la température ambiante à la température de reflux du mélange réactionnel.

8. Procédé selon les revendications 1 à 7, **caractérisé en ce que**
R³ signifie alkyle en C₁-C₄ et
R⁴ signifie aryle, qui peut être halogéné en partie ou en totalité et/ou qui peut porter un à trois radicaux du groupe constitué par cyano, nitro, alkyle en C₁-C₆, halogénoalkyle en C₁-C₆, alcoxy en C₁-C₆, halogénoalcoxy en C₁-C₆, aryle et aryl(alkyle en C₁-C₆).

9. Procédé selon les revendications 1 à 8, **caractérisé en ce que**
R¹, R² signifient alkyle en C₁-C₆, cycloalkyle en C₃-C₆, alcényle en C₂-C₆, alcynyle en C₂-C₆, halogénoalcényle en C₂-C₆, halogénoalcynyle en C₂-C₆ ou alkyle en C₁-C₆-carbonyl-alkyle en C₁-C₆, les radicaux alkyle, cycloalkyle et alcoxy cités pouvant être halogénés en partie ou en totalité et/ou pouvant porter un à trois des groupes suivants : cyano, hydroxy, alkyle en C₁-C₄, cycloalkyle en C₃-C₆, alcoxy en C₁-C₆-alkyle en C₁-C₄, alcoxy en C₁-C₄-alcoxy en C₁-C₄-alkyle en C₁-C₄, alcoxy en C₁-C₄, alkylthio en C₁-C₄, amino, alkyle en C₁-C₄-amino, di (alkyle en C₁-C₄)amino ;
aryle, aryl-alkyle en C₁-C₄, aryl-alcényle en C₂-C₄, aryl-alcynyle en C₂-C₄, aryl-halogénoalkyle en C₁-C₄, aryl-halogénoalcényle en C₂-C₄, aryl-halogénoalcynyle en C₃-C₄, aryl-hydroxyalkyle en C₁-C₄, arylcarbonyl-alkyle en C₁-C₄, aryl-alkyle en C₁-C₄-carbonyl-alkyle en C₁-C₄, aryloxy-alkyle en C₁-C₄, arylamino-alkyle en C₁-C₄, arylthio-alkyle en C₁-C₄, arylsulfinyl-alkyle en C₁-C₄, arylsulfonyl-alkyle en C₁-C₄,
hétérocyclyle, hétérocyclyl-alkyle en C₁-C₄, hétérocyclyl-alcényle en C₂-C₄, hétérocyclyl-alcynyle en C₂-C₄, hétérocyclyl-halogénoalkyle en C₁-C₄, hétérocyclyl-halogénoalcényle en C₂-C₄, hétérocyclyl-halogénoalcynyle en C₃-C₄, hétérocyclyl-hydroxyalkyle en C₁-C₄, hétérocyclyl-carbonyl-alkyle en C₁-C₄, hétérocyclyl-alkyle en C₁-C₄-carbonyl-alkyle en C₁-C₄, hétérocyclyloxy-alkyle en C₁-C₄, hétérocyclylamino-alkyle en C₁-C₄, hétérocyclylthio-alkyle en C₁-C₄, hétérocyclylsulfinyl-alkyle en C₁-C₄, hétérocyclylsulfonyl-alkyle en C₁-C₄,
les radicaux susmentionnés pouvant être halogénés en partie ou en totalité et/ou pouvant porter un à trois radicaux du groupe constitué par cyano, nitro, alkyle en C₁-C₆, halogénoalkyle en C₁-C₆, hydroxy, hydroxyalkyle en C₁-C₆, alcoxy en C₁-C₆, halogénoalcoxy en C₁-C₆, alcoxycarbonyle en C₁-C₆, di (alkyle en C₁-C₆)aminocarbonyle, amino, alkyle en C₁-C₆-amino, di (alkyle en C₁-C₆)amino, alkyle en C₁-C₆-sulfonylamino, halogénoalkyle en C₁-C₆-sulfonylamino, aryle et aryl(alkyle en C₁-C₆).

10. Procédé de fabrication d'amines de formule II, comprenant les étapes suivantes :
a) la mise en réaction d'une cétone de formule III avec une amine de formule IV 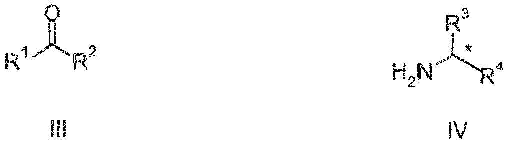 pour former l'imine de formule I 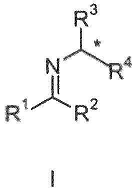 dans laquelle
R¹, R² signifient alkyle en C₁-C₆, cycloalkyle en C₃-C₆, alcényle en C₂-C₆, alcynyle en C₂-C₆, halogénoalcényle en C₂-C₆, halogénoalcynyle en C₂-C₆, alcoxycarbonyle en C₁-C₆, alcényloxycarbonyle en C₃-C₆, alcynyloxycarbonyle en C₃-C₆, aminocarbonyle, alkylaminocarbonyle en C₁-C₆, alcénylaminocarbonyle en C₃-C₆, alcynylaminocarbonyle en C₃-C₆, alkylsulfonylaminocarbonyle en C₁-C₆, di(alkyle en C₁-C₆)aminocarbonyle, N-(alcényle en C₃-C₆)-N- (alkyle en C₁-C₆)aminocarbonyle, N-(alcynyle en C₃-C₆)-N- (alkyle en C₁-C₆)-aminocarbonyle, N- (alcoxy en C₁-C₆)-N- (alkyle en C₁-C₆)-aminocarbonyle, N-(alcényle en C₃-C₆)-N-(alcoxy en C₁-C₆)aminocarbonyle, N-(alcynyle en C₃-C₆)-N- (alcoxy en C₁-C₆)-aminocarbonyle, (alkyle en C₁-C₆)aminothiocarbonyle, di (alkyle en C₁-C₆)aminothiocarbonyle ou alkyle en C₁-C₆-carbonyl-alkyle en C₁-C₆, les radicaux alkyle, cycloalkyle et alcoxy cités pouvant être halogénés en partie ou en totalité et/ou pouvant porter un à trois des groupes suivantes : cyano, hydroxy, alkyle en C₁-C₄, cycloalkyle en C₃-C₆, alcoxy en C₁-C₆-alkyle en C₁-C₄, alcoxy en C₁-C₄-alcoxy en C₁-C₄-alkyle en C₁-C₄, alcoxy en C₁-C₄, alkylthio en C₁-C₄, amino, alkyle en C₁-C₄-amino, di (alkyle en C₁-C₄)amino, alkyle en C₁-C₄-carbonylamino, hydroxycarbonyle, alcoxy en C₁-C₄-carbonyle, aminocarbonyle, alkyle en C₁-C₄-aminocarbonyle, di (alkyle en C₁-C₄)aminocarbonyle ou alkyle en C₁-C₄-carbonyloxy ;
aryle, aryl-alkyle en C₁-C₄, aryl-alcényle en C₂-C₄, aryl-alcynyle en C₂-C₄, aryl-halogénoalkyle en C₁-C₄, aryl-halogénoalcényle en C₂-C₄, aryl-halogénoalcynyle en C₃-C₄, aryl-hydroxyalkyle en C₁-C₄, arylcarbonyl-alkyle en C₁-C₄, aryl-alkyle en C₁-C₄-carbonyl-alkyle en C₁-C₄, arylcarbonyloxy-alkyle en C₁-C₄, aryloxycarbonyl-alkyle en C₁-C₄, aryloxy-alkyle en C₁-C₄, arylamino-alkyle en C₁-C₄, arylthio-alkyle en C₁-C₄, arylsulfinyl-alkyle en C₁-C₄, arylsulfonyl-alkyle en C₁-C₄,
hétérocyclyle, hétérocyclyl-alkyle en C₁-C₄, hétérocyclyl-alcényle en C₂-C₄, hétérocyclyl-alcynyle en C₂-C₄, hétérocyclyl-halogénoalkyle en C₁-C₄, hétérocyclyl-halogénoalcényle en C₂-C₄, hétérocyclyl-halogénoalcynyle en C₃-C₄, hétérocyclyl-hydroxyalkyle en C₁-C₄, hétérocyclyl-carbonyl-alkyle en C₁-C₄, hétérocyclyl-alkyle en C₁-C₄-carbonyl-alkyle en C₁-C₄, hétérocyclylcarbonyloxy-alkyle en C₁-C₄, hétérocyclyloxy-carbonyl-alkyle en C₁-C₄, hétérocyclyloxy-alkyle en C₁-C₄, hétérocyclylamino-alkyle en C₁-C₄, hétérocyclylthio-alkyle en C₁-C₄, hétérocyclylsulfinyl-alkyle en C₁-C₄, hétérocyclylsulfonyl-alkyle en C₁-C₄,
les radicaux susmentionnés pouvant être halogénés en partie ou en totalité et/ou pouvant porter un à trois radicaux du groupe constitué par cyano, nitro, alkyle en C₁-C₆, halogénoalkyle en C₁-C₆, hydroxycarbonyl-alkyle en C₁-C₆, alcoxy en C₁-C₄-carbonyl-alkyle en C₁-C₆, hydroxy, hydroxyalkyle en C₁-C₆, alcoxy en C₁-C₆, halogénoalcoxy en C₁-C₆, hydroxycarbonyle, alcoxycarbonyle en C₁-C₆, aminocarbonyle, (alkyle en C₁-C₆)aminocarbonyle, di (alkyle en C₁-C₆)aminocarbonyle, hydroxycarbonyl-alcoxy en C₁-C₆, alcoxy en C₁-C₆-carbonyl-alcoxy en C₁-C₆, amino, alkyle en C₁-C₆-amino, di (alkyle en C₁-C₆)amino, alkyle en C₁-C₆-sulfonylamino, halogénoalkyle en C₁-C₆-sulfonylamino, (alkyle en C₁-C₆)aminocarbonylamino, di (alkyle en C₁-C₆)amino-carbonylamino, aryle et aryl(alkyle en C₁-C₆) ;
les radicaux R¹ et R² étant différents l'un de l'autre ;
R³ signifie alkyle en C₁-C₆ ;
R⁴ signifie aryle, qui peut être halogéné en partie ou en totalité et/ou qui peut porter un à trois radicaux du groupe constitué par cyano, nitro, alkyle en C₁-C₆, halogénoalkyle en C₁-C₆, hydroxy, hydroxyalkyle en C₁-C₆, alcoxy en C₁-C₆, halogénoalcoxy en C₁-C₆, hydroxycarbonyle, alcoxycarbonyle en C₁-C₆, alkylamino en C₁-C₆, di(alkyle en C₁-C₆)-amino, aryle et aryl(alkyle en C₁-C₆) ;
et
* représente la configuration S ou R et
** représente la configuration S et/ou R ;
puis
b) la mise en réaction de l'imine de formule I obtenue à l'étape a) avec de l'hydrogène et un catalyseur hétérogène contenant du cuivre, **caractérisé en ce que** le catalyseur hétérogène contenant du cuivre contient, par rapport au poids total du catalyseur
0,1 à 95 % en poids de cuivre,
0,1 à 85 % en poids d'au moins un métal choisi dans le groupe constitué par le nickel, le cobalt et le zinc ;
0 à 15 % en poids d'au moins un promoteur choisi dans le groupe constitué par le fer, le rhodium, le ruthénium, le palladium, le platine, l'iridium, l'osmium, l'argent, l'or, le molybdène, le tungstène, le rhénium, le cadmium, le plomb, le manganèse, l'étain, le chrome, le lithium, le sodium, le potassium, le césium, le magnésium, le baryum, le phosphore, l'arsenic, l'antimoine, le bismuth, le sélénium et le tellure ;
la somme des % en poids ne dépassant pas 100 %.

11. Procédé selon la revendication 10, **caractérisé en ce que** la fabrication de l'imine de formule I a lieu en présence d'un acide ou d'un catalyseur hétérogène choisi dans le groupe constitué par l'oxyde d'aluminium, le dioxyde de titane, le dioxyde de zirconium, l'oxyde de silicium et un minéral argileux, et leurs mélanges.

12. Procédé de fabrication d'amines chirales de formule VIII 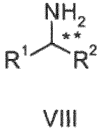 dans laquelle
R¹, R² signifient alkyle en C₁-C₆, cycloalkyle en C₃-C₆, alcényle en C₂-C₆, alcynyle en C₂-C₆, halogénoalcényle en C₂-C₆, halogénoalcynyle en C₂-C₆, alcoxycarbonyle en C₁-C₆, alcényloxycarbonyle en C₃-C₆, alcynyloxycarbonyle en C₃-C₆, aminocarbonyle, alkylaminocarbonyle en C₁-C₆, alcénylaminocarbonyle en C₃-C₆, alcynylaminocarbonyle en C₃-C₆, alkylsulfonylaminocarbonyle en C₁-C₆, di(alkyle en C₁-C₆)aminocarbonyle, N-(alcényle en C₃-C₆)-N- (alkyle en C₁-C₆)aminocarbonyle, N-(alcynyle en C₃-C₆)-N- (alkyle en C₁-C₆)-aminocarbonyle, N- (alcoxy en C₁-C₆)-N- (alkyle en C₁-C₆)-aminocarbonyle, N-(alcényle en C₃-C₆)-N-(alcoxy en C₁-C₆)aminocarbonyle, N-(alcynyle en C₃-C₆)-N- (alcoxy en C₁-C₆)-aminocarbonyle, (alkyle en C₁-C₆)aminothiocarbonyle, di (alkyle en C₁-C₆)aminothiocarbonyle ou alkyle en C₁-C₆-carbonyl-alkyle en C₁-C₆, les radicaux alkyle, cycloalkyle et alcoxy cités pouvant être halogénés en partie ou en totalité et/ou pouvant porter un à trois des groupes suivantes : cyano, hydroxy, alkyle en C₁-C₄, cycloalkyle en C₃-C₆, alcoxy en C₁-C₆-alkyle en C₁-C₄, alcoxy en C₁-C₄-alcoxy en C₁-C₄-alkyle en C₁-C₄, alcoxy en C₁-C₄, alkylthio en C₁-C₄, amino, alkyle en C₁-C₄-amino, di (alkyle en C₁-C₄)amino, alkyle en C₁-C₄-carbonylamino, hydroxycarbonyle, alcoxy en C₁-C₄-carbonyle, aminocarbonyle, alkyle en C₁-C₄-aminocarbonyle, di(alkyle en C₁-C₄)aminocarbonyle ou alkyle en C₁-C₄-carbonyloxy ;
aryle, aryl-alkyle en C₁-C₄, aryl-alcényle en C₂-C₄, aryl-alcynyle en C₂-C₄, aryl-halogénoalkyle en C₁-C₄, aryl-halogénoalcényle en C₂-C₄, aryl-halogénoalcynyle en C₃-C₄, aryl-hydroxyalkyle en C₁-C₄, arylcarbonyl-alkyle en C₁-C₄, aryl-alkyle en C₁-C₄-carbonyl-alkyle en C₁-C₄, arylcarbonyloxy-alkyle en C₁-C₄, aryloxycarbonyl-alkyle en C₁-C₄, aryloxy-alkyle en C₁-C₄, arylamino-alkyle en C₁-C₄, arylthio-alkyle en C₁-C₄, arylsulfinyl-alkyle en C₁-C₄, arylsulfonyl-alkyle en C₁-C₄,
hétérocyclyle, hétérocyclyl-alkyle en C₁-C₄, hétérocyclyl-alcényle en C₂-C₄, hétérocyclyl-alcynyle en C₂-C₄, hétérocyclyl-halogénoalkyle en C₁-C₄, hétérocyclyl-halogénoalcényle en C₂-C₄, hétérocyclyl-halogénoalcynyle en C₃-C₄, hétérocyclyl-hydroxyalkyle en C₁-C₄, hétérocyclyl-carbonyl-alkyle en C₁-C₄, hétérocyclyl-alkyle en C₁-C₄-carbonyl-alkyle en C₁-C₄, hétérocyclylcarbonyloxy-alkyle en C₁-C₄, hétérocyclyloxy-carbonyl-alkyle en C₁-C₄, hétérocyclyloxy-alkyle en C₁-C₄, hétérocyclylamino-alkyle en C₁-C₄, hétérocyclylthio-alkyle en C₁-C₄, hétérocyclylsulfinyl-alkyle en C₁-C₄, hétérocyclylsulfonyl-alkyle en C₁-C₄,
les radicaux susmentionnés pouvant être halogénés en partie ou en totalité et/ou pouvant porter un à trois radicaux du groupe constitué par cyano, nitro, alkyle en C₁-C₆, halogénoalkyle en C₁-C₆, hydroxycarbonyl-alkyle en C₁-C₆, alcoxy en C₁-C₄-carbonyl-alkyle en C₁-C₆, hydroxy, hydroxyalkyle en C₁-C₆, alcoxy en C₁-C₆, halogénoalcoxy en C₁-C₆, hydroxycarbonyle, alcoxycarbonyle en C₁-C₆, aminocarbonyle, (alkyle en C₁-C₆)aminocarbonyle, di (alkyle en C₁-C₆)aminocarbonyle, hydroxycarbonyl-alcoxy en C₁-C₆, alcoxy en C₁-C₆-carbonyl-alcoxy en C₁-C₆, amino, alkyle en C₁-C₆-amino, di(alkyle en C₁-C₆)amino, alkyle en C₁-C₆-sulfonylamino, halogénoalkyle en C₁-C₆-sulfonylamino, (alkyle en C₁-C₆)aminocarbonylamino, di (alkyle en C₁-C₆)amino-carbonylamino, aryle et aryl(alkyle en C₁-C₆) ;
les radicaux R¹ et R² étant différents l'un de l'autre ;
comprenant les étapes suivantes :
a) la mise en réaction d'une cétone de formule III avec une amine de formule IV pour fabriquer l'amine de formule I, dans laquelle
R³ signifie alkyle en C₁-C₆ ;
R⁴ signifie aryle, qui peut être halogéné en partie ou en totalité et/ou qui peut porter un à trois radicaux du groupe constitué par cyano, nitro, alkyle en C₁-C₆, halogénoalkyle en C₁-C₆, hydroxy, hydroxyalkyle en C₁-C₆, alcoxy en C₁-C₆, halogénoalcoxy en C₁-C₆, hydroxycarbonyle, alcoxycarbonyle en C₁-C₆, alkylamino en C₁-C₆, di(alkyle en C₁-C₆)-amino, aryle et aryl(alkyle en C₁-C₆) ;
b) la mise en réaction de l'imine de formule I obtenue à l'étape a) avec de l'hydrogène et un catalyseur hétérogène contenant du cuivre, **caractérisé en ce que** le catalyseur hétérogène contenant du cuivre contient, par rapport au poids total du catalyseur
0,1 à 95 % en poids de cuivre,
0,1 à 85 % en poids d'au moins un métal choisi dans le groupe constitué par le nickel, le cobalt et le zinc ;
0 à 15 % en poids d'au moins un promoteur choisi dans le groupe constitué par le fer, le rhodium, le ruthénium, le palladium, le platine, l'iridium, l'osmium, l'argent, l'or, le molybdène, le tungstène, le rhénium, le cadmium, le plomb, le manganèse, l'étain, le chrome, le lithium, le sodium, le potassium, le césium, le magnésium, le baryum, le phosphore, l'arsenic, l'antimoine, le bismuth, le sélénium et le tellure ;
la somme des % en poids ne dépassant pas 100 % ;
c) puis le clivage hydrogénolytique de l'amine de formule II obtenue à l'étape b).

13. Procédé selon la revendication 12, dans lequel l'hydrogénolyse est réalisée avec de l'hydrogène en présence d'un catalyseur hétérogène choisi dans le groupe des éléments métalliques du platine.

14. Procédé selon la revendication 13, dans lequel l'hydrogénolyse est réalisée avec des hydrures métalliques ou des mélanges d'hydrures métalliques.

15. Procédé selon les revendications 1 à 14, **caractérisé en ce que** celui-ci ou les étapes de procédé sous-jacentes sont réalisés de manière continue, semi-continue ou discontinue.

16. Utilisation du catalyseur hétérogène contenant du cuivre selon les revendications 1 à 15 pour la réaction diastéréosélective d'imines chirales, l'amine sous-jacente à l'imine chirale étant chirale et la cétone sous-jacente à l'imine chirale étant prochirale, avec de l'hydrogène.
